# EUROPEAN PATENT SPECIFICATION

(11) **EP 1 628 977 B1**
(45) Date of publication and mention of the grant of the patent: **12.11.2008**
(21) Application number: 04750953.4
(22) Date of filing: 29.04.2004
(51) Int. Cl.: C07D 471/04

(54) **ANTIBIOTIC TETRAHYDRO-b-CARBOLINE DERIVATIVES**
ANTIBIOTISCHE TETRAHYDRO-b-CARBOLINDERIVATIVE
DERIVES DE TETRAHYDRO-b-CARBOLINE ANTIBIOTIQUES

(30) Priority: 29.04.2003 US 466537 P
(43) Date of publication of application: 01.03.2006
(73) Proprietor: Oscient Pharmaceuticals, Waltham, MA 02451 (US); ARQULE, INC., Woburn, MA 01801 (US)
(72) Inventor: OPPERMAN, Timothy, Somerville, MA 02143 (US); ARVANITES, Anthony, C., New Bedford, MA 02740 (US); PINTO, Julia, C., Beverly, MA 01915 (US); XIANG, Yibin, Acton, MA 01720 (US); ALI, Syed, Masarrat, North Andover, MA 01845 (US); GENG, Bolin, Andover, MA 01810 (US); ASHWELL, Mark, A., Carlisle, MA 01741 (US); KAPLAN, Alan, P., Kings Park, NY 11754 (US)
(74) Representative: Luderschmidt, Schüler & Partner
(86) International application number: PCT/US2004/013322
(87) International publication number: WO 2004/096802

(56) References cited:
- WO-A-00/72846
- WO-A-02/064590
- WO-A-02/064591
- WO-A-03/099821
- FR-A- 1 453 532
- US-A- 4 283 536
- US-A1- 2003 232 850
- US-B1- 6 350 757
- US-B1- 6 573 272
- AHSAN A M ET AL: "RESERPINE ANALOGUES: SYNTHESIS OF BETA-CARBOLINE DERIVATIVES" JOURNAL OF THE CHEMICAL SOCIETY, CHEMICAL SOCIETY. LETCHWORTH, GB, 1963, pages 3928-3930, XP002932022 ISSN: 0368-1769

## Description

### RELATED APPLICATION

This application claims the benefit of U.S. Provisional Application No. 60/466,537, filed on April 29, 2003. The entire teachings of the above application is incorporated herein by reference.

### BACKGROUND OF THE INVENTION

In the last century, antibiotics were developed that led to significant reductions in mortality. Unfortunately, widespread use has led to the rise of antibiotic resistant bacteria, e.g., methicillin resistant *Staphyloccocus aureus* (MRSA), vancomycin resistant *enterococci* (VRE), and penicillin-resistant *Streptococcus pneumoniae* (PRSP). Some bacteria are resistant to a range of antibiotics, e.g., strains of *Mycobacterium tuberculosis* resist isoniazid, rifampin, ethambutol, streptomycin, ethionamide, kanamycin, and rifabutin. In addition to resistance, global travel has spread relatively unknown bacteria from isolated areas to new populations. Furthermore, there is the threat of bacteria as biological weapons. These bacteria may not be easily treated with existing antibiotics.

Infectious bacteria employ the coenzyme A (CoA) biosynthesis pathway, and in particular, in the penultimate step of the pathway, depend on phosphopantetheine adenyl transferase (PPAT), which transfers an adenyl moiety from adenosine triphosphate (ATP) to 4' phosphopanthetheine, forming dephospho-CoA (dPCoA): 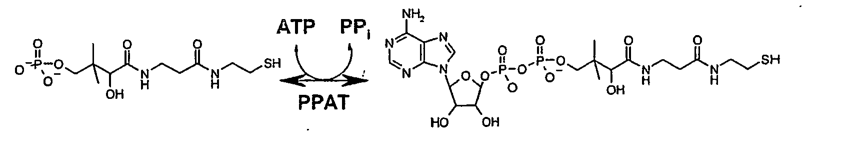 While PPAT is present in mammalian cells, bacterial and mammalian PPAT enzymes differ substantially in primary sequence (about 18% identity) and physical properties. Thus, PPAT presents a desirable, selective target for new antibiotics.

Recently, other efforts reported the identification of compounds which inhibit *E. coli* PPAT (Leslie, et al "Antibacterial Anthranilates with a Novel Mode of Action"; Zhao, et. al. "Inhibitors of Phosphopantetheine Adenylyltransferase"; Presented at the 42nd Interscience Conference on Antimicrobial Agents and Chemotherapy (ICAAC), San Diego, CA, 2002). However, these compounds are not appropriate for drug development. Furthermore, in one case, the structures are peptidic, while in the other case, representative compounds exhibited poor activity against purified PPAT.

Therefore, there is a need for new antibiotics that target PPAT, whereby infections from bacteria dependent on PPAT can be treated.

### SUMMARY OF THE INVENTION

It has now been found that certain tetrahydro-β-carboline derivatives strongly inhibit PPAT, as shown in Example 3. Furthermore, a number of the disclosed compounds are found to have antibiotic activity against bacteria, including drug-resistant bacteria, as shown in Example 4. Based on these discoveries, compounds that are PPAT inhibitors, methods of treatment with the disclosed PPAT inhibitors, and pharmaceutical compositions comprising the disclosed PPAT inhibitors are provided herein.

One embodiment of the invention is the use of an effective amount of a compound represented by structural formula **I**, or a pharmaceutically acceptable salt, solvate, or hydrate thereof: 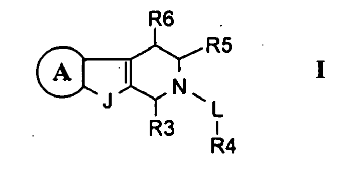 wherein:
Ring **A** is phenyl, optionally substituted at any substitutable ring atom with R1, wherein each R1 is independently halogen, -CN, -NO₂, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, or optionally substituted aryl, aralkyl or C1-C5 alkyl;
   wherein:
   each R^{d} and R^{f} is independently-H, aryl, aralkyl, C1-C5 alkyl, or C1-C5 haloalkyl;
   each R^{e} is independently -H, aryl, aralkyl, or C1-C5 alkyl, or NR^{e}₂ is a non-aromatic heterocyclic group, and
   s is 0 to 5;
   J is -NR2-, R2 is -H or optionally substituted C1-C5 alkyl, and R3 is optionally substituted aryl, aralkyl, heteroaryl, heteroaralkyl, C3-C7 cycloaliphatic, or C3-C7 cycloalkyl;
   L is -(CH₂)-, -(CO)-, -(CS)-, -(SO)-, or -(SO₂)-;
   R4 is an aryl, biaryl, heteroaryl, biheteroaryl, heteroaryl-aryl, aryl-heteroaryl, aralkyl, heteroaralkyl, C1-C8 aliphatic, C3-C7 cycloalkyl, C5-C7 cycloaliphatic, or a 3-7 membered non-aromatic heterocyclic group, wherein the group represented by R4 is substituted with -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂, or -NR^{c}SO₂R^{a};
   R5 is -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂**,** or -NR^{c}SO₂R^{a};
   R6 is -H, -OH, halogen, or optionally substituted C1-C3 alkyl or alkoxy;
   each R^{a} and R^{c} is independently-H, C1-C5 alkyl, aryl, or aralkyl; and
   each R^{b} is independently -H, C1-C5 alkyl, aryl, or aralkyl, or NR^{b}₂ is a nonaromatic heterocyclic group;
wherein the optional substituents for alkyl, aliphatic, cycloalkyl, cycloaliphatic, heterocycle, aryl, heteroaryl, heteroaralkyl and aralkyl are -OH, halogen (-Br, -Cl, -I and -F), -R, -OR, -CH₂R, - CH₂CH₂R, -OCH₂R, -CH₂OR, -CH₂CH₂OR, -CH₂OC(O)R, -O-COR, -COR, -SR, -SCH₂R, - CH₂SF, -SOR, -SO₂R, -CN, -NO₂, -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR, wherein k is 0, 1 or 2, or -NH-C(=NH)-NH₂; wherein each R is independently an alkyl, cycloalkyl, benzyl, aromatic, heteroaromatic, or *N*-anilinyl group or -N(R)₂, taken together, can also form a heterocyclic group; and substituents on the nitrogen of a heterocyclic group or heteroaromatic group include -R', -N(R')₂, -C(O)R', -CO₂ R', -C(O)C(O)R', -C(O)CH₂ C(O)R', -SO₂R', -SO₂ N(R')₂, -C(=S)N(R')₂, -C(=NH)-N(R')₂, and -NR' SO₂R', wherein R' is hydrogen, alkyl, alkoxy, cycloalkyl, cycloalkoxy, phenyl, phenoxy, benzyl, benzyloxy, heteroaromatic or heterocyclic group for the manufacture of a medicament for treating a subject for a bacterial infection.

Another embodiment is a pharmaceutical composition comprising a pharmaceutically acceptable carrier or diluent and the compound represented by structural formula **I'**, or a pharmaceutically acceptable salt, solvate, or hydrate thereof:

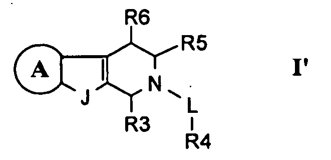

In structural formula **I'**, the variables Ring **A**, J, L, R2, R2', R3, and R5 are as provided above for structural formula **I**.

In structural formula **I'**, R4 is an aryl, biphenyl, heteroaryl, aryl-heteroaryl, aralkyl, heteroaralkyl, C1-C8 aliphatic, C3-C7 cycloalkyl, C5-C7 cycloaliphatic, or a 3-7 membered non-aromatic heterocyclic group. The group represented by R4 in structural formula **I'** is substituted as provided above for R4 in structural formula **I**.

The invention is useful for treating (therapeutically or prophylactically) bacterial infections, particularly infections caused by bacteria that depend on the CoA biosynthesis pathway, and more particularly, infections caused by bacteria that express the PPAT enzyme. Furthermore, it is useful against bacteria that have developed antibiotic resistance, especially multiple drug resistant strains, because it is believed to act through a different mechanism than existing, widely used antibiotics.

### DETAILED DESCRIPTION OF THE INVENTION

The invention is generally related to the use of a compound and to pharmaceutical compositions for treating and preventing bacterial infections. In particular, the invention relates to substituted tetrahydro-β-carboline derivatives that are PPAT inhibitors.

In a preferred embodiment, the compound is represented by structural formula **II'**: 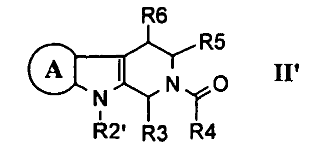 In structural formula **II'**, R3 is -H or C1-C3 alkyl, and the remainder of the variables are as described above for structural formulas **I** and **I'**.

In another preferred embodiment, the compound is represented by structural formula **II**: 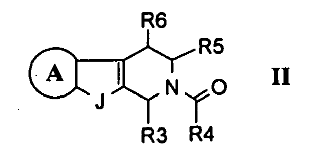 wherein J is -NR2-; R2 is -H or C1-C3 alkyl; and the remainder of the variables are as described above for structural formulas **I** and **I'**.

In yet another preferred embodiment, the compound is represented by structural formula **III**:

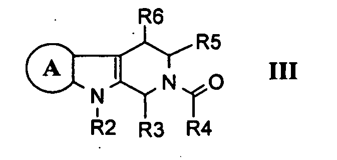

Ring A in structural formulas **II**, **II'**, and **III** is an optionally substituted aryl or heteroaryl group, for example, an optionally substituted phenyl, pyridyl, pyrimidyl, pyrazyl, furanyl, pyrrolyl, thienyl, oxazolyl, isooxazolyl, thiazolyl, isothiazolyl, or imidazolyl group. Preferably, Ring **A** is an optionally substituted six-membered aryl or heteroaryl group, or more preferably, an optionally substituted phenyl, pyrimidyl, or pyridyl group. Most preferably, Ring **A** is an optionally substituted phenyl group.

Suitable optional substituents for substitutable ring atoms in Ring **A** are provided herein below in the section describing substituents for aryl and heteroaryl groups. More preferably, Ring **A** is optionally, independently, substituted at any substitutable ring atom with R1. Each R1 is independently halogen, -CN, -NO₂, -CF₃, -OCF₃, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, or optionally substituted aryl, aralkyl or C1-C5 alkyl. In the preceding, s is from 0 to 5, each R^{d} and R^{f} is independently -H, aryl, aralkyl, C1-C5 alkyl, or C1-C5 haloalkyl, and each R^{e} is independently -H, aryl, aralkyl, or C1-C5 alkyl, or NR^{e}₂ is a nonaromatic heterocyclic group, for example, piperidinyl, morpholinyl, and the like. More preferably, R1 is halogen, -CN, -NO₂, -CF₃, -OCF₃, -OR^{d}, -(CO)R^{d}, -(CO)OR^{d}, -O(CO)R^{d}, -CONR^{e}₂, -O(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, or optionally substituted aryl, aralkyl or C1-C5 alkyl. Even more preferably, R1 is -H, -OH, -F, -CH₃, -CF₃, -OCH₃, or -OCF₃. Most preferably, R1 is -H.

The remainder of the variables in structural formula **III** are as described above for structural formula **II**.

More preferably, the compound is represented by structural formula **IV**:

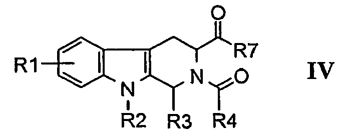

The variables R1, R2, R, and R4 in structural formula **IV** are as described above for structural formula **III**. R7 is R7 is-OR^{o} or -NR^{p}₂. R^{o} is -H or optionally substituted aryl, aroyl, aralkyl, aralkanoyl, C1-C5 alkyl, or C1-C5 alkanoyl; and each R^{p} is independently -H, C1-C5 alkyl, aryl, or aralkyl, or NR^{p}₂ is a nonaromatic heterocyclic group.

Even more preferably, the compound is represented by one of structural formulas **V** to **XI**: 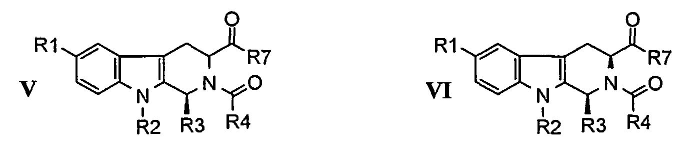 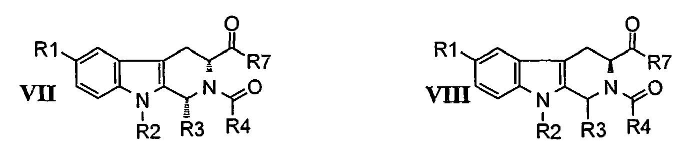 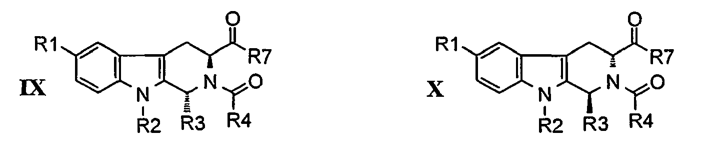 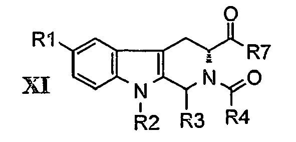
R1 in structural formulas **IV** to **XI** is as provided above for Ring **A**.
R2 in structural formulas **III** to **XI** is as provided above for structural formulas **I, I'**, and **II**. More preferably, R2 is -H, methyl, or ethyl, even more preferably, -H or methyl, and most preferably, -H.
R3 in structural formulas **III** to **XI** is an optionally substituted phenyl, pyridyl, benzo[1,3]dioxolyl, 2,3-dihydro-benzo[1,4]dioxinyl, pyrimidyl, pyrazyl, furanyl, pyrrolyl, thienyl, oxazolyl, isooxazolyl, thiazolyl, isothiazolyl, imidazolyl naphthyl, quinolinyl, biphenyl, benzopyrimidyl, benzopyrazyl, benzofuranyl, indolyl, benzothienyl, benzoxazolyl, benzoisooxazolyl, benzothiazolyl, benzoisothiazolyl, or benzimidazolyl group. Suitable optional substituents for the group represented by R3 are provided herein below in the section describing substituents for aryl and heteroaryl groups.

More preferably, R3 in structural formulas **III** to **XI** is represented by one of structural formulas **R3-i** to **R3-v**:

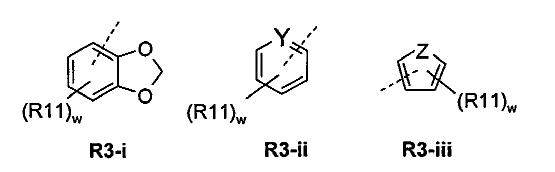

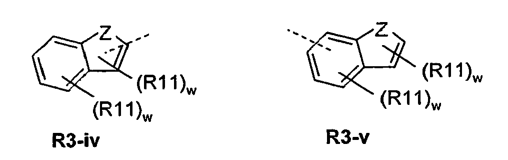

In structural formulas **R3-i** to **R3-v**, Y is -N-, -CH-, or -CR11-. Z is-NR^{z}-, -S-, or -O-, wherein R^{z} is -H or C1-C3 alkyl, more preferably -H or methyl, or most preferably -H. The variable w is 0, 1, 2, or 3. Each R11 is independently halogen, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ(CO)NR^{m}₂, NRⁿ(CO)OR¹, -NRⁿCOR¹, -(SO₂)NR^{m}₂, -NRⁿSO₂R¹, -(CH₂)ᵤNR¹₂, or optionally substituted aryl, aralkyl, or C1-C5 alkyl. In the preceding, u is 0 to 5, each R¹ and Rⁿ is independently-H, aryl, or aralkyl, C1-C5 alkyl, or C1-C5 haloalkyl, and each R^{m} is independently -H, aryl, aralkyl, or C1-C5 alkyl, or NR^{m}₂ is a nonaromatic heterocyclic group.

Even more preferably, R3 in structural formulas **III** to **XI** is represented by one of structural formulas **R3-i'** to **R3-v'**:

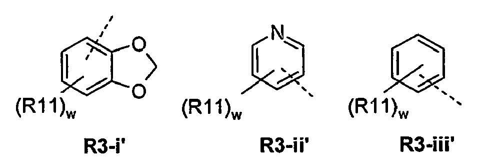

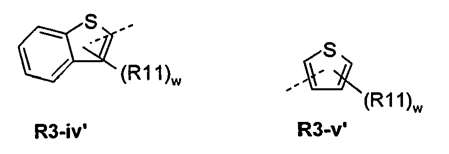

In structural formulas **R3-i'** to **R3-v'**, w is 0, 1, 2, or 3, and each R11 is independently -OH, -NO₂, -F, -Cl, -Br, C1-C4 alkyl, C1-C4 alkoxy, -CF₃, or-OCF_{3.}

Still more preferably, R3 is represented by one of structural formulas **R3^{a}** to **R3^{r}**:

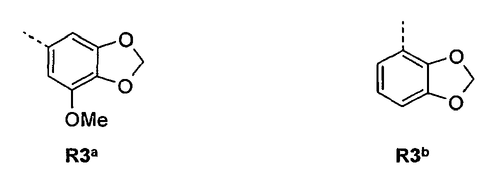

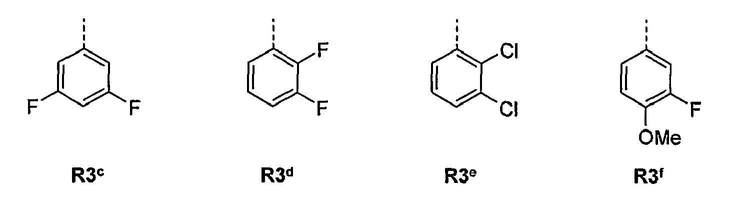

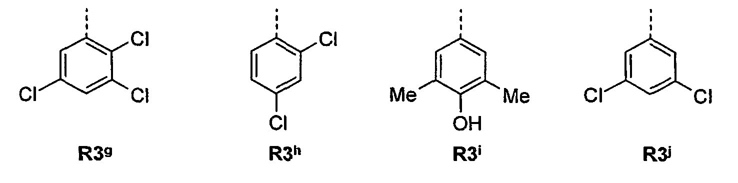

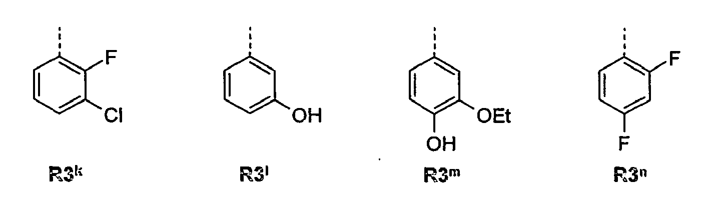

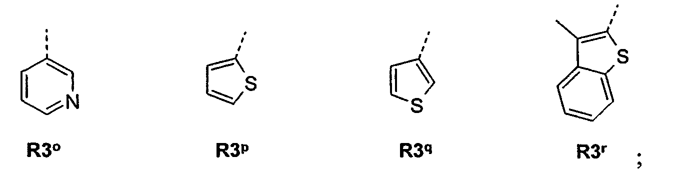

Most preferably, R3 is represented by structural formula **R3^{d}, R3^{e},** or **R3^{f}**.

R4 in structural formulas **II** to **XI** is as provided above in **I** for the method or as provided above in **I'** for the pharmaceutical composition and the compound, and is optionally further substituted as described below in the section describing suitable substituents for aryl, heteroaryl, aliphatic, and cycloalkyl groups. More preferably, R4 is a substituted phenyl, pyridyl, pyrimidyl, pyrazyl, naphthyl, biphenyl, phenyl-pyridyl, quinolinyl, benzopyrimidyl, benzopyrazyl, cyclopropyl, cyclobutyl, cyclopentyl, cyclopentenyl, cyclohexyl, cyclohexenyl, or a C2-C8 alkenyl group.

More preferably, R4 is represented by one of structural formulas **R4-i** to **R4-vii**:

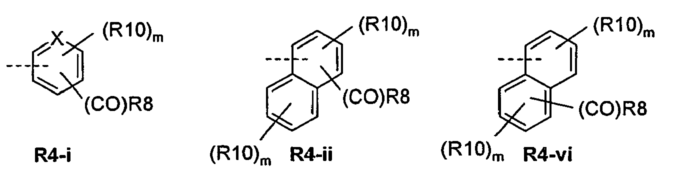

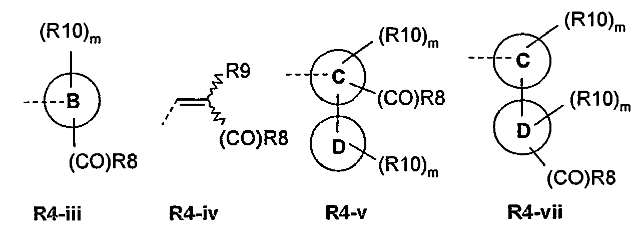

In structural formulas **R4-i** to **R4-vii**, each m is independently 0, 1, 2, or 3 and X is -N-, -CH-, or -CR10-. Ring **B** is C3-C6 cycloalkyl or C3-C6 cycloalkenyl. For the compound of the method, Rings **C** and **D** are each independently aryl or heteroaryl; for the compound and the compound of the pharmaceutical composition, Ring **C** is phenyl and Ring **D** is aryl or heteroaryl. R8 is-OR^{q} or NR^{r}₂. R9 is -H, aryl, aralkyl, or C1-C6 aliphatic. Each R10 is independently halogen, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -NR^{k}(CO)NR^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂, or optionally substituted aryl, aralkyl or C1-C5 alkyl. The variable t is 0 to 5 and each Rⁱ and R^{k} is independently -H, aryl, aralkyl, C1-C5 alkyl, or C1-C5 haloalkyl. Each R^{j} and R^{r} is independently -H, aryl, aralkyl, or C1-C5 alkyl, or each NR^{j}₂ and NR^{r}₂ is independently a nonaromatic heterocyclic group. R^{q} is -H or optionally substituted aryl, aroyl, aralkyl, aralkanoyl, C1-C5 alkyl, or C1-C5 alkanoyl.

Even more preferably, R4 is represented by one of structural formulas **R4-i'** to **R4-vii'**:

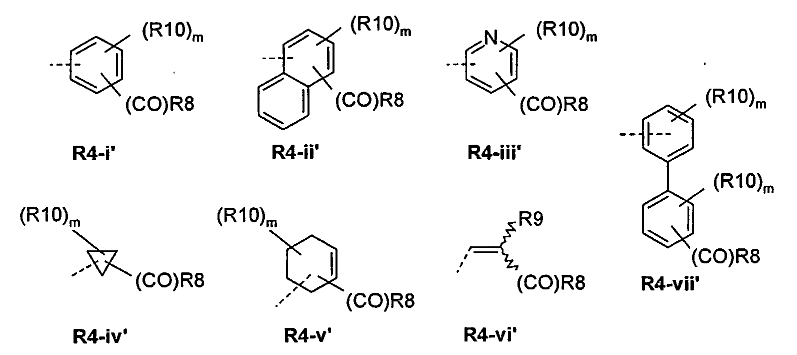

In structural formulas **R4-i'** to **R4-vii'**, each m is independently 0, 1, 2, or 3. **R8** is -OH, C1-C5 alkoxy, or C1-C5 alkanoyloxy, R9 is -H or C1-C6 aliphatic; and each R10 is independently -OR, -NO₂, -F, -Cl, -Br, C1-C4 alkyl, C1-C4 alkoxy, -CF₃, or -OCF₃.

Still more preferably, R4 is represented by one of structural formulas **R4^{a}** to **R4^{q}**:

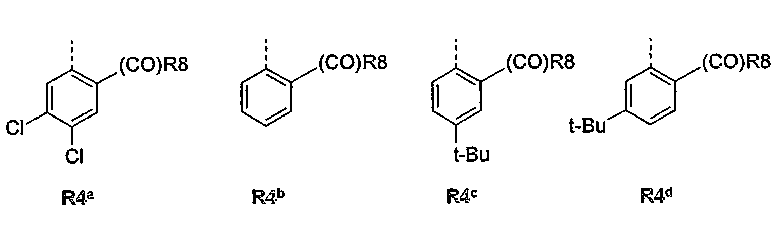

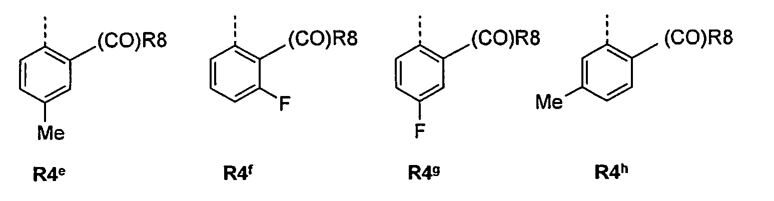

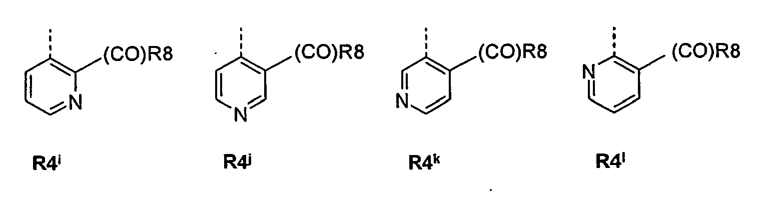

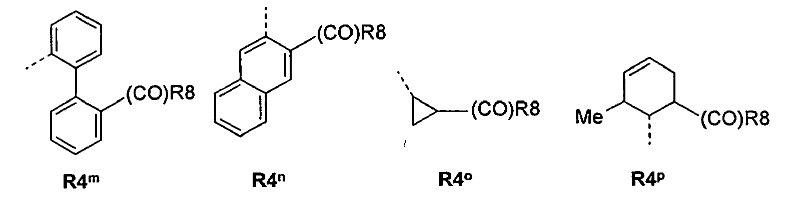

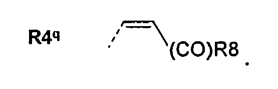

Most preferably, R4 is represented by structural formula **R4^{a}, R4^{c},** or **R4^{e}**.

In **R4^{a}** to **R4^{q}** and structural formulas **V** to **XI**, R8 is as provided above, or more preferably, is -NR^{y}₂, -OH, C1-C5 alkoxy, or C1-C5 alkanoyloxy, wherein each R^{y} is independently -H or C1-C3 alkyl. Even more preferably, R8 is -OH or C1-C4 alkoxy, or still more preferably, -OH, -OCH₃, or -OCH₂CH₃. Most preferably, R8 is -OCH₃ or -OCH₂CH₃.

R7is as provided above, or more preferably, is -NR^{x}₂, -OH, C1-C5 alkoxy, or C1-C5 alkanoyloxy, wherein each R^{x} is independently -H or C1-C3 alkyl. Even more preferably, R7 is -OH or C1-C4 alkoxy, or still more preferably, -OH, -OCH₃, or -OCH₂CH₃. Most preferably, R7 is -OCH₃ or -OCH₂CH₃.

In preferred embodiments, in structural formulas V to XI, one of R7 or R8 is - OH, and the other is -OCH₃ or -OCH₂CH₃. In another embodiment, R7 and R8 are -OH. In a preferred embodiment, R7 and R8 are independently -OCH₃ or -OCH₂CH₃.

In any one of structural formulas **I** to **XI** and **I'**, R3 is represented by one of structural formulas **R3-i** to **R3-v** or R4 is represented by one of structural formulas **R4-i** to **R4-vii**. More preferably, R3 is represented by one of structural formulas **R3-i** to **R3-v** and R4 is represented by one of structural formulas **R4-i** to **R4-vii**. In still another embodiment, in any one of structural formulas **I** to **XI** and **I'**, R3 is represented by one of structural formulas **R3-i'** to **R3-v'** or R4 is represented by one of structural formulas **R4-i'** to **R4-vii'**. More preferably, R3 is represented by one of structural formulas **R3-i'** to **R3-v'** and R4 is represented by one of structural formulas **R4-i'** to **R4-vii'**. In another preferred embodiment, for any one of structural formulas **I** to **XI** and **I'**, either R3 is represented by one of structural formulas **R3^{a}** to **R3^{r}**, or **R4** is represented by one of structural formulas **R4^{a}** to **R4^{q}**. Preferably, **R3** is represented by one of structural formulas **R3^{a}** to **R3^{r},** and R4 is represented by one of structural formulas R4^{a} to R4^{q}. More preferably, R3 is represented by structural formula **R3^{d}, R3^{e},** or **R3^{f},** or R4 is represented by structural formula **R4^{a}, R4^{c},** or **R4^{e}.** Even more preferably, R3 is represented by structural formula **R3^{d}, R3^{e},** or **R3^{f}** and R4 is represented by structural formula **R4^{a}, R4^{c},** or **R4^{e}.**

In other embodiments the compound, the compound of the method, and the compound of the pharmaceutical composition are each represented by one of compounds 1-184, as provided in Table 1.

A "subject" includes mammals, e.g., humans, companion animals (e.g., dogs, cats, birds, aquarium fish, reptiles, and the like), farm animals (e.g., cows, sheep, pigs, horses, fowl, farm-raised fish and the like) and laboratory animals (e.g., rats, mice, guinea pigs, birds, aquarium fish, reptiles, and the like). Alternatively, the subject is a warm-blooded animal. More preferably, the subject is a mammal. Most preferably, the subject is human.

A subject in need of treatment has a bacterial infection (or has been exposed to an infectious environment where bacteria are present, e.g., in a hospital) the symptoms of which may be alleviated by administering an effective amount of the disclosed tetrahydro-β-carboline derivatives. For example, a subject in need of treatment can have an infection for which the disclosed tetrahydro-β-carboline derivatives can be administered as a treatment. In another example, a subject in need of treatment can have an open wound or burn injury, or can have a compromised immune system, for which the disclosed PPAT inhibitors can be administered as a prophylactic. Thus, a subject can be treated therapeutically or prophylactically. More preferably, a subject is treated therapeutically.

Typically, the subject is treated for a bacterial infection caused by a bacteria of a genus selected from *Allochromatium, Acinetobacter, Bacillus, Campylobacter, Chlamydia, Chlamydophila, Clostridium, Citrobacter, Escherichia, Enterobacter, Enterococcus, Francisella, Haemophilus, Helicobacter, Klebsiella, Listeria, Moraxella, Mycobacterium, Neisseria, Proteus, Pseudomonas, Salmonella, Serratia, Shigella, Stenotrophomonas, Staphyloccocus, Streptococcus, Synechococcus, Vibrio,* and *Yersina.*

More preferably, the subject is treated for a bacterial infection from *Allochromatium vinosum, Acinetobacter baumanii, Bacillus anthracis, Campylobacter jejuni, Chlamydia trachomatis, Chlamydia pneumoniae, Clostridium* spp., *Citrobacter* spp., *Escherichia coli, Enterobacter* spp., *Enterococcus faecalis., Enterococcus faecium, Francisella tularensis, Haemophilus influenzae, Helicobacter pylori, Klebsiella* spp., *Listeria monocytogenes, Moraxella catarrhalis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneumoniae, Streptococcus pyogenes, Streptococcus agalactiae, Yersina pestis,* and *Yersina* enterocolitica, and the like.

Preferably, the subject is treated for a bacterial infection caused by a bacterium that expresses a PPAT protein. As used herein, a PPAT protein is a phosphopantetheine adenytransferase enzyme, i.e., systematic name ATP:pantetheine-4'-phosphate adenylyltransferase, IUBMB systematic classification EC 2.7.7.3, (see International Union of Biochemistry and Molecular Biology, www.chem.qmul.ac.uk/iubmb/).

In one embodiment, a subject is also concurrently treated for a fungal infection, for example, a fungal infection caused by a pathogenic dermatophyte, e.g., a species of the genera *Trichophyton, Tinea, Microsporum, Epidermophyton* and the like; or a pathogenic filamentous fungus, e.g., a species of genera such as *Aspergillus, Histoplasma, Cryptococcus, Microsporum,* and the like; or a pathogenic non-filamentous fungus, e.g., a yeast, for example, a species of the genera *Candida, Malassezia, Trichosporon, Rhodotorula, Torulopsis, Blastomyces, Paracoccidioides, Coccidioides,* and the like. Preferably, the subject is concurrently treated for a fungal infection resulting from a species of the genera *Aspergillus* or *Trichophyton.* Species of *Trichophyton* include, for example, *T. mentagrophytes, T. rubrum, T. schoenleinii, T. tonsurans, T. verrucosum,* and *T. violaceum.* Species of *Aspergillus* include, for example, *A. fumigatus, A. flavus, A. niger, A. amstelodami, A. candidus, A. carneus, A. nidulans, A oryzae, A. restrictus, A. sydowi, A. terreus, A. ustus, A. versicolor, A. caesiellus, A. clavatus, A. avenaceus,* and *A. deflectus.* More preferably, the subject is concurrently treated therapeutically for a fungal infection caused by a species of the genus *Aspergillus* selected from *A. fumigatus, A. flavus, A. niger, A. amstelodami, A. candidus, A. carneus, A. nidulans, A oryzae, A. restrictus, A. sydowi, A. terreus, A. ustus, A. versicolor, A. caesiellus, A. clavatus, A. avenaceus,* and *A. deflectus.* Even more preferably the subject is concurrently treated therapeutically for a fungal infection caused by *Aspergillus fumigatus* or *Aspergillus niger,* and most preferably, *Aspergillus fumigatus.*

An "effective amount" of a compound of the disclosed invention is the quantity which, when administered to a subject in need of treatment, improves the prognosis of the subject, e.g., delays the onset of and/or reduces the severity of one or more of the subject's symptoms associated with a bacterial infection. The amount of the disclosed compound to be administered to a subject will depend on the particular disease, the mode of administration, co-administered compounds, if any, and the characteristics of the subject, such as general health, other diseases, age, sex, genotype, body weight and tolerance to drugs. The skilled artisan will be able to determine appropriate dosages depending on these and other factors. Effective amounts of the disclosed compounds typically range between about 0.01 mg/kg per day and about 100 mg/kg per day, and preferably between 0.1 mg/kg per day and about 10 mg/kg/day. Techniques for administration of the disclosed compounds of the invention can be found in Remington: the Science and Practice of Pharmacy, 19th edition, Mack Publishing Co., Easton, PA (1995), the entire teachings of which are incorporated herein by reference.

A "pharmaceutically acceptable salt" of the disclosed compound is a product of the disclosed compound that contains an ionic bond, and is typically produced by reacting the disclosed compound with either an acid or a base, suitable for administering to a subject.

For example, an acid salt of a compound containing an amine or other basic group can be obtained by reacting the compound with a suitable organic or inorganic acid, such as hydrogen chloride, hydrogen bromide, acetic acid, perchloric acid and the like. Compounds with a quaternary ammonium group also contain a counteranion such as chloride, bromide, iodide, acetate, perchlorate and the like. Other examples of such salts include hydrochlorides, hydrobromides, sulfates, methanesulfonates, nitrates, maleates, acetates, citrates, fumarates, tartrates (e.g. (+)-tartrates, (-)-tartrates or mixtures thereof including racemic mixtures), succinates, benzoates and salts with amino acids such as glutamic acid.

Salts of compounds containing a carboxylic acid or other acidic functional group can be prepared by reacting with a suitable base. Such a pharmaceutically acceptable salt may be made with a base which affords a pharmaceutically acceptable cation, which includes alkali metal salts (especially sodium and potassium), alkaline earth metal salts (especially calcium and magnesium), aluminum salts and ammonium salts, as well as salts made from physiologically acceptable organic bases such as trimethylamine, triethylamine, morpholine, pyridine, piperidine, picoline, dicyclohexylamine, N, N'-dibenzylethylenediamine, 2-hydroxyethylamine, bis-(2-hydroxyethyl)amine, tri-(2-hydroxyethyl)amine, procaine, dibenzylpiperidine, N-benzyl-β-phenethylamine, dehydroabietylamine, N,N'-bisdehydroabietylamine, glucamine, N-methylglucamine, collidine, quinine, quinoline, and basic amino acid such as lysine and arginine.

Certain compounds and their salts may also exist in the form of solvates, for example hydrates, and the present invention includes each solvate and mixtures thereof.

As used herein, a "pharmaceutical composition" is a formulation containing the disclosed compounds in a form suitable for administration to a subject. The pharmaceutical composition can be in bulk or in unit dosage form. The unit dosage form can be in any of a variety of forms, including, for example, a capsule, an IV bag, a tablet, a single pump on an aerosol inhaler, or a vial. The quantity of active ingredient (i.e., a formulation of the disclosed compound or salts thereof) in a unit dose of composition is an effective amount and may be varied according to the particular treatment involved. It may be appreciated that it may be necessary to make routine variations to the dosage depending on the age and condition of the patient. The dosage will also depend on the route of administration. A variety of routes are contemplated, including topical, oral, pulmonary, rectal, vaginal, parenternal, transdermal, subcutaneous, intravenous, intramuscular, intraperitoneal and intranasal.

The compounds described herein, and the pharmaceutically acceptable salts thereof can be used in pharmaceutical preparations in combination with a pharmaceutically acceptable carrier or diluent. Suitable pharmaceutically acceptable carriers include inert solid fillers or diluents and sterile aqueous or organic solutions. The compounds will be present in such pharmaceutical compositions in amounts sufficient to provide the desired dosage amount in the range described herein. Techniques for formulation and administration of the disclosed compounds of the invention can be found in *Remington: the Science and Practice of Pharmacy,* above.

For oral administration, the disclosed compounds or salts thereof can be combined with a suitable solid or liquid carrier or diluent to form capsules, tablets, pills, powders, syrups, solutions, suspensions and the like.

The tablets, pills, capsules, and the like contain from about 1 to about 99 weight percent of the active ingredient and a binder such as gum tragacanth, acacias, corn starch or gelatin; excipients such as dicalcium phosphate; a disintegrating agent such as corn starch, potato starch or alginic acid; a lubricant such as magnesium stearate; and/or a sweetening agent such as sucrose, lactose or saccharin. When a dosage unit form is a capsule, it may contain, in addition to materials of the above type, a liquid carrier such as a fatty oil.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor, and the like.

For parental administration of the disclosed compounds, or salts, solvates, or hydrates thereof, can be combined with sterile aqueous or organic media to form injectable solutions or suspensions. For example, solutions in sesame or peanut oil, aqueous propylene glycol and the like can be used, as well as aqueous solutions of water-soluble pharmaceutically-acceptable salts of the compounds. Dispersions can also be prepared in glycerol, liquid polyethylene glycols and mixtures thereof in oils. Under ordinary conditions of storage and use, these preparations contain a preservative to prevent the growth of microorganisms.

In addition to the formulations previously described, the compounds may also be formulated as a depot preparation. Suitable formulations of this type include biocompatible and biodegradable polymeric hydrogel formulations using crosslinked or water insoluble polysaccharide formulations, polymerizable polyethylene oxide formulations, impregnated membranes, and the like. Such long acting formulations may be administered by implantation or transcutaneous delivery (for example subcutaneously or intramuscularly), intramuscular injection or a transdermal patch. Preferably, they are implanted in, or applied to, the microenvironment of an affected organ or tissue, for example, a membrane impregnated with the disclosed compound can be applied to an open wound or bum injury. Thus, for example, the compounds may be formulated with suitable polymeric or hydrophobic materials, for example, as an emulsion in an acceptable oil, or ion exchange resins, or as sparingly soluble derivatives, for example, as a sparingly soluble salt.

For topical administration, suitable formulations may include biocompatible oil, wax, gel, powder, polymer, or other liquid or solid carriers. Such formulations may be administered by applying directly to affected tissues, for example, a liquid formulation to treat infection of conjunctival tissue can be administered dropwise to the subject's eye, a cream formulation can be administer to a wound site, or a bandage may be impregnated with a formulation, and the like.

For rectal administration, suitable pharmaceutical compositions are, for example, topical preparations, suppositories or enemas.

For vaginal administration, suitable pharmaceutical compositions are, for example, topical preparations, pessaries, tampons, creams, gels, pastes, foams or sprays.

In addition, the compounds may also be formulated to deliver the active agent by pulmonary administration, e.g., administration of an aerosol formulation containing the active agent from, for example, a manual pump spray, nebulizer or pressurized metered-dose inhaler. Suitable formulations of this type can also include other agents, such as antistatic agents, to maintain the disclosed compounds as effective aerosols.

The term "pulmonary" as used herein refers to any part, tissue or organ whose primary function is gas exchange with the external environment, i.e., O₂/CO₂ exchange, within a patient. "Pulmonary" typically refers to the tissues of the respiratory tract. Thus, the phrase "pulmonary administration" refers to administering the formulations described herein to any part, tissue or organ whose primary function is gas exchange with the external environment (e.g., mouth, nose, pharynx, oropharynx, laryngopharynx, larynx, trachea, carina, bronchi, bronchioles, alveoli). For purposes of the present invention, "pulmonary" is also meant to include a tissue or cavity that is contingent to the respiratory tract, in particular, the sinuses.

A drug delivery device for delivering aerosols comprises a suitable aerosol canister with a metering valve containing a pharmaceutical aerosol formulation as described and an actuator housing adapted to hold the canister and allow for drug delivery. The canister in the drug delivery device has a head space representing greater than about 15% of the total volume of the canister. Often, the polymer intended for pulmonary administration is dissolved, suspended or emulsified in a mixture of a solvent, surfactant and propellant. The mixture is maintained under pressure in a canister that has been sealed with a metering valve.

For nasal administration, either a solid or a liquid carrier can be used. The solid carrier includes a coarse powder having particle size in the range of, for example, from about 20 to about 500 microns and such formulation is administered by rapid inhalation through the nasal passages. Where the liquid carrier is used, the formulation may be administered as a nasal spray or drops and may include oil or aqueous solutions of the active ingredients.

In addition to the formulations described above, a formulation can optionally include, or be co-administered with one or more additional drugs, e.g., other antibiotics, anti-inflammatories, antifungals, antivirals, immunomodulators, antiprotozoals, steroids, decongestants, bronchodialators, and the like. For example, the disclosed compound can be co-administered with drugs such as such as ibuprofen, prednisone (corticosteroid) pentoxifylline, Amphotericin B, Fluconazole, Ketoconazol, Itraconazol, penicillin, ampicillin, amoxicillin, and the like. The formulation may also contain preserving agents, solubilizing agents, chemical buffers, surfactants, emulsifiers, colorants, odorants and sweeteners.

The term "derivative", e.g., in the term "tetrahydro-β-carboline derivatives", refers to compounds that have a common core structure, and are substituted with various groups as described herein. For example, all of the compounds represented by structural formulas **I** to **XI** are tetrahydro-β-carboline derivatives, and have structural formula **I** as a common core.

In the structural formulas depicted herein, a dashed line indicates a bond by which the depicted or moiety or group is connected to the remainder of the molecule. For example, the dashed line in **R4-i** indicates the bond that connects the depicted group to another structural formula, for example, one of structural formulas **II** to **X** at the position indicated by R4. A dashed or solid line across a bond in a ring, for example, the solid line from R11 in **R4-i**, indicates that the represented bond can be connected to any substitutable atom in the ring. A zig-zag line, for example, the zig-zag line connecting R9 and (CO)R8 in **R4-iv** indicates either cis or trans arrangement of the respective substituents with respect to the bond represented by the dashed line.

The term "aryl" group, (e.g., the aryl groups represented by Ring **A**, R3, and R4) refers to carbocyclic aromatic groups such as phenyl, naphthyl, and anthracyl. The term "heteroaryl" group (e.g., the heteroaromatic groups represented by Ring **A**, R3, and R4) refers to heteroaromatic groups such as imidazolyl, isoimidazolyl, thienyl, furanyl, pyridyl, pyrimidyl, pyranyl, pyrazolyl, pyrrolyl, pyrazinyl, thiazolyl, isothiazolyl, oxazolyl, isooxazolyl, 1,2,3-triazolyl, 1,2,4-triazolyl, and tetrazolyl. As used herein, a "heteroaryl" group is a 5 membered carbocyclic ring containing at least one N, S, or O atom and two double bonds, or a 6 membered carbocyclic ring containing at least one N, S, or O atom and three double bonds.

The term "nonaromatic heterocyclic" (e.g., the nonaromatic heterocyclic groups represented by R4 in structural formula **I**) refers to non-aromatic ring systems typically having four to eight members, preferably five to six, in which one or more ring carbons, preferably one to four, are each replaced by a heteroatom such as N, O, or S. Examples of non-aromatic heterocyclic rings include 3-tetrahydrofuranyl, 2-tetrahydropyranyl, 3-tetrahydropyranyl, 4-tetrahydropyranyl, [1,3]-dioxalanyl, [1,3]-dithiolanyl, [1,3]-dioxanyl, 2-tetrahydrothiophenyl, 3-tetrahydrothiophenyl, 2-morpholinyl, 3-morpholinyl, 4-morpholinyl, 2-thiomorpholinyl, 3-thiomorpholinyl, 4-thiomorpholinyl, 1-pyrrolidinyl, 2-pyrrolidinyl, 3-pyrorolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, and 1-pthalimidinyl.

The disclosed compounds can contain one or more chiral centers. For example, in structural formula **I**, the carbons substituted with R3, R5, and R6 can each be a chiral center. The presence of chiral centers in a molecule gives rise to stereoisomers. For example, a pair of optical isomers, referred to as "enantiomers", exist for every chiral center in a molecule. For example, structural formulas **VIII** and **XI** represent a pair of enantiomers defined by the chiral center at the carbon substituted with -(CO)R7. A pair of diastereomers exist for every chiral center in a compound having two or more chiral centers. For example, structural formulas **VI** and **IX** are diastereomers defined by the chiral center at the carbon substituted with -(CO)R7, and **VII** and **X** are diastereomers defined by the chiral center at the carbon substituted with -R3. Where the structural formulas do not explicitly depict stereochemistry, for example in structural formula **I**, it is to be understood that these formulas encompass enantiomers free from the corresponding optical isomer, racemic mixtures, mixtures enriched in one enantiomer relative to its corresponding optical isomer, a diastereomer free of other diastereomers, a pair of diastereomers free from other diasteromeric pairs, mixtures of diasteromers, mixtures of diasteromeric pairs, mixtures of diasteromers in which one diastereomer is enriched relative to the other diastereomer(s) and mixtures of diasteromeric pairs in which one diastereomeric pair is enriched relative to the other diastereomeric pair(s).

The term "alkyl" (e.g., the alkyl groups represented by R1, R2, R^{a}, R^{b}, R^{c}, R^{d}, R^{e}, and R^{f}), used alone or as part of a larger moiety (e.g., aralkyl, alkoxy, alkylamino, alkylaminocarbonyl, haloalkyl), is a straight or branched non-aromatic hydrocarbon which is completely saturated. Typically, a straight or branched alkyl group has from 1 to about 10 carbon atoms, preferably from 1 to about 5 if not otherwise specified, Examples of suitable straight or branched alkyl group include methyl, ethyl, *n*-propyl, 2-propyl, *n*-butyl, *sec*-butyl, *tert*-butyl, pentyl, hexyl, heptyl or octyl. A C1-C10 straight or branched alkyl group or a C3-C8 cyclic alkyl group can also be referred to as a "lower alkyl" group. An "alkoxy" group refers to an alkyl group that is connected through an intervening oxygen atom, e.g., methoxy, ethoxy, 2-propyloxy, *tert*-butoxy, 2-butyloxy, 3-pentyloxy, and the like.

The terms "optionally halogenated alkyl", and "optionally halogenated alkoxy", as used herein, includes the respective group substituted with one or more of -F, -Cl, -Br, or -I.

The terms "alkanoyl", "aroyl", and the like, as used herein, indicates the respective group connected through an intervening carbonyl, for example, -(CO)CH₂CH₃, benzoyl, and the like. The terms "alkanoyloxy", "aroyloxy", and the like, as used herein, indicates the respective group connected through an intervening carboxylate, for example, -O(CO)CH₂CH₃, -O(CO)C₆H₅, and the like.

The term "cycloalkyl group" (e.g, the cycloalkyl groups represented by R4) is a cyclic alkyl group has from 3 to about 10 carbon atoms, preferably from 3 to about 7. Examples of suitable cycloalkyl groups include cyclopropyl, cyclobutyl, cyclopentyl, cyclohexyl, cycloheptyl and cyclooctyl. A "cycloalkoxy" group refers to a cycloalkyl group that is connected through an intervening oxygen atom, e.g., cyclopentyloxy, cyclohexyloxy, and the like.

The term "aliphatic" (e.g., the aliphatic groups represented by R4) includes branched and linear alkyl groups that contain one or more units of carbon-carbon unsaturation, i.e., carbon-carbon double or triple bonds. A cycloaliphatic group is a cyclic aliphatic group, for example, cyclohexenyl or cyclopentenyl.

The terms "aralkyl", "heteroaralkyl", "cycloalkylalkyl" "cycloaliphaticalkyl", and "nonaromatic heterocycloalkyl" refer to aryl, heteroaryl, cycloalkyl, cycloaliphatic, and nonaromatic heterocyclic groups, respectively, that are connected through an alkyl chain, e.g., benzyl, -CH₂H₂-pyridine, (3-cyclohexyl)propyl, and the like.

The terms biaryl, biheteroaryl, aryl-heteroaryl and heteroaryl-aryl, as used herein, indicate two aryl groups connected by a single covalent bond, two heteroaryl groups connected by a single covalent bond, an aryl and heteroaryl group connected by single covalent bond, and a heteroaryl and aryl group connected by a single covalent bond, respectively. Examples of biaryl, biheteroaryl, heteroaryl-aryl and aryl-heteroaryl groups include biphenyl, bipyridyl, pyrimidyl-phenyl, and phenyl-pyridyl, respectively. When an biaryl, biheteroaryl, heteroaryl-aryl or aryl-heteroaryl group is a substituent, as in the definition of R4 for structural formula **I**, the first recited group is bonded to the remainder of the molecule, i.e., "L" in structural formula **I**. For example, when R4 in structural formula **I** is a phenyl-pyridyl group, the phenyl of the phenyl-pyridyl group is bonded to L.

An "acyclic" group is a substituent that does not contain a ring. A "monocyclic" group contains only a single ring, for example, a phenyl ring that is not fused to another ring. A "polycyclic" group is a group that contains multiple fused rings, for example, naphthyl.

A "substitutable atom" is any atom such as nitrogen or carbon that is bonded through a single covalent bond to a hydrogen atom, wherein the hydrogen atom can be replaced with another group. A "substitutable ring atom" in an aromatic ring is any ring atom, e.g., a carbon or nitrogen, which is bonded by a single covalent bond to a hydrogen atom, wherein the hydrogen atom can be replaced with another group. For example, when Ring **A** is a phenyl ring fused to another aromatic group, there are 4 substitutable carbons, i.e., the non-fused carbons.

Suitable substituents are those that do not substantially interfere with the pharmaceutical activity of the disclosed compound. A compound or group can have one or more substituents, which can be identical or different. Examples of suitable substituents for a substitutable carbon atom in an alkyl, aliphatic, cycloalkyl, cycloaliphatic, non-aromatic heterocyclic, aryl, or heteroaryl group include -OH, halogen (-Br, -Cl, -I and -F), -R, -OR, -CH₂R, - CH₂CH₂R, -OCH₂R, -CH₂OR, -CH₂CH₂OR, -CH₂OC(O)R, -O-COR, -COR, -SR, -SCH₂R, - CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR (k is 0, 1 or 2) and -NH-C(=NH)-NH₂. Each R is independently an alkyl, cycloalkyl, benzyl, aromatic, heteroaromatic, or *N*-anilinyl group that is optionally substituted. Preferably, R is unsubstituted. In addition, -N(R)₂, taken together, can also form a substituted or unsubstituted heterocyclic group, such as pyrrolidinyl, piperidinyl, morpholinyl and thiomorpholinyl. Examples of substituents on group represented by R include amino, alkylamino, dialkylamino, aminocarbonyl, halogen, alkyl, alkylaminocarbonyl, dialkylaminocarbonyloxy, alkoxy, nitro, cyano, carboxy, alkoxycarbonyl, alkylcarbonyl, hydroxy, haloalkoxy, or haloalkyl.

Suitable substituents on the nitrogen of a heterocyclic group or heteroaromatic group include -R', -N(R')₂, -C(O)R', -CO₂ R', -C(O)C(O)R', -C(O)CH₂ C(O)R', -SO₂R', -SO₂ N(R')₂, -C(=S)N(R')₂, -C(=NH)-N(R')₂, and -NR' SO₂R'. R' is hydrogen, an alkyl, alkoxy, cycloalkyl, cycloalkoxy, phenyl, phenoxy, benzyl, benzyloxy, heteroaromatic, or heterocyclic group that is optionally substituted. Examples of substituents on the groups represented by R' include amino, alkylamino, dialkylamino, aminocarbonyl, halogen, alkyl, alkylaminocarbonyl, dialkylaminocarbonyloxy, alkoxy, nitro, cyano, carboxy, alkoxycarbonyl, alkylcarbonyl, hydroxy, haloalkoxy, or haloalkyl. Preferably, R' is unsubstituted.

### EXEMPLIFICATION

### Examples 1: Synthesis of PPAT inhibitors of structural formula I: Compound 4

The disclosed compounds can be prepared by standard methods starting from appropriate commercially available starting materials.

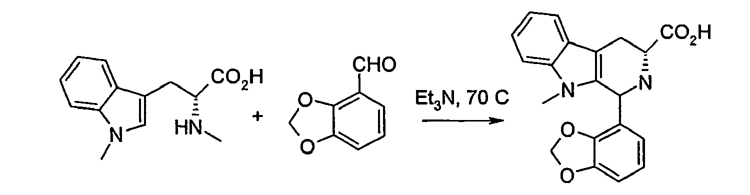

Step 1: A 3 mM solution of *N-*methyl-D-tryptophan in a 1:1 (v/v) acetic acid:H2O mixture was treated with 1 molar equivalent each of 2, 3-(methylenedioxy)-benzaldehyde and triethylamine. The mixture was heated at 70° C for 3 h and cooled to ambient temperature. The solvent was removed under reduced pressure, and the residue was dissolved in ethyl acetate, washed with 2X10 mL of water, and dried. The ethyl acetate was removed under reduced pressure to afford crude product.

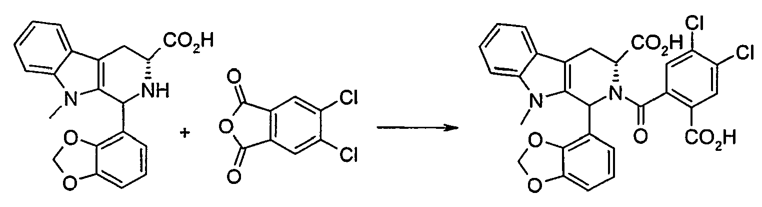

Step 2: Crude product from step 1 was dissolved in 5 mL of dimethyl sulfoxide (DMSO) and treated with 2 molar equivalents of dichloropthalic anhydride. The mixture was stirred at ambient temperature for 15 h, or until the reaction is completed as monitored by high pressure liquid chromatography (HPLC). The reaction mixture was then diluted with 15 mL of ethyl acetate and washed with 2X10 mL of water. The organic layer was separated, dried, and passed through a silica gel plug. Removal of the solvent under reduced pressure gave the final product, 1-Benzo[1,3]dioxol-4-yl-2-(2-carboxy-4,5-dichloro-benzoyl)-2,3,4,9-tetrahydro-1H-β-carboline-3-carboxylic acid, Compound 4.

Using the methods in the above example, compounds represented by structural formula **I**, i.e., Compounds 1-184 (Table 1) were prepared by starting from appropriate reagents.

Compounds containing ester or amide groups were prepared by well-known esterification or amidation methods respectively; see for example, Larock, R."Comprehensive Organic Transformations", 2nd Ed, Wiley, New York, NY, 1999 pp 1932-1949 and references therein, the entire teachings of which are incorporated herein by reference.

Compounds that are racemic, stereochemically enriched, or stereochemically pure can be prepared by an appropriate combination of methods selected from employing appropriate starting materials or reagents, crystallization, and chromatographic purification. See, for example, Ahuja, S. "Chiral Separations by Chromatography", American Chemical Society, 2000; Ahuja, S. "Chiral Separations: Applications and Technology", American Chemical Society, 1996, and references therein, the entire teachings of which are incorporated herein by reference.

**Table 1: Synthesis of PPAT Inhibitors of Structural Formula I***

| **#** | **Structure** | **#** | **Structure** | **#** | **Structure** |
|---|---|---|---|---|---|
| 1 | 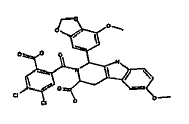 | 63 | 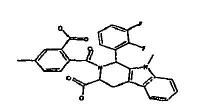 | 125 | 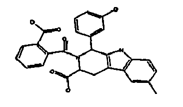 |
| 2 | 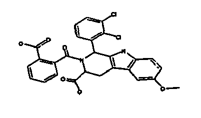 | 64 | 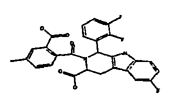 | 126 | 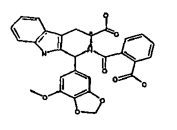 |
| 3 | 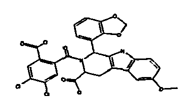 | 65 | 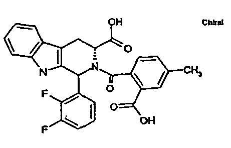 | 127 | 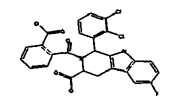 |
| 4 | 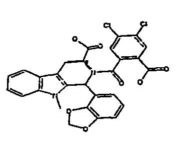 | 66 | 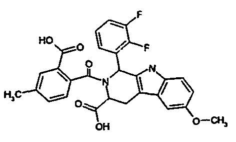 | 128 | 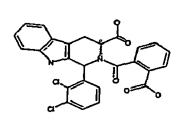 |
| 5 | 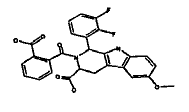 | 67 | 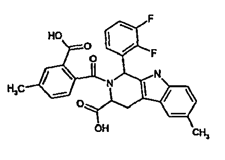 | 129 | 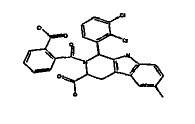 |
| 6 | 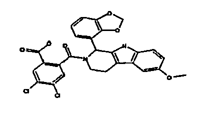 | 68 | 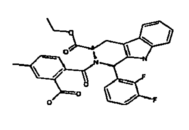 | 130 | 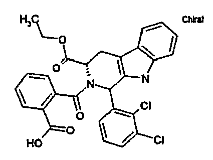 |
| 7 | 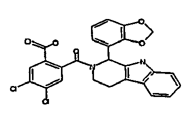 | 69 | 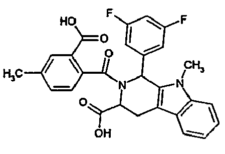 | 131 | 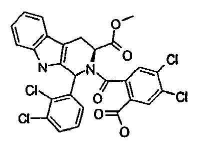 |
| 8 | 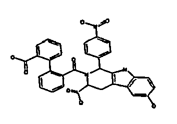 | 70 | 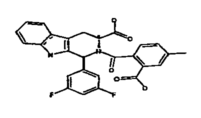 | 132 | 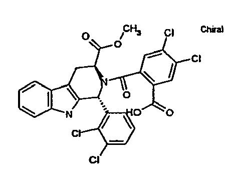 |
| 9 | 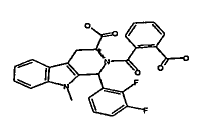 | 71 | 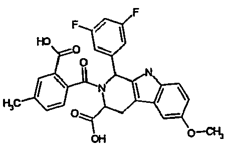 | 133 | 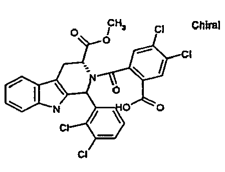 |
| 10 | 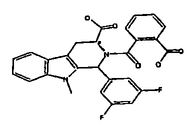 | 72 | 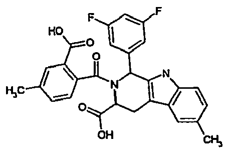 | 134 | 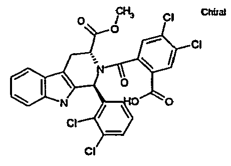 |
| 11 | 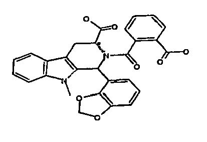 | 73 | 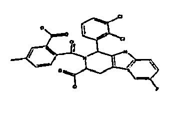 | 135 | 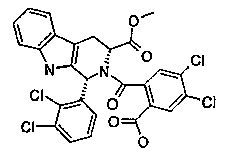 |
| 12 | 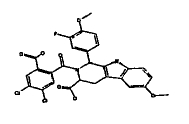 | 74 | 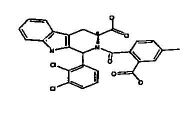 | 136 | 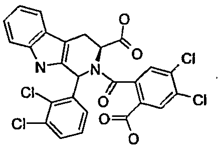 |
| 13 | 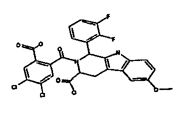 | 75 | 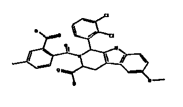 | 137 | 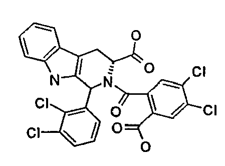 |
| 14 | 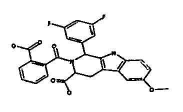 | 76 | 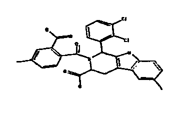 | 138 | 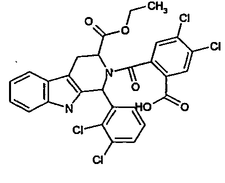 |
| 15 | 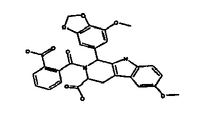 | 77 | 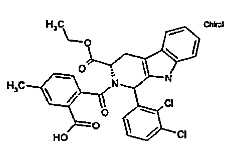 | 139 | 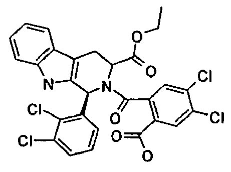 |
| 16 | 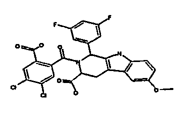 | 78 | 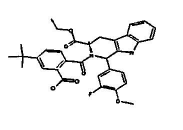 | 140 | 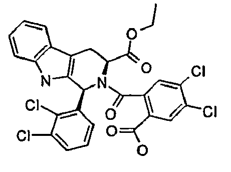 |
| 17 | 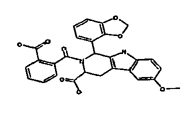 | 79 | 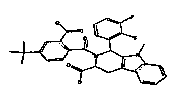 | 141 | 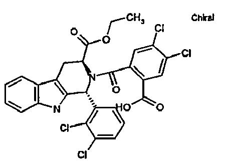 |
| 18 | 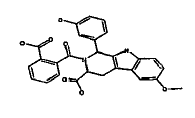 | 80 | 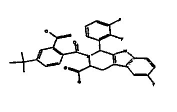 | 142 | 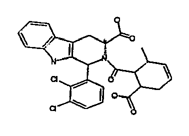 |
| 19 | 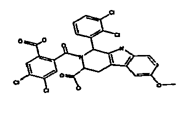 | 81 | 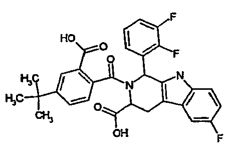 | 143 | 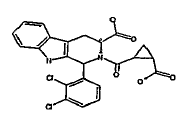 |
| 20 | 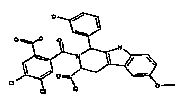 | 82 | 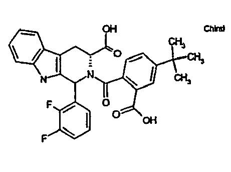 | 144 | 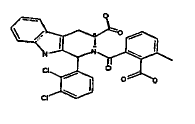 |
| 21 | 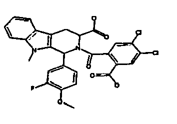 | 83 | 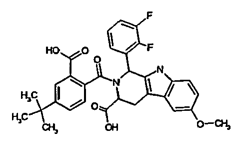 | 145 | 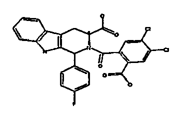 |
| 22 | 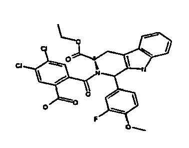 | 84 | 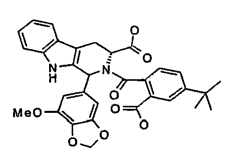 | 146 | 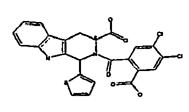 |
| 23 | 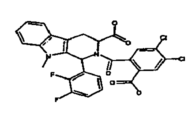 | 85 | 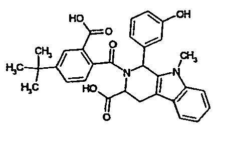 | 147 | 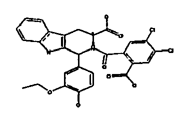 |
| 24 | 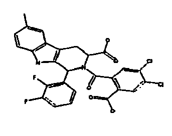 | 86 | 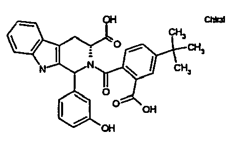 | 148 | 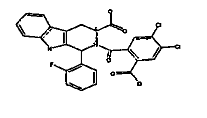 |
| 25 | 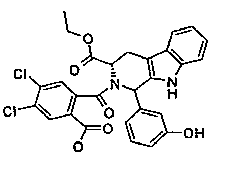 | 87 | 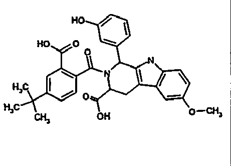 | 149 | 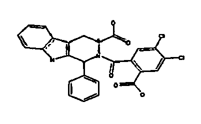 |
| 26 | 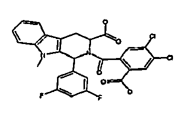 | 88 | 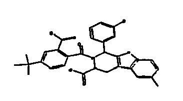 | 150 | 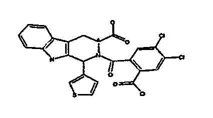 |
| 27 | 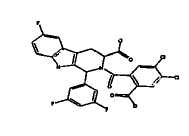 | 89 | 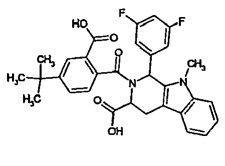 | 151 | 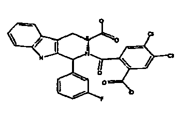 |
| 28 | 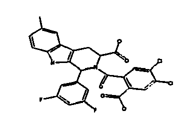 | 90 | 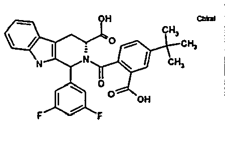 | 152 | 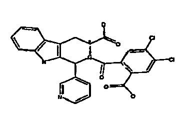 |
| 29 | 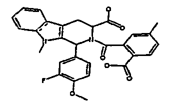 | 91 | 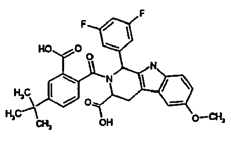 | 153 | 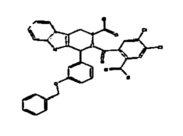 |
| 30 | 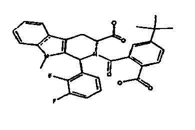 | 92 | 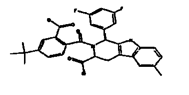 | 154 | 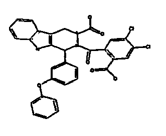 |
| 31 | 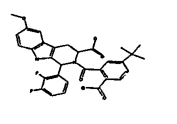 | 93 | 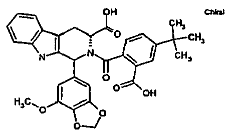 | 155 | 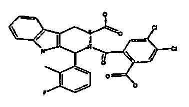 |
| 32 | 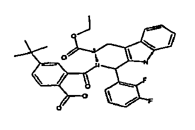 | 94 | 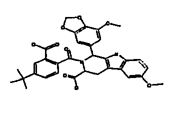 | 156 | 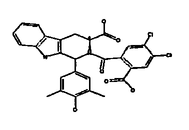 |
| 33 | 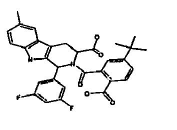 | 95 | 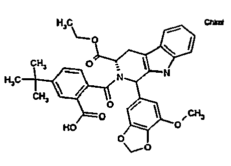 | 157 | 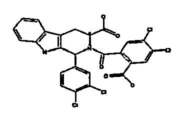 |
| 34 | 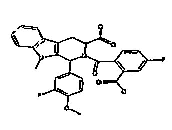 | 96 | 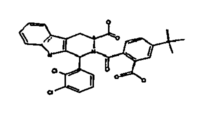 | 158 | 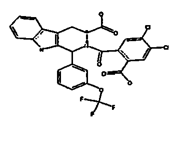 |
| 35 | 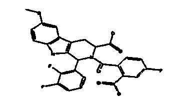 | 97 | 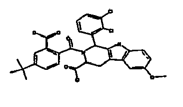 | 159 | 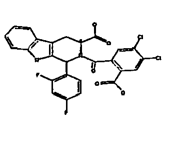 |
| 36 | 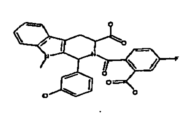 | 98 | 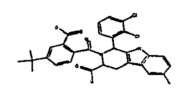 | 160 | 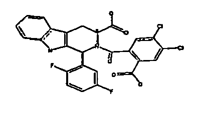 |
| 37 | 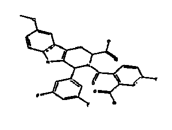 | 99 | 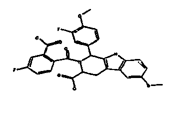 | 161 | 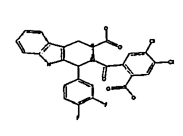 |
| 38 | 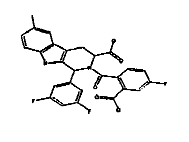 | 100 | 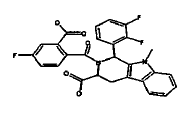 | 162 | 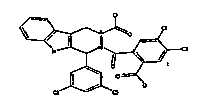 |
| 39 | 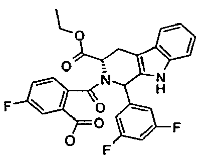 | 101 | 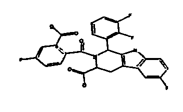 | 163 | 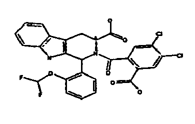 |
| 40 | 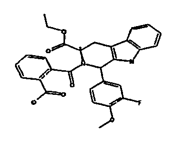 | 102 | 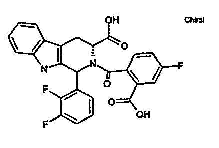 | 164 | 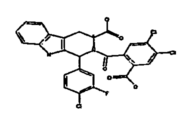 |
| 41 | 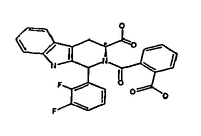 | 103 | 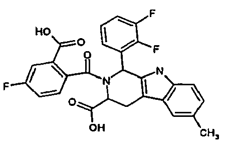 | 165 | 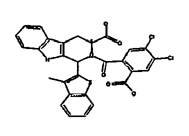 |
| 42 | 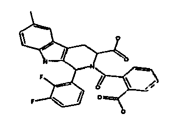 | 104 | 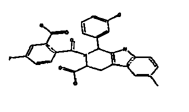 | 166 | 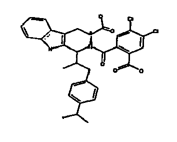 |
| 43 | 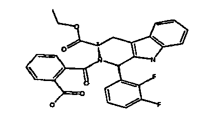 | 105 | 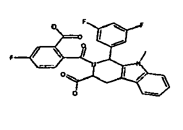 | 167 | 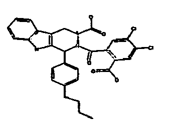 |
| 44 | 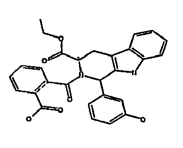 | 106 | 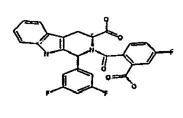 | 168 | 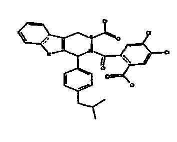 |
| 45 | 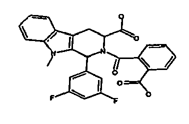 | 107 | 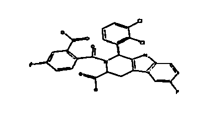 | 169 | 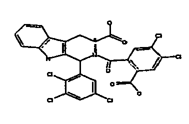 |
| 46 | 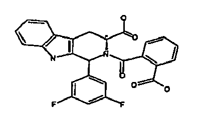 | 108 | 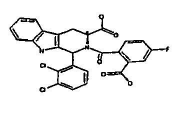 | 170 | 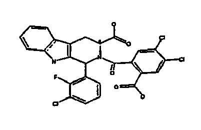 |
| 47 | 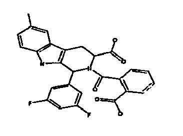 | 109 | 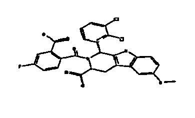 | 171 | 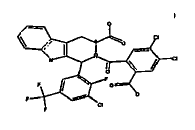 |
| 48 | 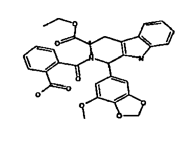 | 110 | 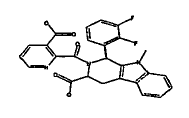 | 172 | 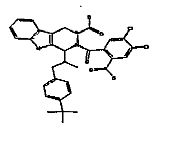 |
| 49 | 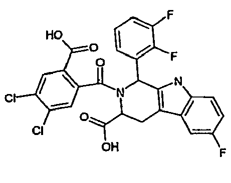 | 111 | 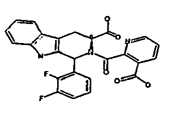 | 173 | 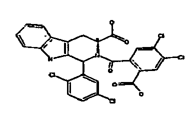 |
| 50 | 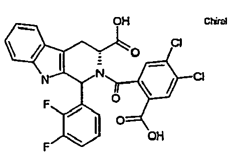 | 112 | 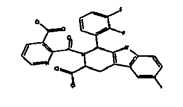 | 174 | 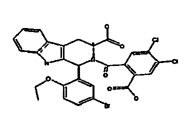 |
| 51 | 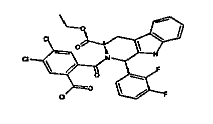 | 113 | 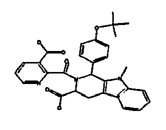 | 175 | 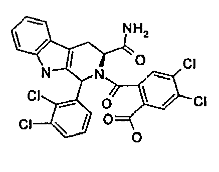 |
| 52 | 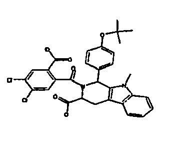 | 114 | 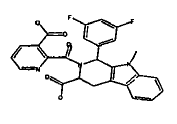 | 176 | 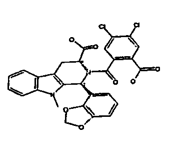 |
| 53 | 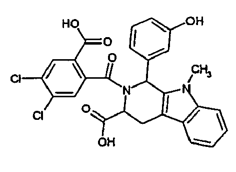 | 115 | 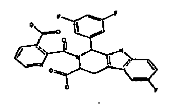 | 177 | 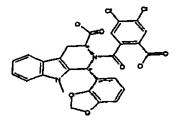 |
| 54 | 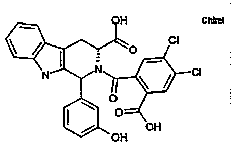 | 116 | 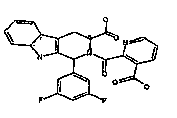 | 178 | 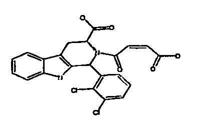 |
| 55 | 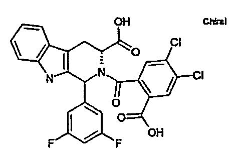 | 117 | 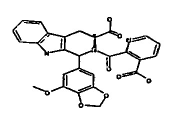 | 179 | 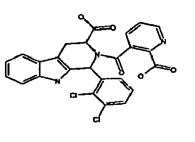 |
| 56 | 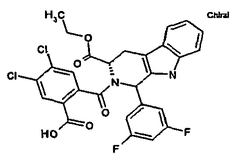 | 118 | 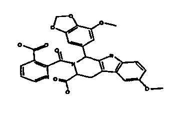 | 180 | 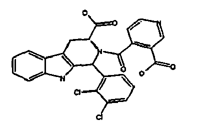 |
| 57 | 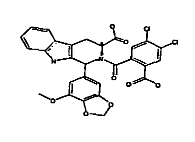 | 119 | 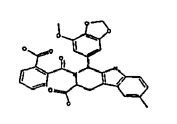 | 181 | 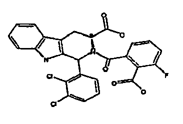 |
| 58 | 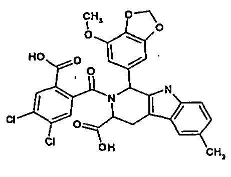 | 120 | 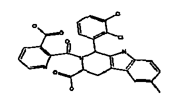 | 182 | 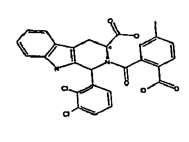 |
| 59 | 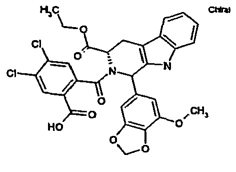 | 121 | 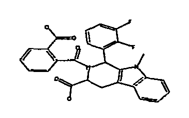 | 183 | 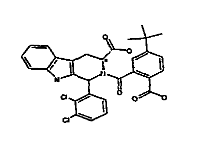 |
| 60 | 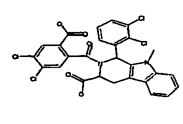 | 122 | 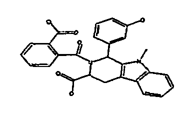 | 184 | 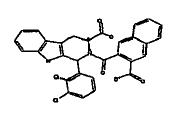 |
| 61 | 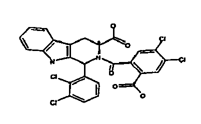 | 123 | 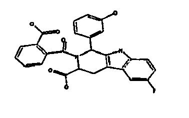 | | |
| 62 | 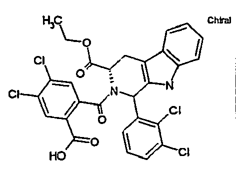 | 124 | 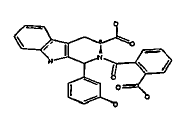 | | |

| | | | | | |
|---|---|---|---|---|---|
| *Structures depicting unfilled valences on N or O, i.e., the indole N in structural formula 1, are understood to be bonded to -H. | | | | | |

### Example 2: Bacteria are dependent on PPAT, a general target for antibiotics

The gene for PPAT, named *coaD* (alternatively, *kdtB*), has recently been identified: see Geerlof, et. al, "Purification and characterization of Phosphopantetheine Adenylyltransferase from E. Coli" J. Biol. Chem., 1999, 274(38), pp 27105-11, the entire teachings of which are incorporated herein by reference. The gene sequence was searched in a range of bacteria and in mammals using BLAST^{®} (Basic Local Alignment Search Tool, available online at http://www.ncbi.nlm.nih.gov/BLAST/). The results are provided in Table 2.

**Table 2: Conservation of PPAT gene (coaD) among range of bacterial species**

| Bacteria | Gram p/n | P(N) | % Identity | % Similarity |
|---|---|---|---|---|
| *Klebsiella pneumoniae* | negative | 1.4E-72 | 85 | 91 |
| *Pseudomonas aeruginosa* | negative | 7.20E-49 | 61 | 81 |
| *Neisseria meningitidis* | negative | 4.80E-27 | 35 | 62 |
| *Enterococcus faecium* | positive | 4.20E-36 | 46 | 67 |
| *Staphylococcus aureus* | positive | 2.10E-36 | 46 | 69 |
| *Staphylococcus epidermidis* | positive | 1.10E-35 | 44 | 67 |
| *Streptococcus pneumoniae* | positive | 2.60E-26 | 36 | 61 |
| *(mammalian)* | NA | - | 18 | - |

PPAT is seen to be highly conserved across a range of bacterial pathogens. Thus, PPAT is a general target for antibiotics. Furthermore, although PPAT is present in mammalian cells, the mammalian sequence is sufficiently different to indicate that the disclosed PPAT inhibitors can be selective for bacterial PPAT.

The gene for PPAT, *coaD*, was disrupted from a range of bacteria by allelic exchange; see, for example, Geerlof, et al, above, and Freiberg, et. al.. 2001. "Identification of novel essential Escherichia coli genes conserved among pathogenic bacteria" J Mol Microbiol Biotechnol 2001, 3, pp 483-9, the entire teachings of which are incorporated herein by reference. The survival of *Escherichia coli*, *Bacillus subtilis*, *Staphylococcus aureus*, and *Streptococcus pneumoniae* in complex growth media was studied. The inability of the modified bacteria to survive without the *coaD* gene indicates that PPAT is necessary for bacterial survival and is thus a potential antibiotic target.

An additional experiment tested the survival of *Escherichia coli* in media containing exogenous dePhospho-CoA and/or CoA. Mammalian, including human cells, can make CoA from pantothenate (vitamin B₅) scavenged from the environment. Thus, it is possible that in a human subject, human cells/tissues could supply CoA to a bacterium that is unable to synthesize CoA. The inability of modified *Escherichia coli* to survive in media containing exogenous dePhospho-CoA and/or CoA further indicates that PPAT can be an antibacterial target.

### Example 3: Kinetic assay of disclosed inhibitors shows strong PPAT inhibition

The IC50 (Inhibition Concentration at 50 percent) values for the disclosed compounds against PPAT were determined with various concentrations of the compounds in a range of 0.003 ~ 200µg/ml. These inhibition assays were performed in 96-well assay plates, using a similar method to the screening assay above. The reaction buffer contained 20mM Hepes (pH7.5), 100mM NaCl, 1mM MgCl2, 0.5mM DTT, 0.006% Brij 35, 10% Glycerol, 25µM PPT, 0.5mM ATP, 0.2 Unit of pyrophosphatase, 200ng of PPAT in a total volume of 100µl. The reaction was performed for 2 minutes, and then stopped with 150ml Malachite Green reagent. Absorption at 650nm was measured after 10 minutes color development. The IC50s were determined with fitting data to the four-parameter method using XLfit (ID Business Solutions Inc., Cambridge, MA). The IC₅₀ value was derived from the curve as the compound concentration that gave 50% inhibition of the enzymatic reaction. The IC₅₀ values are shown in Table 4.

**Table 3: Many Disclosed Compounds Are Strong Inhibitors of PPAT**

| **#** | **IC50 (µM)** | **#** | **IC50 (µm)** | **#** | **IC50 (µM)** | **#** | **IC50 (µm)** | **#** | **IC50 (µm)** |
|---|---|---|---|---|---|---|---|---|---|
| 1 | <10 | 41 | <10 | 81 | <10 | 121 | <10 | 161 | <10 |
| 2 | <10 | 42 | <10 | 82 | <10 | 122 | <10 | 162 | <10 |
| 3 | <10 | 43 | >100 | 83 | <10 | 123 | <10 | 163 | |
| 4 | <10 | 44 | >100 | 84 | <10 | 124 | <10 | 164 | <10 |
| 5 | <10 | 45 | <10 | 85 | <10 | 125 | <10 | 165 | <10 |
| 6 | >100 | 46 | <10 | 86 | <10 | 126 | <10 | 166 | >100 |
| 7 | >100 | 47 | <10 | 87 | <10 | 127 | <10 | 167 | 10-100 |
| 8 | <10 | 48 | 10-100 | 88 | <10 | 128 | <10 | 168 | <10 |
| 9 | <10 | 49 | <10 | 89 | <10 | 129 | <10 | 169 | <10 |
| 10 | <10 | 50 | <10 | 90 | <10 | 130 | 10-100 | 170 | <10 |
| 11 | <10 | 51 | 10-100 | 91 | <10 | 131 | | 171 | <10 |
| 12 | >100 | 52 | >100 | 92 | <10 | 132 | | 172 | <10 |
| 13 | <10 | 53 | <10 | 93 | <10 | 133 | | 173 | <10 |
| 14 | <10 | 54 | <10 | 94 | <10 | 134 | | 174 | <10 |
| 15 | <10 | 55 | <10 | 95 | <10 | 135 | | 175 | |
| 16 | <10 | 56 | <10 | 96 | <10 | 136 | | 176 | 10-100 |
| 17 | <10 | 57 | <10 | 97 | <10 | 137 | <10 | 177 | <10 |
| 18 | <10 | 58 | <10 | 98 | <10 | 138 | | 178 | <10 |
| 19 | <10 | 59 | <10 | 99 | >100 | 139 | | 179 | <10 |
| 20 | <10 | 60 | <10 | 100 | <10 | 140 | | 180 | <10 |
| 21 | 10-100 | 61 | <10 | 101 | <10 | 141 | | 181 | <10 |
| 22 | >100 | 62 | | 102 | <10 | 142 | 10-100 | 182 | <10 |
| 23 | <10 | 63 | <10 | 103 | <10 | 143 | <10 | 183 | <10 |
| 24 | <10 | 64 | <10 | 104 | <10 | 144 | <10 | 184 | <10 |
| 25 | | 65 | <10 | 105 | <10 | 145 | <10 | | |
| 26 | <10 | 66 | <10 | 106 | <10 | 146 | <10 | | |
| 27 | <10 | 67 | <10 | 107 | <10 | 147 | <10 | | |
| 28 | <10 | 68 | 10-100 | 108 | <10 | 148 | <10 | | |
| 29 | 10-100 | 69 | <10 | 109 | <10 | 149 | <10 | | |
| 30 | <10 | 70 | <10 | 110 | <10 | 150 | <10 | | |
| 31 | | 71 | <10 | 111 | <10 | 151 | <10 | | |
| 32 | | 72 | <10 | 112 | <10 | 152 | <10 | | |
| 33 | <10 | 73 | <10 | 113 | >20 | 153 154 | <10 | | |
| 34 | >100 | 74 | <10 | 114 | <10 | 155 | <10 | | |
| 35 | <10 | 75 | <10 | 115 | <10 | | <10 | | |
| 36 | <10 | 76 | <10 | 116 | <10 | 156 | <10 | | |
| 37 | <10 | 77 | 10-100 | 117 | <10 | 157 | <10 | | |
| 38 | <10 | 78 | >100 | 118 | <10 | 158 | <10 | | |
| 39 | | 79 | <10 | 119 | <10 | 159 | <10 | | |
| 40 | > 100 | 80 | | 120 | <10 | 160 | <10 | | |

In order to perform the above IC₅₀ assays, purified PPAT was needed. The *E. coli* PPAT gene was cloned into the pET28a expression vector (Novagen, Inc., Madison, WI) and expressed in *E. coli* BL21(DE3) cells. A chromatographic purification procedure employed Q-sepharose, gel filtration, and MonoQ chromatography, as follows. The methods are described in detailed in Geerlof, et. al., above.

Each cell pellet was suspended in a 4 fold-volume of lysis buffer (50mM KH₂PO₄ pH 8.0, 100mM NaCl, 2mM EGTA, and 10% glycerol. Cells were broken by passage through a Microfluidics cell disrupter 4 times, and the cell lysate was centrifuged at 3,000 g for 20 minutes. The supernatant was applied to a pre-equilibrated Q-sepharose column (10mM Tris-HCl pH 8.0, 0.1mM EGTA, 1mM PMSF, 100mM NaCl, 10% glycerol, 0.1% β-mercaptoethanol, and 0.02% Brij 35). PPAT was eluted with NaCl gradient (0.1∼1M) in the equilibrium buffer. The major peak fractions were pooled and concentrated, then applied to a Sephacryl S200 HR column (10mM Tris-HCl pH 7.5, 150mM NaCl, 0.1mM EGTA, 0.1mM PMSF, 10% glycerol, 0.1% β-mercaptoethanol, and 0.02% Brij 35). PPAT was eluted with the same buffer. The major peak fractions were pooled and loaded on a pre-equilibrated MonoQ column (10mM Tris-HCl, pH 7.0, 0.1mM EGTA, 0.1mM PMSF, 10% glycerol, 0.1% β-mercaptoethanol, and 0.02% Brij 35). PPAT was eluted with a gradient of NaCl from 100mM up to 1000mM. The peak fractions was pooled and dialyzed in the storing buffer (10mM MOPS pH7.0, 150mM NaCl, 0.1mM EGTA, 50% glycerol, 0.02% Brij 35), then stored at -20°C.

### Example 4: Disclosed PPAT inhibitors have antibiotic activity against drug-resistant bacteria

Potency, spectrum, target specificity and serum effect were evaluated by measuring the MIC (Minimum Inhibitory Concentration). This is the lowest concentration, in µg/mL, in a series of 2-fold dilutions of the compound that completely inhibits growth, for a panel of pathogenic bacteria. The strains comprising the bacterial panel are either obtained from American Type Culture Collection (ATCC, Manassas, VA), or genetically engineered to express varying levels *of PPAT*. The ATCC strains included the following: *Escherichia coli* (ATCC 35218), *Staphylococcus aureus* (ATCC 700699), and *Enterococcus faecium* (ATCC 700221). Other strains include *Staphylococcus aureus* RN4220, *Escherichia coli* WO-0159, *Escherichia coli* WO-0153, and *Bacillus subtilis* BD170 with endogenous PPAT disrupted and complemented with PPAT under the regulation of inducible promoter, P_{space}.

The MIC assays were performed essentially as described in the NCCLS recommendations, the entire teachings of which are incorporated herein by reference (National Center for Clinical Laboratory Standards, 1997, METHODS FOR DILUTION ANTIMICROBIAL SUSCEPTIBILITY TESTS FOR BACTERIA THAT GROW AEROBICALLY, 4th ed.; approved standard. NCCLS document M7-A4. NCCLS, Wayne, PA.), with the following exceptions: both Tryptic Soy broth, and Mueller Hinton broth with and without the presence of serum were used as the growth medium. The concentration range tested was 200 to 0.39 mcg/ml. Concentrations of 50-fold the desired final concentration were made by 2-fold serial dilutions in 96-well microtiter plates, after which 2 µL were transferred to the assay plates. Cells were grown up in the appropriate culture media and diluted back to final OD₆₀₀ of 0.001, after which 98 µL were inoculated into the assay plates. The final volume in each assay well was 100 µL. After an overnight incubation at 37°C, the assay plates were read. The MIC was determined as the minimal concentration that resulted in ≥80% inhibition of growth. The MIC values for the disclosed inhibitors are shown in Table 4, in µg/mL,. A value of >=64 indicates that 80% inhibition was not reached at a concentration of 64 µg/mL, or, in some cases, that the compound was tested at a concentration between 64 and 200 µg/mL and 80% inhibition was not observed or was observed at a value higher than 64.

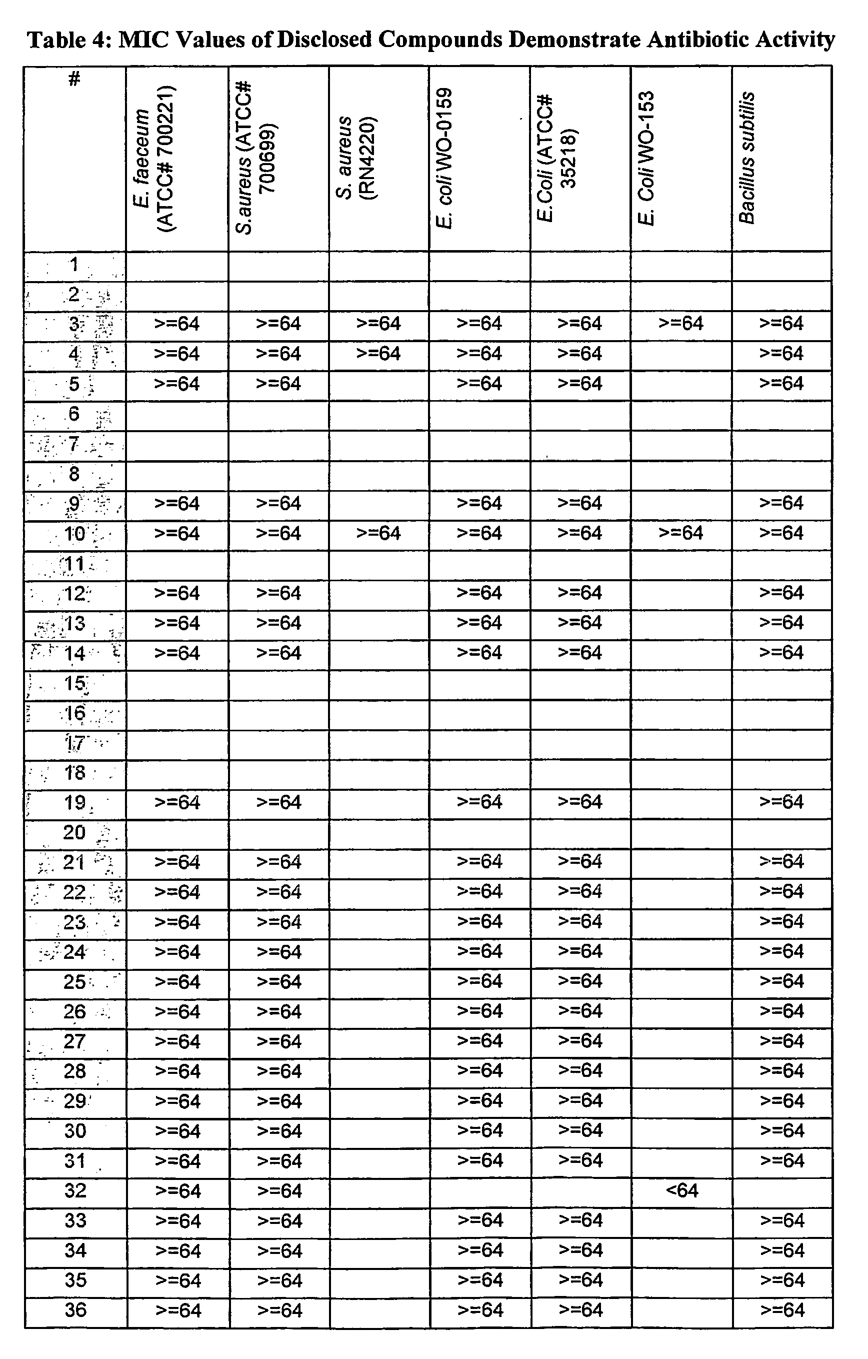

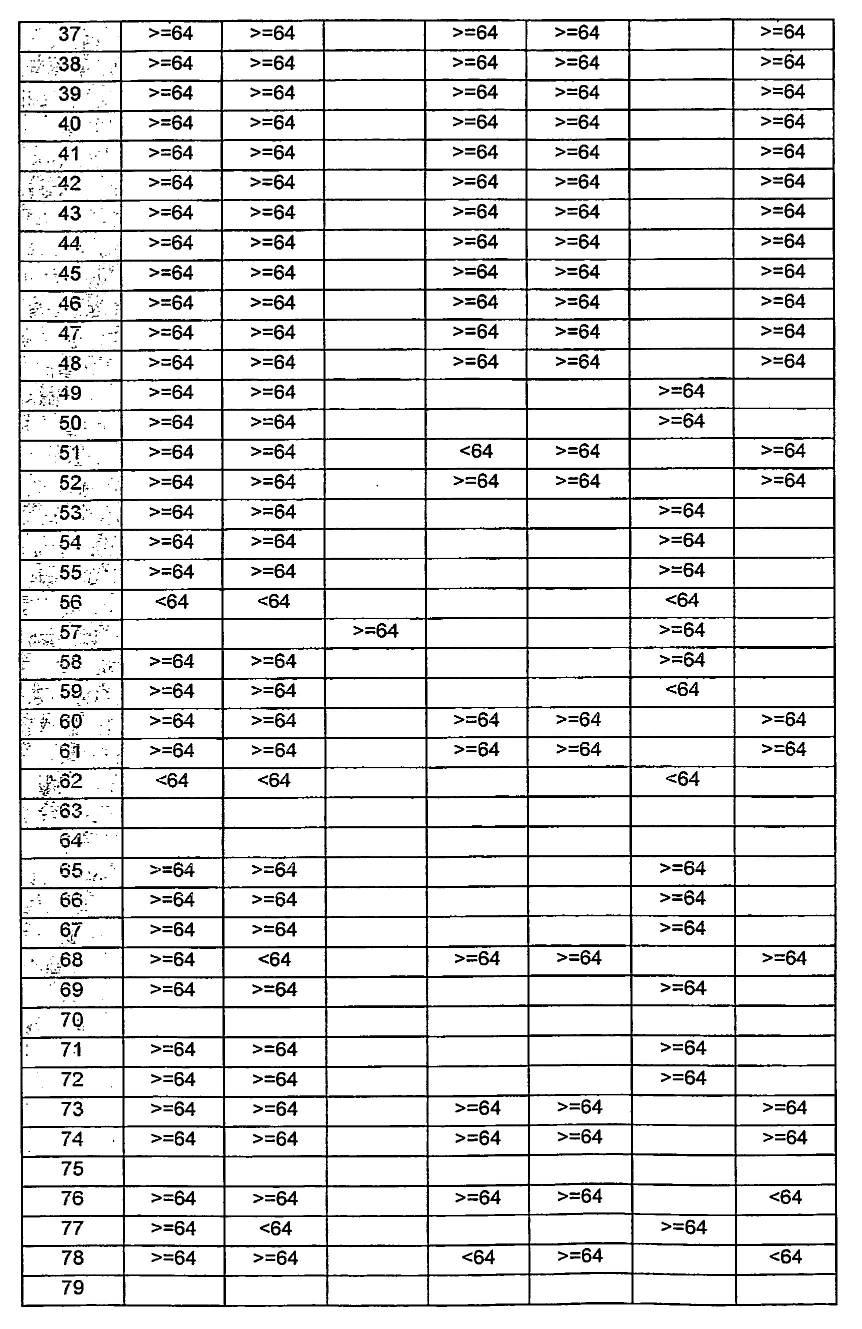

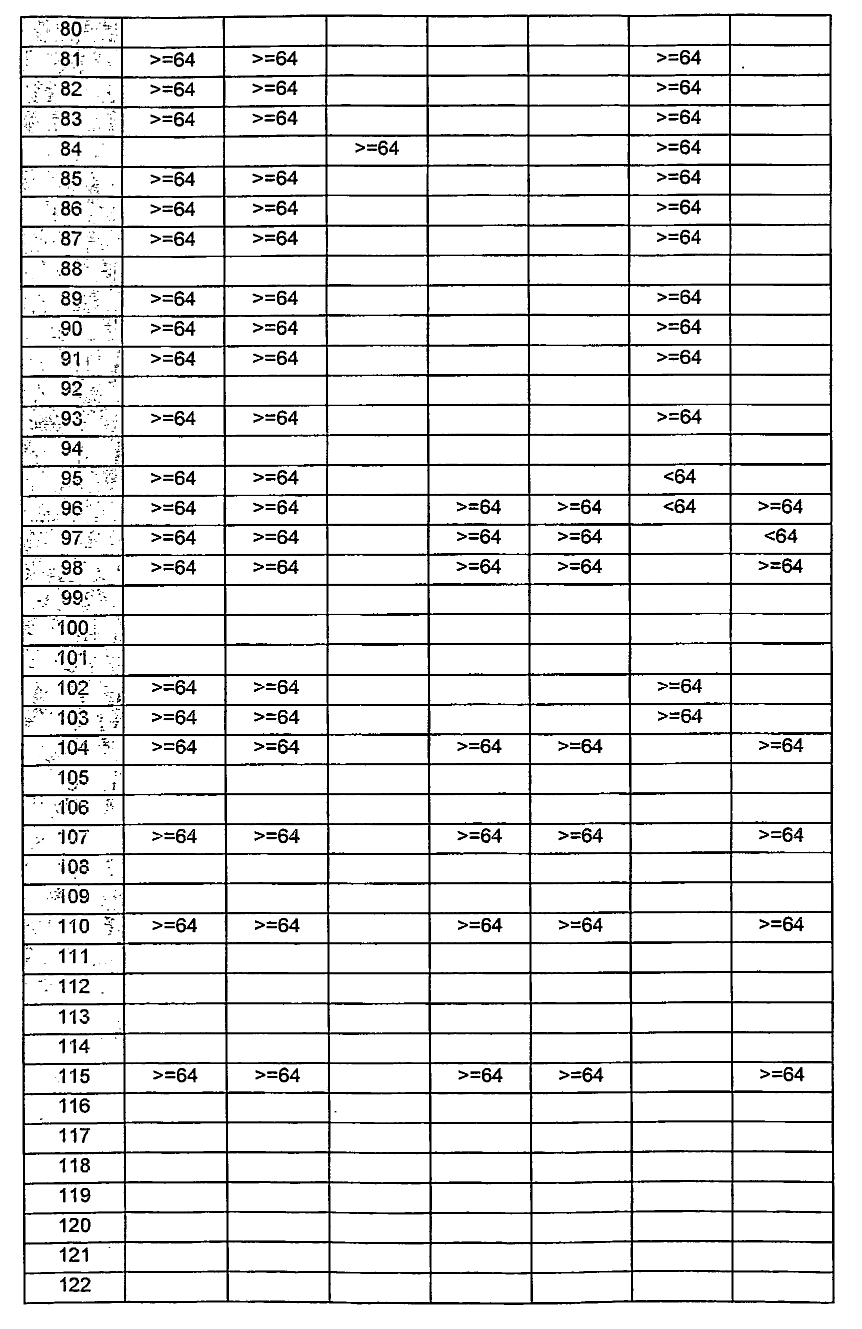

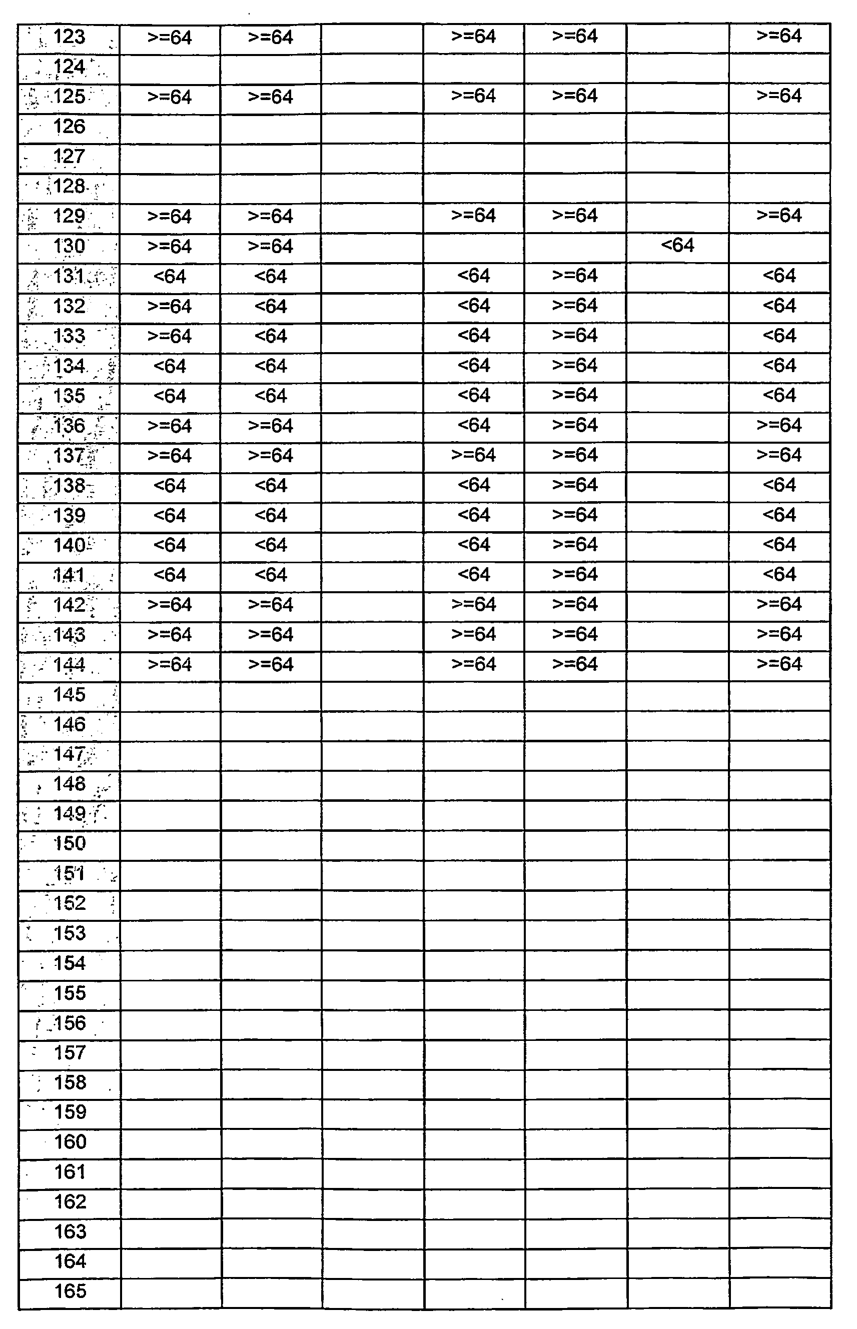

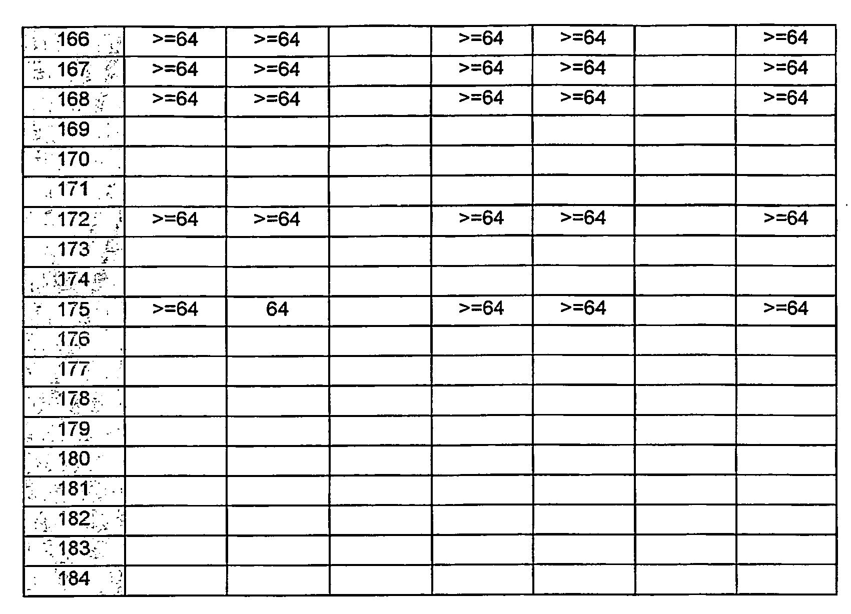

## Claims

1. Use of an effective amount of a compound represented by structural formula **I**: 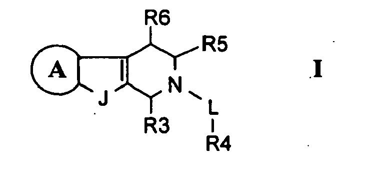 or a pharmaceutically acceptable salt, solvate, or hydrate thereof, wherein:
Ring A is phenyl, optionally substituted at any substitutable ring atom with R1, wherein each R1 is independently halogen, -CN, -NO₂, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, or optionally substituted aryl, aralkyl or C1-C5 alkyl;
wherein:
each R^{d} and R^{f} is independently -H, aryl, aralkyl, C1-C5 alkyl, or C1-C5 haloalkyl;
each R^{e} is independently -H, aryl, aralkyl, or C1-C5 alkyl, or NR^{e}₂ is a non-aromatic heterocyclic group, and
s is 0 to 5;
J is -NR2-, R2 is -H or optionally substituted C1-C5 alkyl, and R3 is optionally substituted aryl, aralkyl, heteroaryl, heteroaralkyl, C3-C7 cycloaliphatic, or C3-C7 cycloalkyl;
L is -(CH₂)-, -(CO)-, -(CS)-, -(SO)-, or -(SO₂)-;
R4 is an aryl, biaryl, heteroaryl, biheteroaryl, heteroaryl-aryl, aryl-heteroaryl, aralkyl, heteroaralkyl, C1-C8 aliphatic, C3-C7 cycloalkyl, C5-C7 cycloaliphatic, or a 3-7 membered non-aromatic heterocyclic group, wherein the group represented by R4 is substituted with -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂, or -NR^{c}SO₂R^{a};
R5 is -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂, or -NR^{c}SO₂R^{a};
R6 is -H, -OH, halogen, or optionally substituted C1-C3 alkyl or alkoxy;
each R^{a} and R^{c} is independently-H, C1-C5 alkyl, aryl, or aralkyl; and
each R^{b} is independently -H, C1-C5 alkyl, aryl, or aralkyl, or NR^{b}₂ is a nonaromatic heterocyclic group;
wherein the optional substituents for alkyl, aliphatic, cycloalkyl, cycloaliphatic, heterocycle, aryl, heteroaryl, heteroaralkyl and aralkyl are -OH, halogen (-Br, -Cl, -I and -F), -R, -OR, -CH₂R, - CH₂CH₂R, -OCH₂R, -CH₂OR, -CH₂CH₂OR, -CH₂OC(O)R, -O-COR, -COR, -SR, -SCH₂R, - CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR, wherein k is 0, 1 or 2, or -NH-C(=NH)-NH₂; wherein each R is independently an alkyl, cycloalkyl, benzyl, aromatic, heteroaromatic, or *N*-anilinyl group or -N(R)₂, taken together, can also form a heterocyclic group; and substituents on the nitrogen of a heterocyclic group or heteroaromatic group include -R', -N(R')₂, -C(O)R', -CO₂ R', -C(O)C(O)R', -C(O)CH₂ C(O)R', -SO₂R', -SO₂ N(R')₂, -C(=S)N(R')₂, -C(=NH)-N(R')₂, and -NR' SO₂R', wherein R' is hydrogen, alkyl, alkoxy, cycloalkyl, cycloalkoxy, phenyl, phenoxy, benzyl, benzyloxy, heteroaromatic or heterocyclic group for the manufacture of a medicament for treating a subject for a bacterial infection.

2. The use of Claim 1, wherein the subject is a human.

3. The use of Claim 2, wherein the infection is caused by a bacteria that expresses phosphopantetheine adenylytransferase.

4. The use of Claim 2, wherein the infection is caused by a bacteria of a genus selected from *Acinetobacter, Bacillus, Campylobacter, Chlamydia, Chlamydophila, Clostridium, Citrobacter, Escherichia, Enterobacter, Enterococcus, Francisella, Haemophilus, Helicobacter, Klebsiella, Listeria, Moraxella, Mycobacterium, Neisseria, Proteus, Pseudomonas, Salmonella, Serratia, Shigella, Stenotrophomonas, Staphyloccocus, Streptococcus,* and *Yersina.*

5. The use of Claim 4 wherein the bacterial infection is from *Acinetobacter baumanii, Bacillus anthracis, Campylobacter jejuni, Chlamydia trachomatis, Chlamydia pneumoniae, Clostridium* spp., *Citrobacter* spp., *Escherichia coli, Enterobacter* spp., *Enterococcus faecalis., Enterococcus faecium, Francisella tularensis, Haemophilus influenzae, Helicobacter pylori, Klebsiella* spp., *Listeria monocytogenes, Moraxella catarrhalis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneumoniae, Streptococcus pyogenes, Streptococcus agalactiae, Yersina pestis,* and *Yersina enterocolitica.*

6. The use of any of the preceding claims wherein R3 is an optionally substituted phenyl, pyridyl, benzo[1,3]dioxolyl, 2,3-dihydro-benzo[1,4]dioxine, pyrimidyl, pyrazyl, furanyl, pyrrolyl, thienyl, oxazolyl, isooxazolyl, thiazolyl, isothiazolyl, imidazolyl naphthyl, quinolinyl, biphenyl, benzopyrimidyl, benzopyrazyl, benzofuranyl, indolyl, benzothienyl, benzoxazolyl, benzoisooxazolyl, benzothiazolyl, benzoisothiazolyl, or benzimidazolyl group.

7. The use of any of the preceding claims wherein R4 is a substituted phenyl, pyridyl, pyrimidyl, pyrazyl, naphthyl, biphenyl, phenyl-pyridyl, bipyridyl, quinolinyl, benzopyrimidyl, benzopyrazyl, cyclopropyl, cyclobutyl, cyclopentyl, cyclopentenyl, cyclohexyl, cyclohexenyl, or C2-C8 alkenyl group.

8. The use of Claim 7 wherein the compound is represented by structural formula IV: 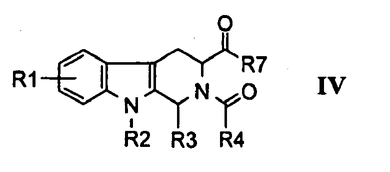 wherein:
R7 is-OR^{o} or -NR^{p}₂;
R^{o} is -H or optionally substituted aryl, aroyl, aralkyl, aralkanoyl, C1-C5 alkyl, or C1-C5 alkanoyl; and
each R^{p} is independently -H, C1-C5 alkyl, aryl, or aralkyl, or NR^{p}₂ is a nonaromatic heterocyclic group.

9. The use of Claim 8 wherein R4 is represented by one of structural formulas R4-i to **R4-vii:** 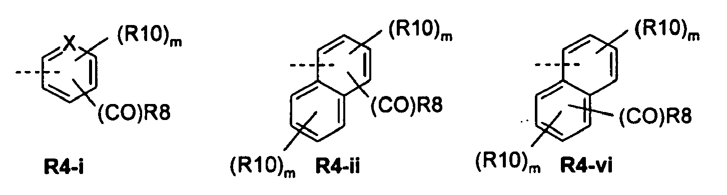 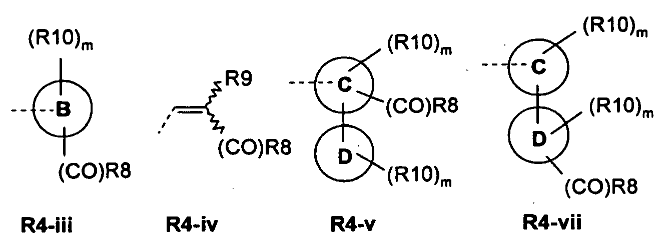 wherein:
each m is independently 0, 1, 2, or 3;
X is -N-, -CH-, or -CR10-;
Ring **B** is C3-C6 cycloalkyl or C3-C6 cycloalkenyl;
Rings **C** and **D** are each independently aryl or heteroaryl;
R8 is-OR^{q} or -NR^{r}₂;
R9 is -H, aryl, aralkyl, or C1-C6 aliphatic;
each R10 is independently halogen, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -NR^{k}(CO)NR^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂, or optionally substituted aryl, aralkyl orC1-C5 alkyl;
each Rⁱ and R^{k} is independently -H, aryl, aralkyl, C1-C5 alkyl, or C1-C5 haloalkyl;
each R^{j} and R^{r} is independently -H, aryl, aralkyl, or C1-C5 alkyl, or each NR^{j}₂ and NR^{r}₂ is independently a nonaromatic heterocyclic group;
R^{q} is -H or optionally substituted aryl, aroyl, aralkyl, aralkanoyl, C1-C5 alkyl, or C1-C5 alkanoyl; and
t is 0 to 5.

10. The use of Claim 9 wherein R3 is represented by one of structural formulas **R3-i** to **R3-v:** 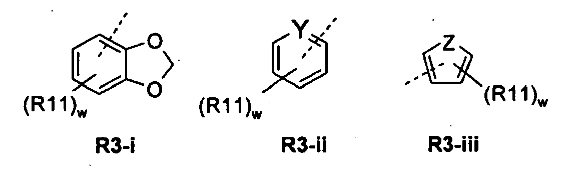 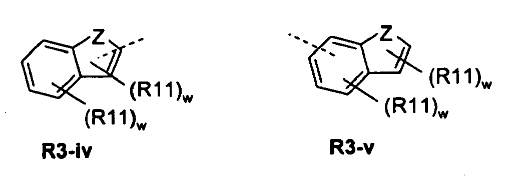 wherein:
Y is -N-, -CH-, or -CR11-;
each Z is independently -NR^{z}-, -S-, or -O-, wherein R^{z} is -H or C1-C3 alkyl;
w is 0, 1, 2, or 3;
each R11 is independently halogen, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ(CO)NR^{m}₂, -NRⁿ(CO)OR¹, -NRⁿCOR¹, -(SO₂)NR^{m}₂, -NRⁿSO₂R¹, -(CH₂)ᵤNR¹₂, or optionally substituted aryl, aralkyl, or C1-C5 alkyl;
each R¹ and Rⁿ is independently -H, aryl, or aralkyl, C1-C5 alkyl, or C1-C5 haloalkyl;
each R^{m} is independently -H, aryl, aralkyl, or C1-C5 alkyl, or NR^{m}₂ is a nonaromatic heterocyclic group; and
u is 0 to 5.

11. The use of Claim 10 wherein R4 is represented by one of structural formulas **R4-i'** to **R4-vii'**: 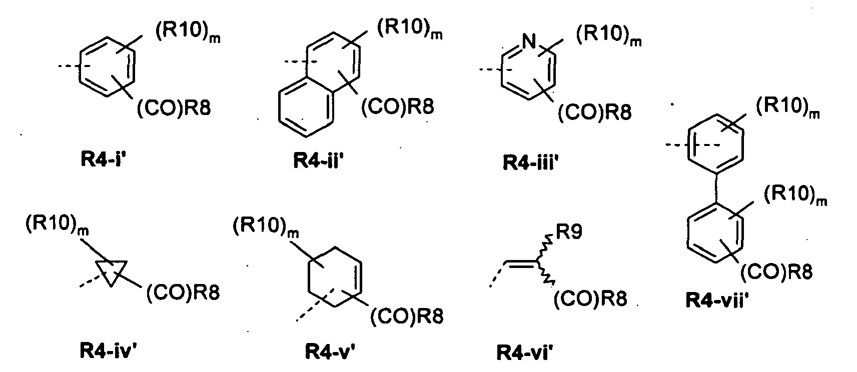 wherein:
each m is independently 0, 1, 2, or 3;
R8 is -NR^{y}₂, -OH, C1-C5 alkoxy, or C1-C5 alkanoyloxy, wherein each R^{y} is independently -H or C1-C3 alkyl;
R9 is -H or C1-C6 aliphatic; and
each R10 is independently -OH, -NO₂, -F, -Cl, -Br, C1-C4 alkyl, C1-C4 alkoxy, -CF₃, or -OCF₃.

12. The use of Claim 11 wherein R3 is represented by one of structural formulas **R3-i'** to **R3-v'**: 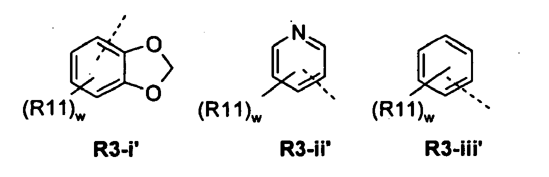 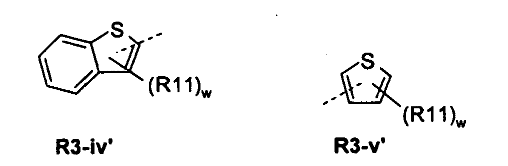 wherein:
w is 0, 1, 2, or 3; and
each R11 is independently -OH, -NO₂, -F, -Cl, -Br, C1-C4 alkyl, C1-C4 alkoxy, -CF₃, or -OCF₃.

13. The use of Claim 12, wherein R7 is -NR^{x}₂, -OH, C1-C5 alkoxy, or C1-C5 alkanoyloxy, wherein each R^{x} is independently-H or C1-C3 alkyl.

14. The use of Claim 13, wherein:
R3 is represented by one of structural formulas **R3^{a}** to **R3^{r}:** 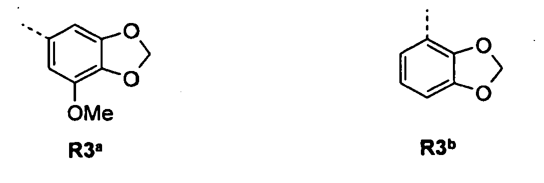 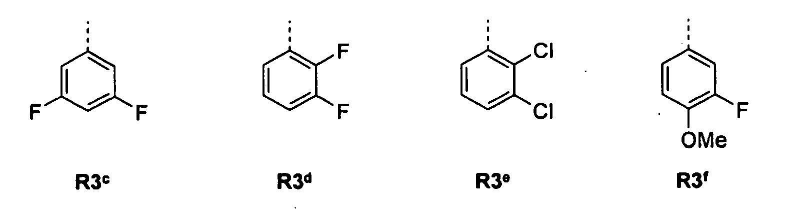 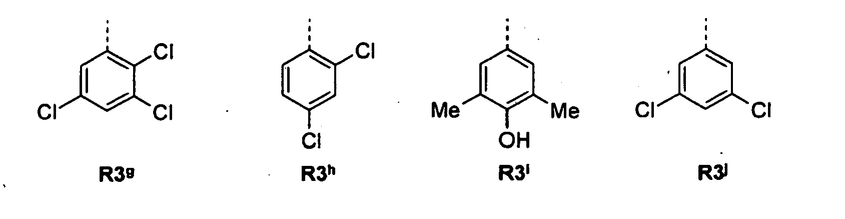 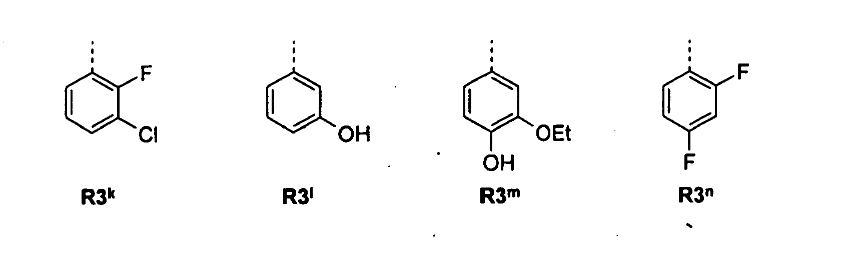 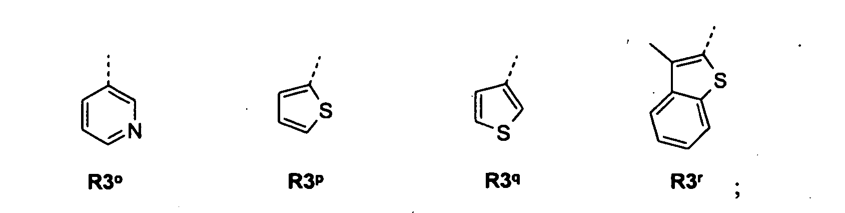
or, R4 is represented by one of structural formulas **R4^{a}** to **R4^{q}:**

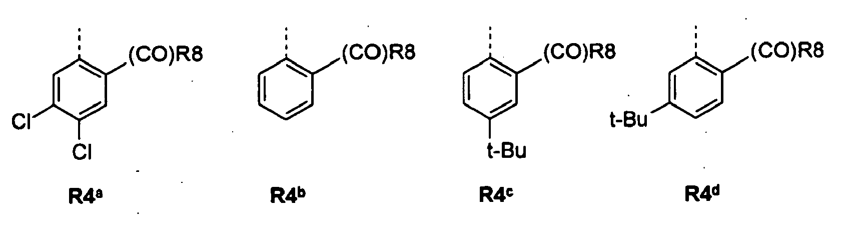

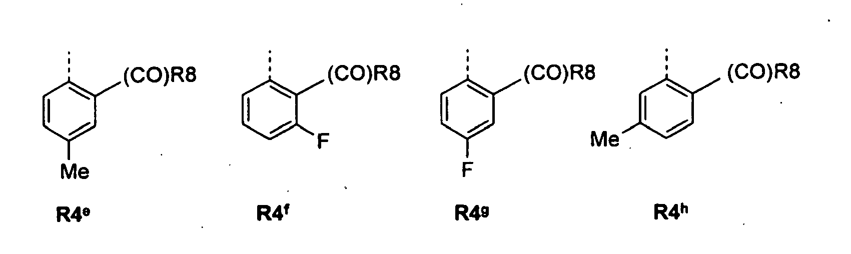

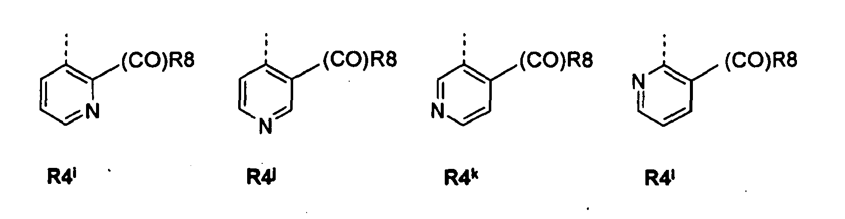

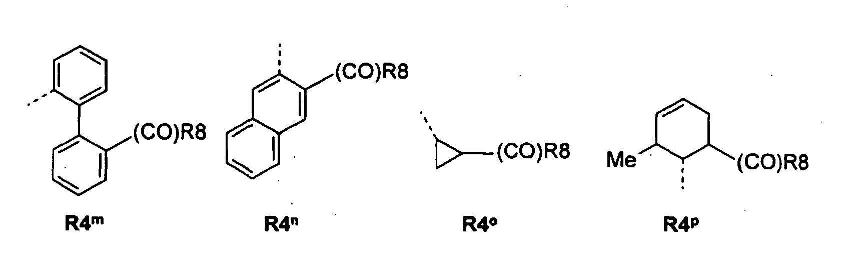

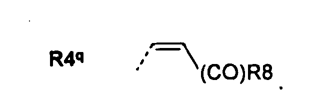

15. The use of Claim 14 wherein:
R3 is represented by one of structural formulas **R3^{a}** to **R3^{r};** and
R4 is represented by one of structural formulas **R4^{a}** to **R4^{q}.**

16. The use of Claim 15 wherein the compound is represented by one of structural formulas **V** to **XI**: 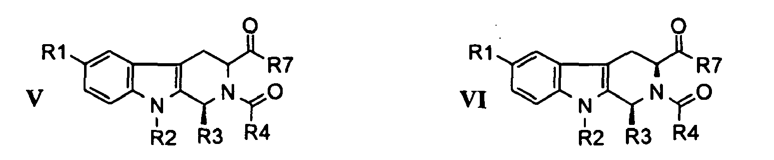 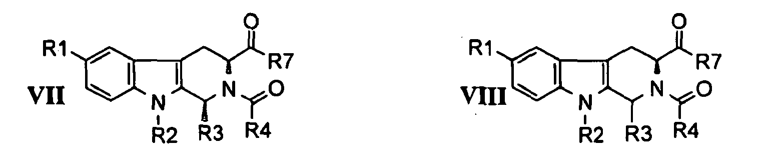 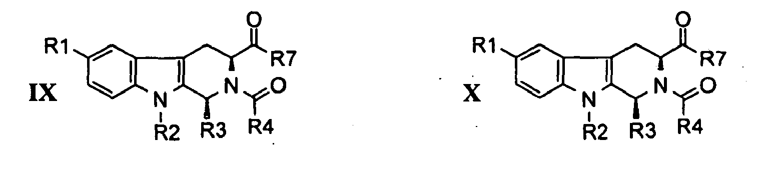 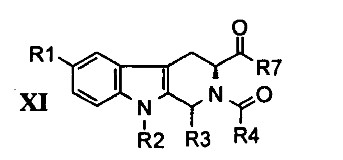 wherein R7 is -OH or C1-C4 alkoxy.

17. The use of Claim 16 wherein R1 is -H, -OH, -F, -CH₃, -CF₃, -OCH₃, or -OCF₃.

18. The use of Claim 17 wherein:
R3 is represented by structural formula **R3^{d}, R3^{e},** or **R3^{f};** or
R4 is represented by structural formula **R4^{a}, R4^{c},** or **R4^{e}.**

19. The use of Claim 17 wherein:
R3 is represented by structural formula **R3^{d}, R3^{e},** or **R3^{f};** and
R4 is represented by structural formula **R4^{a}, R4^{c},** or **R4^{e}.**

20. The use of Claim 19 wherein R7 and R8 are -OH.

21. The use of Claim 19 wherein one of R7 or R8 is -OH, and the other is -OCH₃ or -OCH₂CH₃.

22. The use of Claim 19 wherein R7 and R8 are independently -OCH₃ or -OCH₂CH₃.

23. A pharmaceutical composition comprising a compound represented by structural formula **I'**: 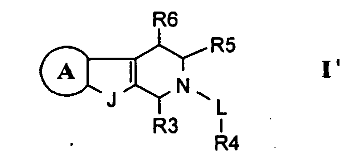 or a pharmaceutically acceptable salt, solvate, or hydrate thereof, wherein:
Ring A is phenyl, optionally substituted at any substitutable ring atom with R1, wherein each R1 is independently halogen, -CN, -NO₂, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, or optionally substituted aryl, aralkyl or C1-C5 alkyl;
wherein:
each R^{d} and R^{f} is independently-H, aryl, aralkyl, C1-C5 alkyl, or C1-C5 haloalkyl;
each R^{e} is independently -H, aryl, aralkyl, or C1-C5 alkyl, or NR^{e}₂ is a non-aromatic heterocyclic group, and
s is 0 to 5;
J is -NR2-, R2 is -H or optionally substituted C1-C5 alkyl, and R3 is "optionally substituted aryl, aralkyl, heteroaryl, heteroaralkyl, C3-C7 cycloaliphatic, or C3-C7 cycloalkyl;
L is -(CH₂)-, -(CO)-, -(CS)-, -(SO)-, or -(SO₂)-;
R4 is an aryl, biaryl, heteroaryl, biheteroaryl, heteroaryl-aryl, aryl-heteroaryl, aralkyl, heteroaralkyl, C1-C8 aliphatic, C3-C7 cycloalkyl, C5-C7 cycloaliphatic, or a 3-7 membered non-aromatic heterocyclic group, wherein the group represented by R4 is substituted with -(CO)OR^{a}, -(CO)O(CO)R^{a}, -(CS)OR^{a}, -(SO)OR^{a}, -SO_{3R}^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂, or -NR^{c}SO₂R^{a};
R5 is -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂, or -NR^{c}SO₂R^{a};
R6 is -H, -OH, halogen, or optionally substituted C1-C3 alkyl or alkoxy;
each R^{a} and R^{c} is independently-H, C1-C5 alkyl, aryl, or aralkyl; and
each R^{b} is independently -H, C1-C5 alkyl, aryl, or aralkyl, or NR^{b}₂ is a nonaromatic heterocyclic group:
wherein the optional substituents for alkyl, aliphatic, cycloalkyl, cycloaliphatic, heterocycle, aryl, heteroaryl, heteroaralkyl and aralkyl -OH, halogen (-Br, -Cl, -I and -F), -R, -OR, -CH₂R, - CH₂CH₂R, -OCH₂R, -CH₂OR, -CH₂CH₂OR, -CH₂OC(O)R, -O-COR, -COR, -SR, -SCH₂R, - CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR, wherein k is 0, 1 or 2, or -NH-C(=NH)-NH₂; wherein each R is independently an alkyl, cycloalkyl, benzyl, aromatic, heteroaromatic, or *N*-anilinyl group or -N(R)₂, taken together, can also form a heterocyclic group; and substituents on the nitrogen of a heterocyclic group or heteroaromatic group include -R', -N(R')₂, -C(O)R', -CO₂ R', -C(O)C(O)R', -C(O)CH₂ C(O)R', -SO₂R' -SO₂ N(R')₂, -C(=S)N(R')₂, -C(=NH)-N(R')₂, and -NR' SO₂R', wherein R' is hydrogen, alkyl, alkoxy, cycloalkyl, cycloalkoxy, phenyl, phenoxy, benzyl, benzyloxy, heteroaromatic or heterocyclic group.

24. The composition of Claim 23 wherein R3 is an optionally substituted phenyl, pyridyl, benzo[1,3]dioxolyl, 2,3-dihydro-benzo[1,4]dioxine, pyrimidyl, pyrazyl, furanyl, pyrrolyl, thienyl, oxazolyl, isooxazolyl, thiazolyl, isothiazolyl, imidazolyl naphthyl, quinolinyl, biphenyl, benzopyrimidyl, benzopyrazyl, benzofuranyl, indolyl, benzothienyl, benzoxazolyl, benzoisooxazolyl, benzothiazolyl, benzoisothiazolyl, or benzimidazolyl group.

25. The composition of Claim 24 wherein R4 is a substituted phenyl, pyridyl, pyrimidyl, pyrazyl, naphthyl, biphenyl, phenyl-pyridyl, quinolinyl, benzopyrimidyl, benzopyrazyl, cyclopropyl, cyclobutyl, cyclopentyl, cyclopentenyl, cyclohexyl, cyclohexenyl, or C2-C8 alkenyl group.

26. The composition of Claim 25 wherein the compound is represented by structural formula **IV**: 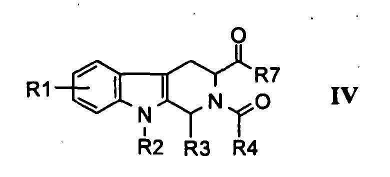 wherein:
R7 is-OR^{o} or -NR^{p}₂;
R^{o} is -H or optionally substituted aryl, aroyl, aralkyl, aralkanoyl, C1-C5 alkyl, or C1-C5 alkanoyl; and
each R^{p} is independently -H, C1-C5 alkyl, aryl, or aralkyl, or NR^{p}₂ is a nonaromatic heterocyclic group.

27. The composition of Claim 26 wherein R4 is represented by one of structural formulas **R4-i to R4-vii**: 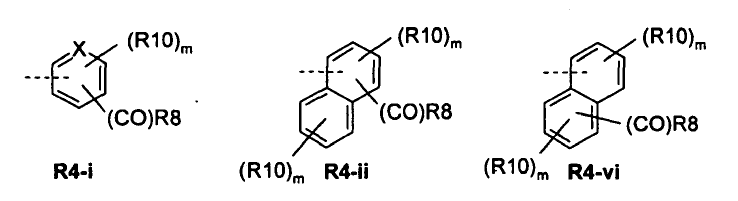 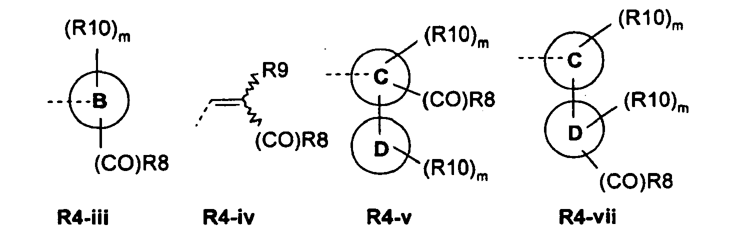 wherein:
each m is independently 0,1, 2, or 3;
X is N-, -CH-, or -CR10-;
Ring **B** is C3-C6 cycloalkyl or C3-C6 cycloalkenyl;
Ring **C** is phenyl;
Ring **D** is aryl or heteroaryl;
R8 is-OR^{q} or -NR^{r}₂;
R9 is -H, aryl, aralkyl, or C1-C6 aliphatic;
each R10 is independently halogen, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -N^{k}(CO)NR^{j}₂, -NR^{k}(CO)ORⁱ, -NR^{k}CORⁱ, =(SO₂)NR^{j}₂, -NR^{k}SO₂Rⁱ, -(CH₂)ₜNR^{j}₂, or optionally substituted aryl, aralkyl or C1-C5 alkyl;
each Rⁱ and R^{k} is independently -H, aryl, aralkyl, C1-C5 alkyl, or C1-C5 haloalkyl;
each R^{j} and R^{r} is independently -H, aryl, aralkyl, or C1-C5 alkyl, or each NR^{j}₂ and NR^{r}₂ is independently a nonaromatic heterocyclic group;
R^{q} is -H or optionally substituted aryl, aroyl, aralkyl, aralkanoyl, C1-C5 alkyl, or C1-C5 alkanoyl; and
t is 0 to 5.

28. The composition of Claim 27 wherein R3 is represented by one of structural formulas **R3-i to R3-v**: 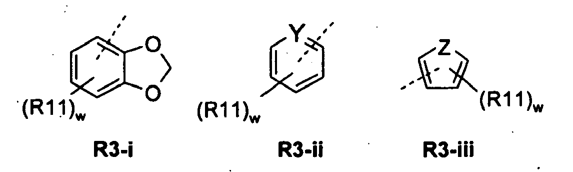 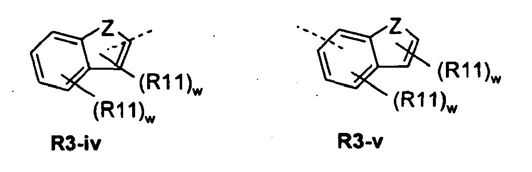 wherein:
Y is -N-, -CH-, or -CR11-;
each Z is independently -NR^{z}-, -S-, or -O-, wherein R^{z} is -H or C1-C3 alkyl;
wis0, 1,2,or3;
each R11 its independently halogen, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ(CO)NR^{m}₂, -NRⁿ(CO)OR¹, -NRⁿCOR¹, -(SO₂)NR^{m}₂, -NRⁿSO₂R¹, -(CH₂)ᵤNR¹₂, or optionally substituted aryl, aralkyl, or C1-C5 alkyl;
each R¹ and Rⁿ is independently -H, aryl, or aralkyl, C1-C5 alkyl, or C1-C5 haloalkyl;
each R^{m} is independently -H, aryl, aralkyl, or C1-C5 alkyl, or NR^{m}₂ is a nonaromatic heterocyclic group; and
u is 0 to 5.

29. The composition of Claim 28 wherein R4 is represented by one of structural formulas **R4-i'** to **R4-vii':** 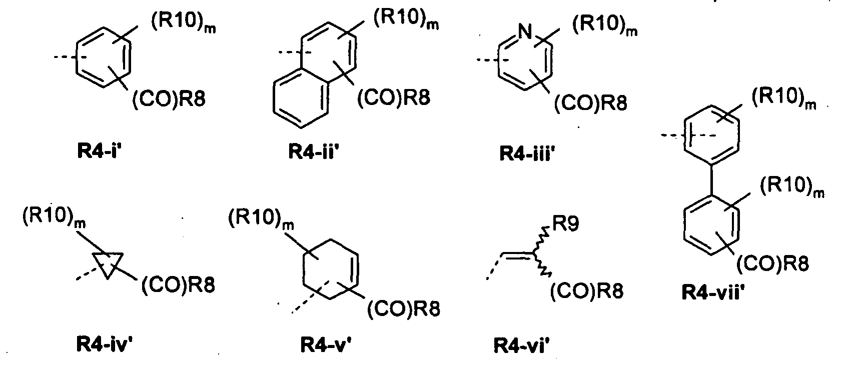 wherein:
each m is independently 0, 1, 2, or 3;
R8 is -NR^{y}₂, -OH, C1-C5 alkoxy, or C1-C5 alkanoyloxy, wherein each R^{y} is independently -H or C1-C3 alkyl;
R9 is -H or C1-C6 aliphatic; and
each R10 is independently -OH, -NO₂, -F, -Cl, -Br, C1-C4 alkyl, C1-C4 alkoxy, -CF₃, or -OCF₃.

30. The composition of Claim 29 wherein R3 is represented by one of structural formulas **R3-i'** to **R3-v'**: 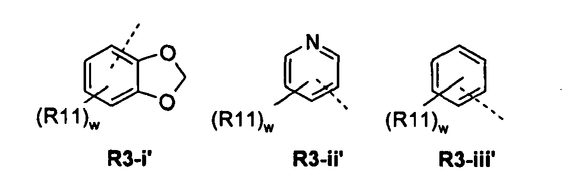 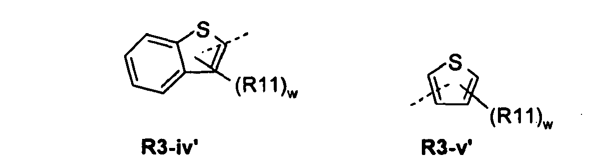 wherein:
w is 0, 1, 2, or 3; and
each R11 is independently-OH, -NO₂, -F, -Cl, -Br, C1-C4 alkyl, C1-C4 alkoxy, -CF₃, or -OCF₃.

31. The composition of Claim 30, wherein R7 is -NR^{x}₂, -OH, C1-C5 alkoxy, or C1-C5 alkanoyloxy, wherein each R^{x} is independently-H or C1-C3 alkyl.

32. The composition of Claim 31, wherein:
R3 is represented by one of structural formulas **R3^{a}** to **R3^{r}**: 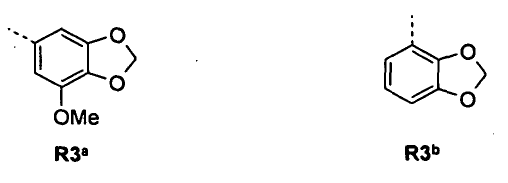 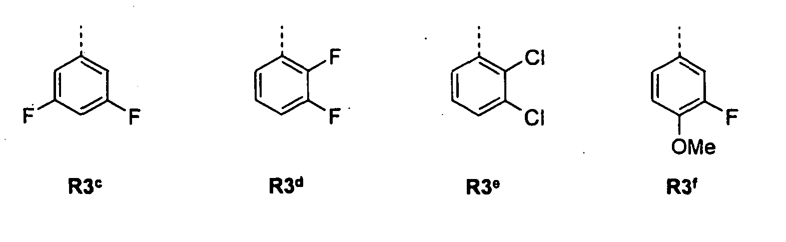 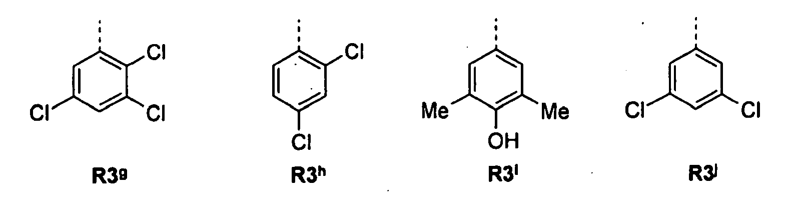 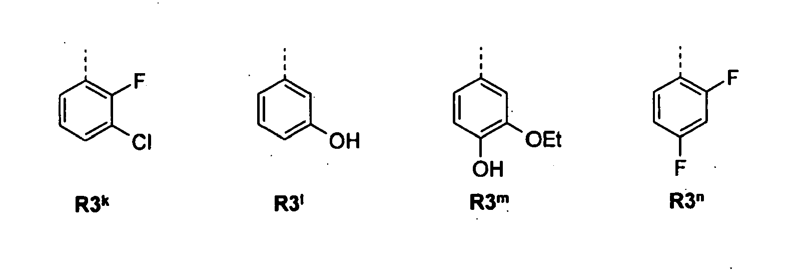 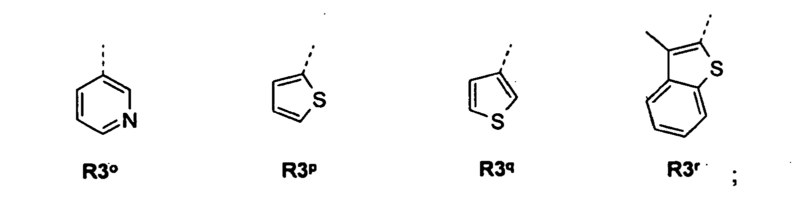
or, R4 is represented by one of structural formulas **R4^{a}** to **R4^{q}**:

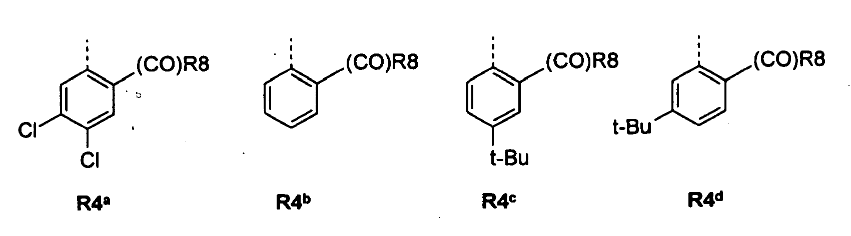

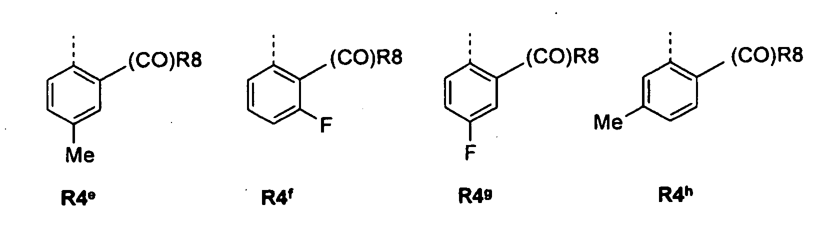

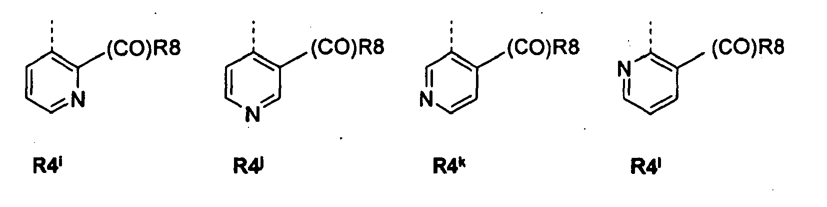

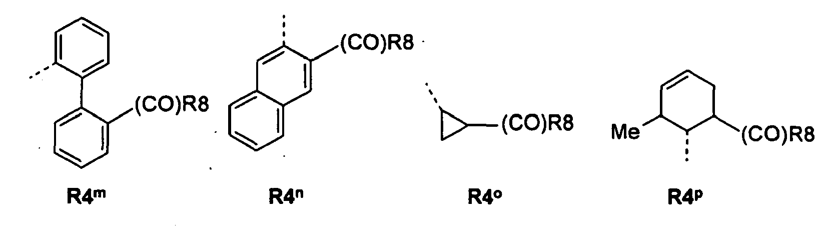

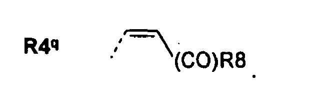

33. The composition of Claim 32 wherein:
R3 is represented by one of structural formulas **R3^{a}** to **R3^{r}**; and
R4 is represented by one of structural formulas **R4^{a}** to **R4^{q}**.

34. The composition of Claim 33 wherein the compound is represented by one of structural formulas **V** to **XI**: 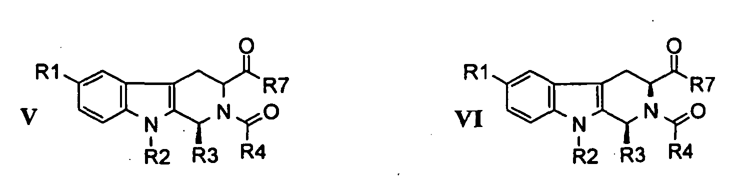 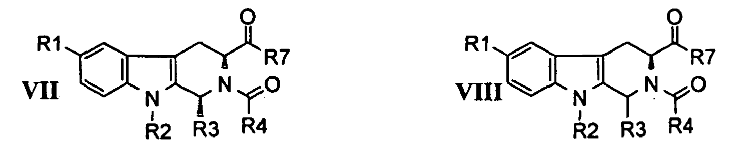 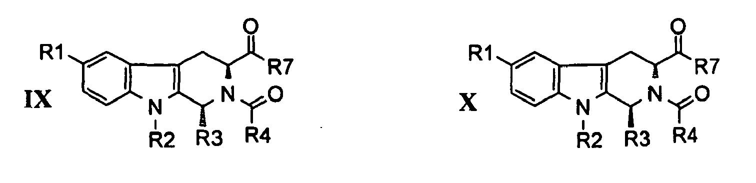 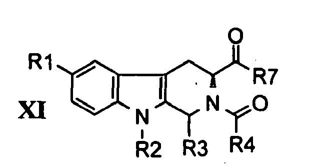 wherein R7 is -OH or C1-C4 alkoxy.

35. The composition of Claim 34 wherein R1 is -H, -OH, -F, -CH₃, -CF₃, -OCH₃, or -OCF₃.

36. The composition of Claim 35 wherein:
R3 is represented by structural formula **R3^{d}**, **R3^{e}**, or **R3^{f}**; or
R4 is represented by structural formula **R4^{a}**, **R4^{c}**, or **R4^{e}**.

37. The composition of Claim 35 wherein:
R3 is represented by structural formula **R3^{d}**, **R3^{e}**, or **R3^{f}**; and
R4 is represented by structural formula **R4^{a}**, **R4^{c}**, or **R4^{e}**.

38. The composition of Claim 37 wherein R7 and R8 are -OH.

39. The composition of Claim 37 wherein one of R7 or R8 is -OH, and the other is -OCH₃ or -OCH₂CH₃.

40. The composition of Claim 37 wherein R7 and R8 are independently -OCH₃ or -OCH₂CH₃.

## Patentansprüche

1. Verwendung einer wirksamen Menge einer Verbindung, dargestellt durch Strukturformel **I**: 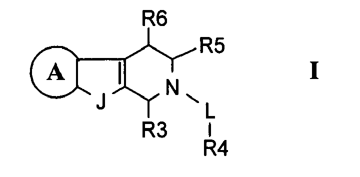 oder ein pharmazeutisch akzeptables Salz, Solvat oder Hydrat davon, wobei:
Ring A Phenyl ist, wahlweise substituiert an irgendeinem geeigneten Ringatom mit R1, wobei jedes R1 unabhängig Halogen, -CN, -NO₂, -OR^{d}, -(CO)R^{d}, -(CO)OR^{d}, -O(CO)R^{d} -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂ oder wahlweise substituiertes Aryl, Aralkyl oder C1-C5-Alkyl ist,
wobei:
jedes R^{d} und R^{f} unabhängig -H, Aryl, Aralkyl, C1-C5-Alkyl, oder C1-C5-Halogenalkyl ist,
jedes R^{e} unabhängig -H, Aryl, Aralkyl, oder C1-C5-Alkyl ist, oder NR^{e}₂ eine nichtaromatische heterocyclische Gruppe ist, und
s 0 bis 5 ist,
J -NR2- ist, R2 -H oder wahlweise substituiertes C1-C5-Alkyl ist, und R3 wahlweise substituiertes Aryl, Aralkyl, Heteroaryl, Heteroaralkyl, C3-C7-cycloaliphatisch oder C3-C7-Cycloalkyl ist,
L -(CH₂)-, -(CO)-, -(CS)-, -(SO)- oder -(SO₂)- ist,
R4 ein Aryl, Biaryl, Heteroaryl, Biheteroaryl, Heteroaryl-aryl, Aryl-heteroaryl, Aralkyl, Heteroaralkyl, C1-C8-aliphatisch, C3-C7-Cycloalkyl, C5-C7-cycloaliphatisch ist, oder eine 3-7-gliederige nicht-aromatische heterocyclische Gruppe, wobei die Gruppe, die durch R4 dargestellt wird, mit -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂ oder -NR^{c}SO₂R^{a} substituiert ist,
R5 -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR(CO)R^{a}, -SO₂NR^{b}₂ oder -NR^{c}SO₂R^{a} ist,
R6 -H, -OH, Halogen oder wahlweise substituiertes C1-C3-Alkyl oder Alkoxy ist; jedes R^{a} und R^{c} unabhängig -H, C1-C5-Alkyl, Aryl oder Aralkyl ist, und jedes R^{b} unabhängig -H, C1-C5-Alkyl, Aryl oder Aralkyl ist, oder NR^{b}₂ eine nicht-aromatische heterocyclische Gruppe ist,
wobei die optionalen Substituenten für Alkyl, aliphatisch, Cycloalkyl, cycloaliphatisch, Heterozyklus, Aryl, Heteroaryl, Heteroaralkyl and Aralkyl -OH, Halogen (-Br, -Cl, -I und -F), -R, -OR, -CH₂R, -CH₂CH₂R, -OCH₂R, -CH₂OR, -CH₂CH₂OR, -CH₂OC(O)R, -O-COR, -COR, -SR, -SCH₂R, -CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR sind, wobei k 0, 1 oder 2 ist, oder -NH-C(=NH)-NH₂, wobei jedes R unabhängig eine Alkyl-, Cycloalkyl-, Benzyl-, aromatische, heteroaromatische oder *N-*Anilinyl-Gruppe ist, oder -N(R)₂ zusammengenommen auch eine heterocyclische Gruppe bilden kann, und Substituenten am Stickstoff einer heterocyclischen Gruppe oder heteroaromatischen Gruppe beinhalten -R', -N(R')₂, -C(O)R', -CO₂R', -C(O)C(O)R', -C(O)CH₂ C(O)R', -SO₂R', -SO₂N(R')₂, -C(=S)N(R')₂, -C(=NH)-N(R')₂ und -NR' SO₂R', wobei R' Wasserstoff, Alkyl, Alkoxy, Cycloalkyl, Cycloalkoxy, Phenyl, Phenoxy, Benzyl, Benzyloxy, heteroaromatische oder heterocyclische Gruppe ist, für die Herstellung eines Medikaments zur Behandlung eines Individuums gegen eine bakterielle Infektion.

2. Verwendung nach Anspruch 1, wobei das Individuum ein Mensch ist.

3. Verwendung nach Anspruch 2, wobei die Infektion durch ein Bakterium verursacht wird, das Phosphopantetheinadenyltransferase exprimiert.

4. Verwendung nach Anspruch 2, wobei die Infektion durch ein Bakterium einer Gattung, ausgewählt aus *Acinetobacter, Bacillus, Campylobacter, Chlamydia, Chlamydophila, Clostridium, Citrobacter, Escherichia, Enterobacter, Enterococcus, Francisella, Haemophilus, Helicobacter, Klebsiella, Listeria, Moraxella, Mycobacterium, Neisseria, Proteus, Pseudomonas, Salmonella, Serratia, Shigella, Stenotrophomonas, Staphyloccocus, Streptococcus* und *Yersinia,* verursacht wird.

5. Verwendung nach Anspruch 4, wobei die bakterielle Infektion von *Acinetobacter baumanii, Bacillus anthracis, Campylobacter jejuni, Chlamydia trachomatis, Chlamydia pneumoniae, Clostridium* spp., *Citrobacter* spp., *Escherichia coli, Enterobacter* spp., *Enterococcus faecalis, Enterococcus faecium, Francisella tularensis, Haemophilus influenzae, Helicobacter pylori, Klebsiella* spp., *Listeria monocytogenes, Moraxella catarrhalis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneumoniae, Streptococcus pyogenes, Streptococcus agalactiae, Yersinia pestis* und *Yersinia enterocolitica* herrührt.

6. Verwendung nach irgendeinem der vorhergehenden Ansprüche, wobei R3 eine wahlweise substituierte Phenyl-, Pyridyl-, Benzo[1,3]dioxolyl-, 2,3-Dihydrobenzo[1,4]dioxin-, Pyrimidyl-, Pyrazyl-, Furanyl-, Pyrrolyl-, Thienyl-, Oxazolyl-, Isooxazolyl-, Thiazolyl-, Isothiazolyl-, Imidazolyl-, Naphthyl-, Chinolinyl-, Biphenyl-, Benzopyrimidyl-, Benzopyrazyl-, Benzofuranyl-, Indolyl-, Benzothienyl-, Benzoxazolyl-, Benzoisooxazolyl-, Benzothiazolyl-, Benzoisothiazolyl- oder Benzimidazolyl-Gruppe ist.

7. Verwendung nach irgendeinem der vorhergehenden Ansprüche, wobei R4 eine substituierte Phenyl-, Pyridyl-, Pyrimidyl-, Pyrazyl-, Naphthyl-, Biphenyl-, Phenyl-pyridyl-, Bipyridyl-, Chinolinyl-, Benzopyrimidyl-, Benzopyrazyl-, Cyclopropyl-, Cyclobutyl-, Cyclopentyl-, Cyclopentenyl-, Cyclohexyl-, Cyclohexenyl- oder C2-C8-AlkenylGruppe ist.

8. Verwendung nach Anspruch 7, wobei die Verbindung dargestellt wird durch Strukturformel **IV**: 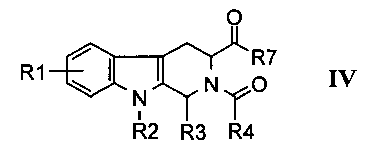 wobei:
R7 -OR^{o} ist oder -NR^{p}₂,
R^{o} -H oder wahlweise substituiertes Aryl, Aroyl, Aralkyl, Aralkanoyl, C1-C5-Alkyl oder C1-C5-Alkanoyl ist, und
jedes R^{p} unabhängig -H, C1-C5-Alkyl, Aryl oder Aralkyl ist, oder NR^{p}₂ eine nichtaromatische heterocyclische Gruppe ist.

9. Verwendung nach Anspruch 8, wobei **R4** dargestellt wird durch eine der Strukturformeln **R4-i** bis **R4-vii**: 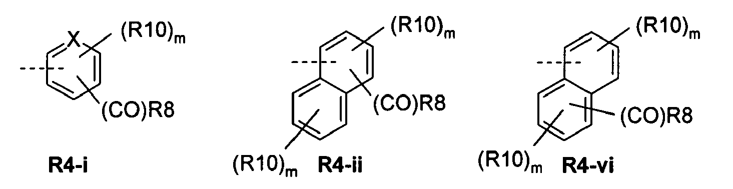 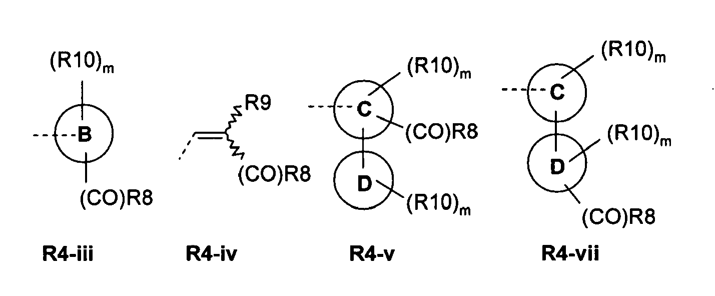 wobei:
jedes m unabhängig 0, 1, 2 oder 3 ist,
X -N-, -CH- oder -CR10- ist,
Ring **B** C3-C6-Cycloalkyl oder C3-C6-Cycloalkenyl ist,
Ringe **C** und **D** jeweils unabhängig Aryl oder Heteroaryl sind,
R8 -OR^{q} oder -NR^{r}₂ ist,
R9 -H, Aryl, Aralkyl oder C1-C6-aliphatisch ist,
jedes R10 unabhängig Halogen, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -NR^{k}(CO)NR^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂ oder wahlweise substituiertes Aryl, Aralkyl oder C1-C5-Alkyl ist,
jedes Rⁱ und R^{k} unabhängig -H, Aryl, Aralkyl, C1-C5-Alkyl oder C1-C5-Halogenalkyl ist,
jedes R^{j} und R^{r} unabhängig -H, Aryl, Aralkyl oder C1-C5-Alkyl ist, oder jedes NR^{j}₂ und NR^{r}₂ unabhängig eine nichtaromatische heterocyclische Gruppe ist,
R^{q} -H oder wahlweise substituiertes Aryl, Aroyl, Aralkyl, Aralkanoyl, C1-C5-Alkyl oder C1-C5-Alkanoyl ist, und
t 0 bis 5 ist.

10. Verwendung nach Anspruch 9, wobei R3 dargestellt wird durch eine der Strukturformeln **R3-i** bis **R3-v**: 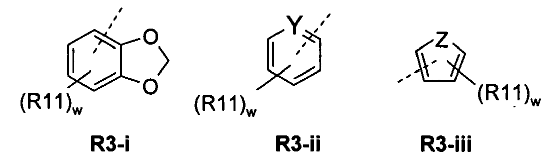 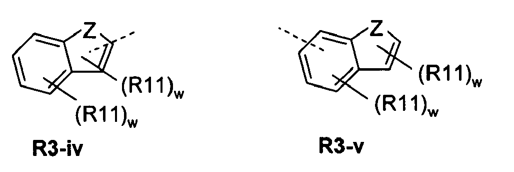 wobei:
Y -N-, -CH- oder -CR11- ist,
jedes Z unabhängig -NR^{z}-, -S- oder -O- ist, wobei R^{z} -H oder C1-C3-Alkyl ist,
w 0, 1, 2 oder 3 ist,
jedes R11 unabhängig Halogen, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ(CO)NR^{m}₂, -NRⁿ(CO)OR¹, -NRⁿCOR¹, -(SO₂)NR^{m}₂, -NRⁿSO₂R¹, -(CH₂)ᵤNR¹₂ oder wahlweise substituiertes Aryl, Aralkyl oder C1-C5-Alkyl ist,
jedes R¹ und Rⁿ unabhängig -H, Aryl oder Aralkyl, C1-C5-Alkyl oder C1-C5-Halogenalkyl ist,
jedes R^{m} unabhängig -H, Aryl, Aralkyl oder C1-C5-Alkyl ist, oder NR^{m}₂ eine nichtaromatische heterocyclische Gruppe ist, und
u 0 bis 5 ist.

11. Verwendung nach Anspruch 10, wobei R4 dargestellt wird durch eine der Strukturformeln **R4-i'** bis **R4-vii'**: 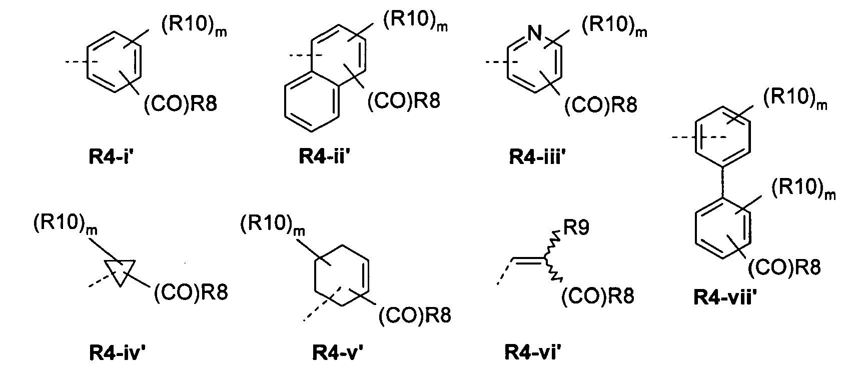 wobei:
jedes m unabhängig 0, 1, 2 oder 3 ist,
R8 -NR^{y}₂, -OH, C1-C5-Alkoxy oder C1-C5-Alkanoyloxy ist, wobei jedes R^{y} unabhängig -H oder C1-C3-Alkyl ist,
R9 -H oder C1-C6-aliphatisch ist, und
jedes R10 unabhängig -OH, -NO₂, -F, -Cl, -Br, C1-C4-Alkyl, C1-C4-Alkoxy, -CF₃ oder -OCF₃ ist.

12. Verwendung nach Anspruch 11, wobei R3 dargestellt wird durch eine der Strukturformeln **R3-i'** bis **R3-v'**: 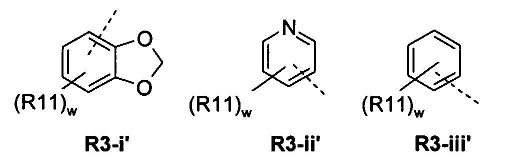 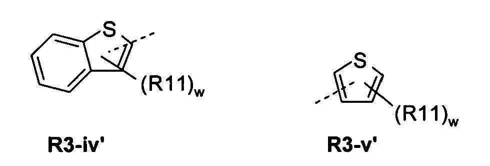 wobei:
w 0, 1,2 oder 3 ist, und
jedes R11 unabhängig -OH, -NO₂, -F, -Cl, -Br, C1-C4-Alkyl, C1-C4-Alkoxy, -CF₃ oder -OCF₃ ist.

13. Verwendung nach Anspruch 12, wobei R7 -NR^{x}₂, -OH, C1-C5-Alkoxy oder C1-C5-Alkanoyloxy ist, wobei jedes R^{x} unabhängig -H oder C1-C3-Alkyl ist.

14. Verwendung nach Anspruch 13, wobei:
R3 dargestellt wird durch eine der Strukturformeln **R3^{a}** bis **R3^{r}**: 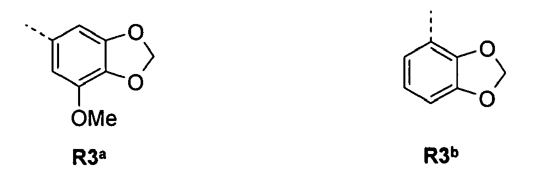 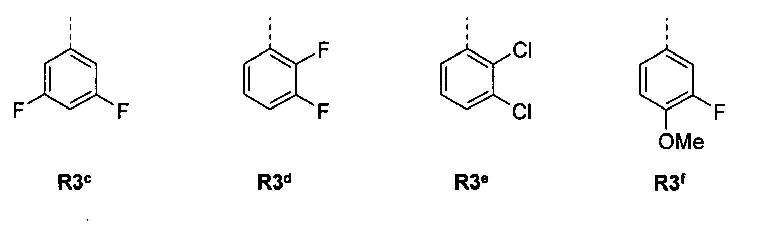 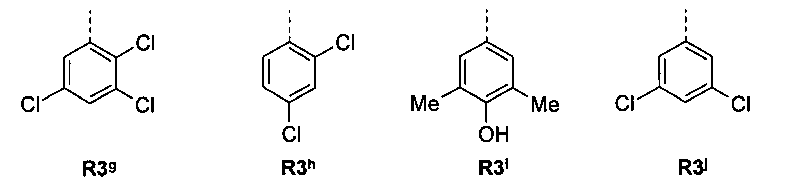 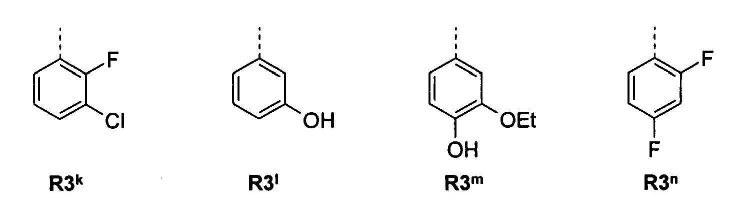 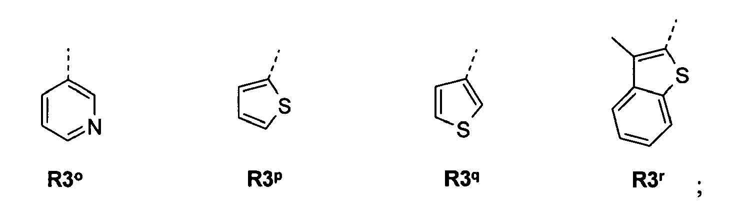
oder R4 dargestellt wird durch eine der Strukturformeln **R4^{a}** bis **R4^{q}**:

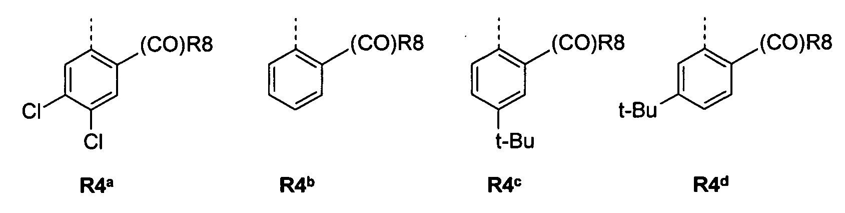

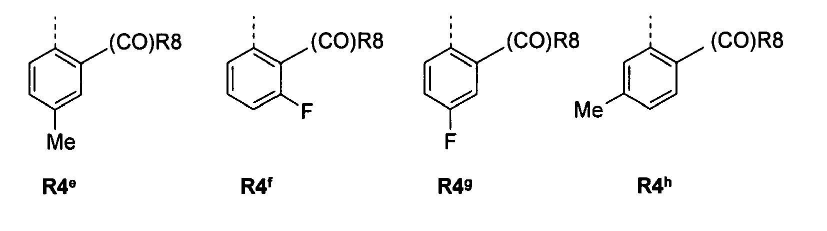

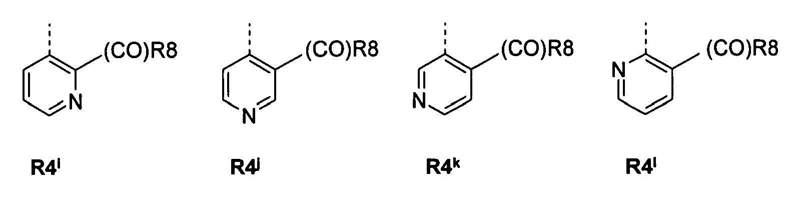

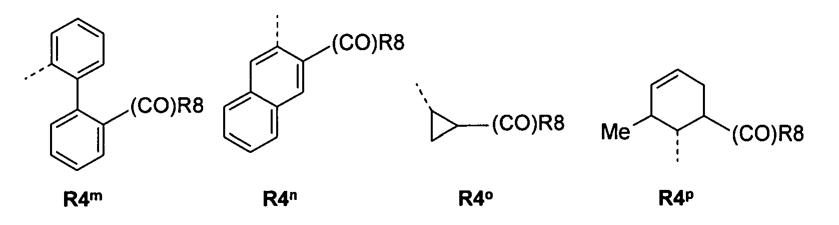

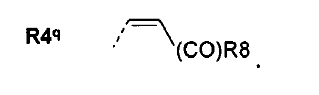

15. Verwendung nach Anspruch 14 wobei:
R3 dargestellt wird durch eine der Strukturformeln **R3^{a}** bis **R3^{r}**, und
R4 dargestellt wird durch eine der Strukturformeln **R4^{a}** bis **R4^{q}**.

16. Verwendung nach Anspruch 15, wobei die Verbindung dargestellt wird durch eine der Strukturformeln **V** bis **XI**: 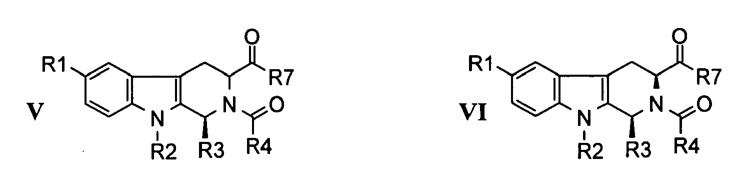 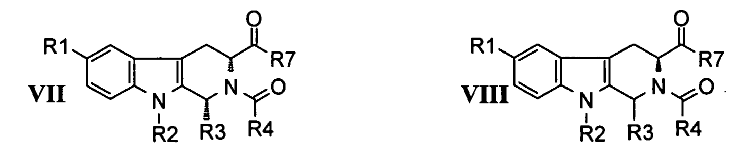 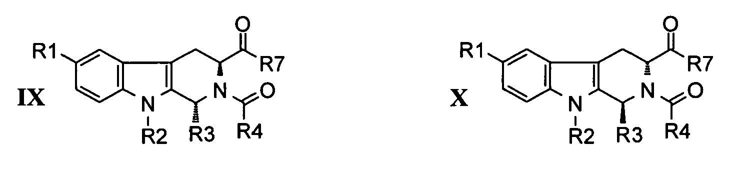 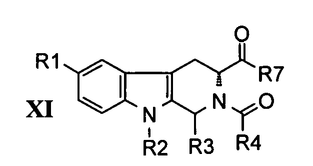 wobei R7 -OH oder C1-C4-Alkoxy ist.

17. Verwendung nach Anspruch 16, wobei R1 -H, -OH, -F, -CH₃, -CF₃, -OCH₃ oder -OCF₃ ist.

18. Verwendung nach Anspruch 17, wobei:
R3 dargestellt wird durch Strukturformel **R3^{d}**, **R3^{e}** oder **R3^{f}**; oder
R4 dargestellt wird durch Strukturformel **R4^{a}**, **R4^{c}** oder **R4^{e}**.

19. Verwendung nach Anspruch 17, wobei:
R3 dargestellt wird durch Strukturformel **R3^{d}**, **R3^{e}** oder **R3^{f}**; und
R4 dargestellt wird durch Strukturformel **R4^{a}**, **R4^{c}** oder **R4^{e}**.

20. Verwendung nach Anspruch 19, wobei R7 und R8 -OH sind.

21. Verwendung nach Anspruch 19, wobei eines von R7 oder R8 -OH ist und das andere -OCH₃ oder -OCH₂CH₃ ist.

22. Verwendung nach Anspruch 19, wobei R7 und R8 unabhängig -OCH₃ oder -OCH₂CH₃ sind.

23. Pharmazeutische Zusammensetzung, aufweisend eine Verbindung, dargestellt durch Strukturformel **I'**: 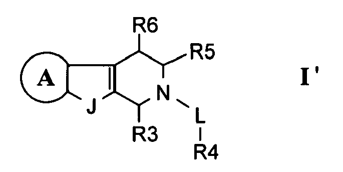 oder ein pharmazeutisch akzeptables Salz, Solvat oder Hydrat davon, wobei:
Ring A Phenyl ist, wahlweise substituiert an irgendeinem geeigneten Ringatom mit R1, wobei jedes R1 unabhängig Halogen, -CN, -NO₂, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂ oder wahlweise substituiertes Aryl, Aralkyl oder C1-C5-Alkyl ist,
wobei:
jedes R^{d} und R^{f} unabhängig -H, Aryl, Aralkyl, C1-C5-Alkyl oder C1-C5-Halogenalkyl ist,
jedes R^{e} unabhängig -H, Aryl, Aralkyl oder C1-C5-Alkyl ist, oder NR^{e}₂ eine nichtaromatische heterocyclische Gruppe ist, und
s 0 bis 5 ist;
J -NR2- ist, R2 -H ist oder wahlweise substituiertes C1-C5-Alkyl, und R3 wahlweise substituiertes Aryl, Aralkyl, Heteroaryl, Heteroaralkyl, C3-C7-cycloaliphatisch oder C3-C7-Cycloalkyl ist,
L -(CH₂)-, -(CO)-, -(CS)-, -(SO)- oder -(SO₂)- ist,
R4 eine Aryl-, Biaryl-, Heteroaryl-, Biheteroaryl-, Heteroaryl-aryl-, Aryl-heteroaryl-, Aralkyl-, Heteroaralkyl-, C1-C8-aliphatische, C3-C7-Cycloalkyl-, C5-C7-cycloaliphatische oder eine 3-7-gliedrige nicht-aromatische heterocyclische Gruppe ist, wobei die Gruppe, die durch R4 dargestellt wird, mit -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂ oder -NR^{c}SO₂R^{a} substituiert ist,
R5 -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO)(OR^{a})₂, -B(OR^{a})₂, -(CO)NR^{b}₂, -NR^{c}(CO)R^{a}, -SO₂NR^{b}₂ oder -NR^{c}SO₂R^{a} ist,
R6 -H, -OH, Halogen oder wahlweise substituiertes C1-C3-Alkyl oder Alkoxy ist, jedes R^{a} und R^{c} unabhängig -H, C1-C5-Alkyl, Aryl oder Aralkyl ist, und jedes R^{b} unabhängig -H, C1-C5-Alkyl, Aryl oder Aralkyl ist, oder NR^{b}₂ eine nicht-aromatische heterocyclische Gruppe ist,
wobei die optionalen Substituenten für Alkyl, aliphatisch, Cycloalkyl, cycloaliphatisch, Heterozyklus, Aryl, Heteroaryl, Heteroaralkyl und Aralkyl -OH, Halogen (-Br, -Cl, -I und -F), -R, -OR, -CH₂R, -CH₂CH₂R, -OCH₂R, -CH₂OR, -CH₂CH₂OR, -CH₂OC(O)R, -O-COR, -COR, -SR, -SCH₂R, -CH₂SR, -SOR, -SO₂R, -CN, -NO₂ -COOH, -SO₃H, -NH₂, -NHR, -N(R)₂, -COOR, -CH₂COOR, -CH₂CH₂COOR, -CHO, -CONH₂, -CONHR, -CON(R)₂, -NHCOR, -NRCOR, -NHCONH₂, -NHCONRH, -NHCON(R)₂, -NRCONH₂, -NRCONRH, -NRCON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C(=NH)-NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR sind, wobei k 0, 1 oder 2 ist, oder -NH-C(=NH)-NH₂ sind, wobei jedes R unabhängig eine Alkyl-, Cycloalkyl-, Benzyl-, aromatische, heteroaromatische oder *N-*Anilinyl-Gruppe ist, oder -N(R)₂ zusammengenommen auch eine heterocyclische Gruppe bilden kann, und Substituenten am Stickstoff einer heterocyclischen Gruppe oder heteroaromatischen Gruppe -R', -N(R')₂, -C(O)R', -CO₂ R', -C(O)C(O)R', -C(O)CH₂ C(O)R', -SO₂R', -SO₂N(R')₂, -C(=S)N(R')₂, -C(=NH)-N(R')₂ und -NR'SO₂R' beinhalten, wobei R' Wasserstoff, Alkyl, Alkoxy, Cycloalkyl, Cycloalkoxy, Phenyl, Phenoxy, Benzyl, Benzyloxy, heteroaromatische oder heterocyclische Gruppe ist.

24. Zusammensetzung nach Anspruch 23, wobei R3 eine wahlweise substituierte Phenyl-, Pyridyl-, Benzo[1,3]dioxolyl-, 2,3-Dihydrobenzo[1,4]dioxin-, Pyrimidyl-, Pyrazyl-, Furanyl-, Pyrrolyl-, Thienyl-, Oxazolyl-, Isooxazolyl-, Thiazolyl-, Isothiazolyl-, Imidazolyl-, Naphthyl-, Chinolinyl-, Biphenyl-, Benzopyrimidyl-, Benzopyrazyl-, Benzofuranyl-, Indolyl-, Benzothienyl-, Benzoxazolyl-, Benzoisooxazolyl-, Benzothiazolyl-, Benzoisothiazolyl- oder Benzimidazolyl-Gruppe ist.

25. Zusammensetzung nach Anspruch 24, wobei R4 eine substituierte Phenyl-, Pyridyl-, Pyrimidyl-, Pyrazyl-, Naphthyl-, Biphenyl-, Phenyl-pyridyl-, Chinolinyl-, Benzopyrimidyl-, Benzopyrazyl-, Cyclopropyl-, Cyclobutyl-, Cyclopentyl-, Cyclopentenyl-, Cyclohexyl-, Cyclohexenyl- oder C2-C8-Alkenyl-Gruppe ist.

26. Zusammensetzung nach Anspruch 25, wobei die Verbindung dargestellt wird durch die Strukturformel **IV**: 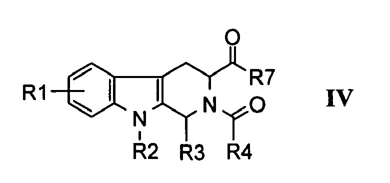 wobei:
R7 -OR^{o} oder -NR^{P}₂ ist,
R^{o} -H oder wahlweise substituiertes Aryl, Aroyl, Aralkyl, Aralkanoyl, C1-C5-Akyl oder C1-C5-Alkanoyl ist, und
jedes R^{p} unabhängig -H, C1-C5-Alkyl, Aryl oder Aralkyl ist, oder NR^{P}₂ eine nichtaromatische heterocyclische Gruppe ist.

27. Zusammensetzung nach Anspruch 26, wobei R4 dargestellt wird durch eine der Strukturformeln **R4-i** bis **R4-vii**: 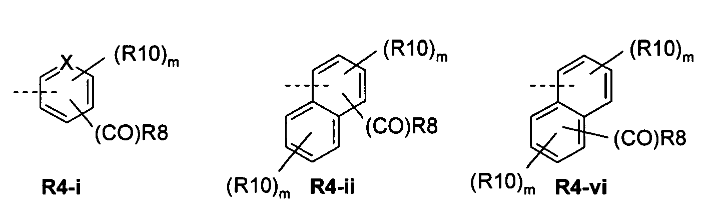 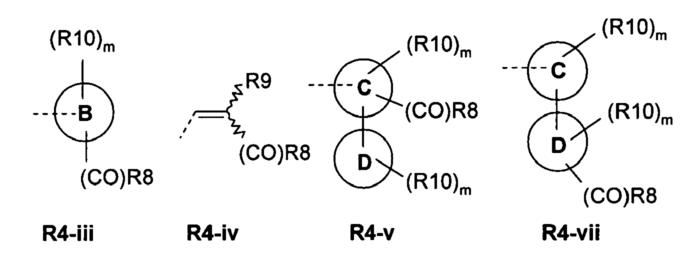 wobei:
jedes m unabhängig 0, 1, 2 oder 3 ist
X -N-, -CH- oder -CR10- ist,
Ring **B** C3-C6-Cycloalkyl oder C3-C6-Cycloalkenyl ist,
Ring **C** Phenyl ist,
Ring **D** Aryl oder Heteroaryl ist.
R8-OR^{q} oder NR^{r}₂ ist,
R9 -H, Aryl, Aralkyl oder C1-C6-aliphatisch ist,
jedes R10 unabhängig Halogen, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -NR^{k}(CO)NR^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂ oder wahlweise substituiertes Aryl, Aralkyl oder C1-C5-Alkyl ist,
jedes Rⁱ und R^{k} unabhängig -H, Aryl, Aralkyl, C1-C5-Alkyl oder C1-C5-Halogenalkyl ist,
jedes R^{j} und R^{r} unabhängig -H, Aryl, Aralkyl oder C1-C5-Alkyl ist, oder jedes NR^{j}₂ und NR^{r}₂ unabhängig eine nichtaromatische heterocyclische Gruppe ist,
R^{q} -H ist oder wahlweise substituiertes Aryl, Aroyl, Aralkyl, Aralkanoyl, C1-C5-Alkyl oder C1-C5-Alkanoyl ist, und
t 0 bis 5 ist.

28. Zusammensetzung nach Anspruch 27, wobei R3 dargestellt wird durch eine der Strukturformeln **R3-i** bis **R3-v**: 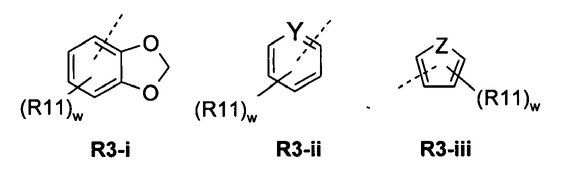 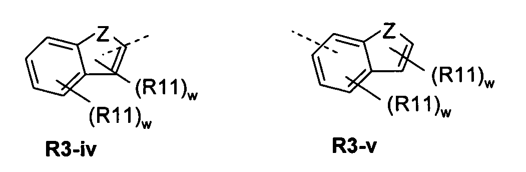 wobei:
Y -N-, -CH- oder -CR11- ist,
jedes Z unabhängig -NR^{z}-, -S- oder -O- ist, wobei R^{z} -H oder C1-C3-Alkyl ist,
w 0, 1, 2 oder 3 ist,
jedes R11 unabhängig Halogen, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ(CO)NR^{m}₂ -NRⁿ(CO)OR¹, -NRⁿCOR¹, -(SO₂NR^{m}₂ -NRⁿSO₂R¹, -(CH₂)ᵤNR¹₂oder wahlweise substituiertes Aryl, Aralkyl oder C1-C5-Alkyl ist,
jedes R¹ und Rⁿ unabhängig -H, Aryl oder Aralkyl, C1-C5-Alkyl oder C1-C5-Halogenalkyl ist,
jedes R^{m} unabhängig -H, Aryl, Aralkyl oder C1-C5-Alkyl ist, oder NR^{m}₂ eine nichtaromatische heterocyclische Gruppe ist, und
u 0 bis 5 ist.

29. Zusammensetzung nach Anspruch 28, wobei **R4** dargestellt wird durch eine der Strukturformeln **R4-i'** bis **R4-vii'**: 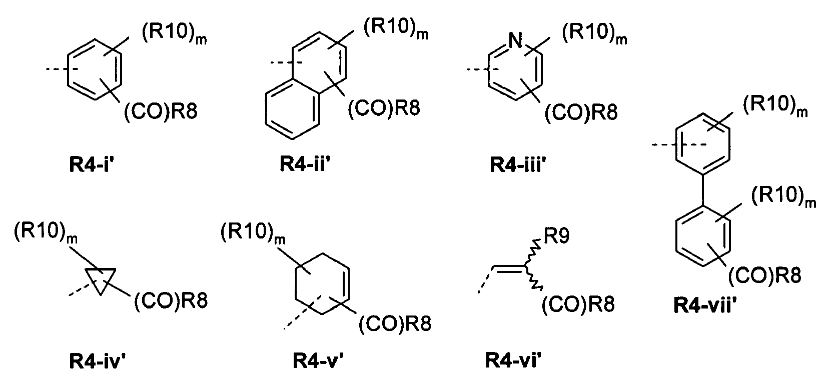 wobei:
jedes m unabhängig 0, 1, 2 oder 3 ist,
R8 -NR^{y}₂, -OH, C1-C5-Alkoxy oder C1-C5-Alkanoyloxy ist, wobei jedes R^{y} unabhängig -H oder C1-C3-Alkyl ist,
R9 -H oder C1-C6-aliphatisch ist, und
jedes R10 unabhängig -OH, -NO₂, -F, -Cl, -Br, C1-C4-Alkyl, C1-C4-Alkoxy, -CF₃ oder -OCF₃ ist.

30. Zusammensetzung nach Anspruch 29, wobei R3 dargestellt wird durch Strukturformeln **R3-i'** bis **R3-v'**: 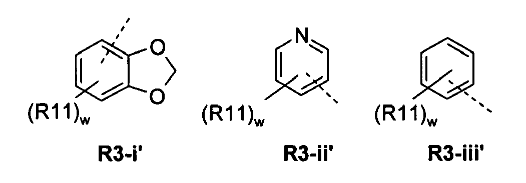 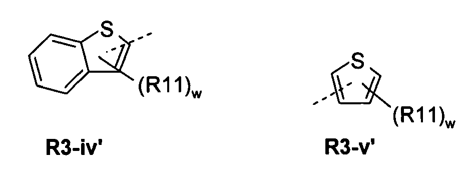 wobei:
w 0, 1, 2 oder 3 ist, und
jedes R11 unabhängig -OH, -NO₂, -F, -Cl, -Br, C1-C4-Alkyl, C1-C4-Alkoxy, -CF₃ oder -OCF₃ ist.

31. Zusammensetzung nach Anspruch 30, wobei R7 -NR^{x}₂, -OH, C1-C5-Alkoxy oder C1-C5-Alkanoyloxy ist, wobei jedes R^{x} unabhängig -H oder C1-C3-Alkyl ist.

32. Zusammensetzung nach Anspruch 31, wobei:
R3 dargestellt wird durch eine der Strukturformeln **R3^{a}** bis **R3^{r}**: 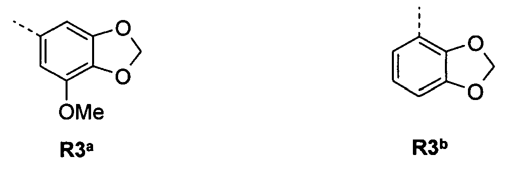 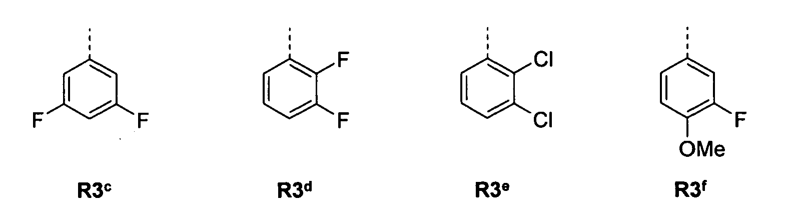 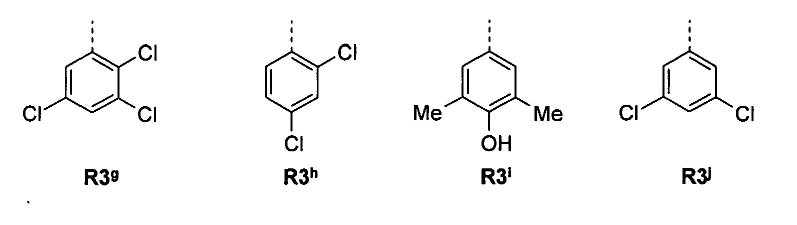 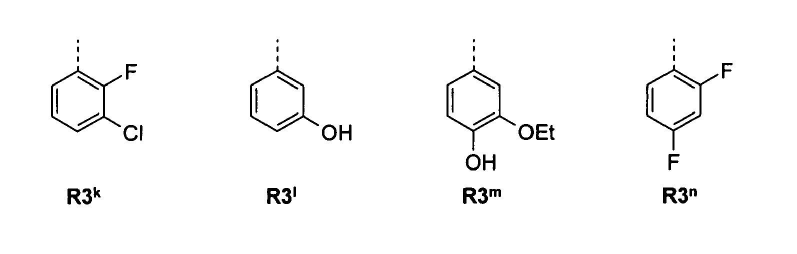 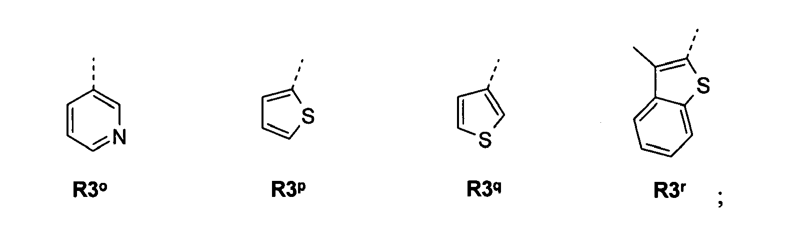
oder R4 dargestellt wird durch eine der Strukturformeln **R4^{a}** bis **R4^{q}**:

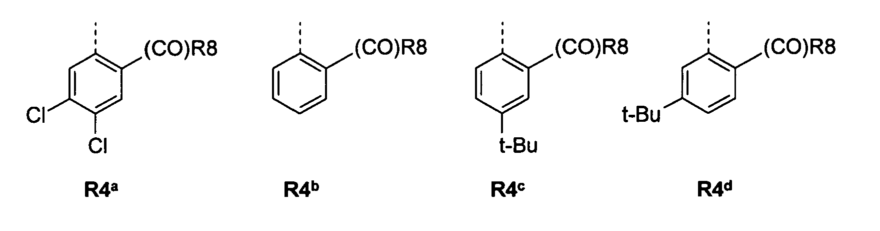

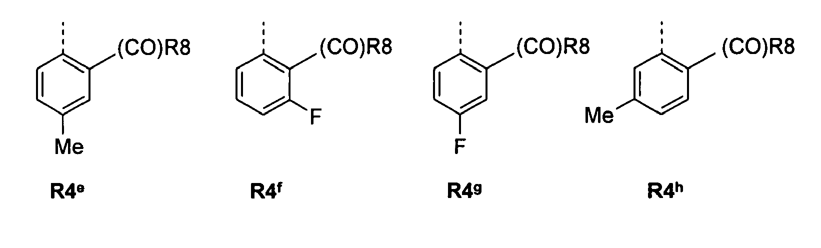

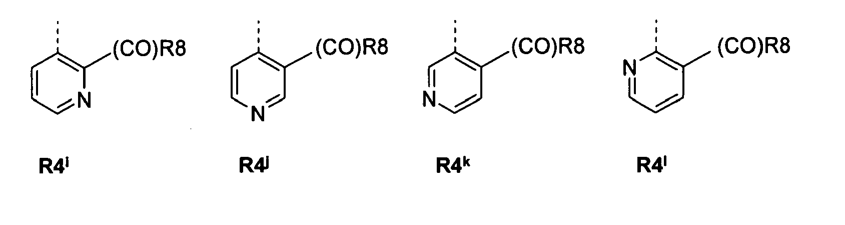

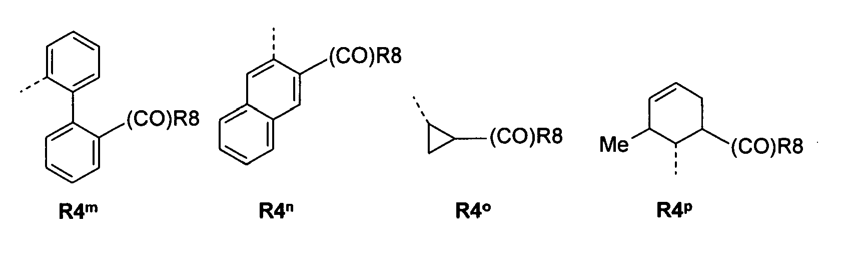

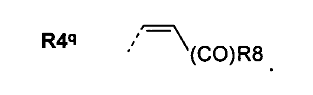

33. Zusammensetzung nach Anspruch 32, wobei:
R3 dargestellt wird durch eine der Strukturformeln **R3^{a}** bis **R3^{r}**, und
R4 dargestellt wird durch eine der Strukturformeln **R4^{a}** bis **R4^{q}**.

34. Zusammensetzung nach Anspruch 33, wobei die Verbindung dargestellt wird durch eine der Strukturformeln **V** bis **XI**: 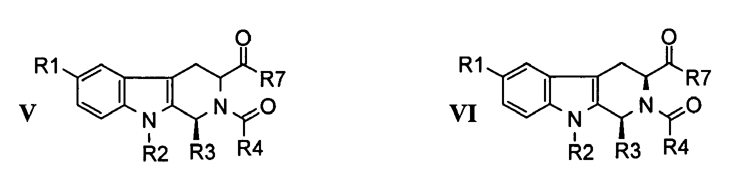 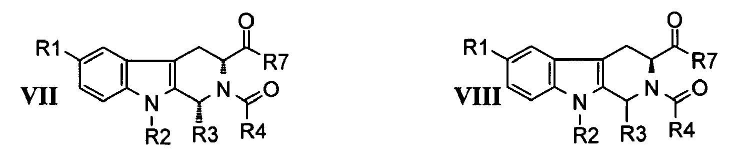 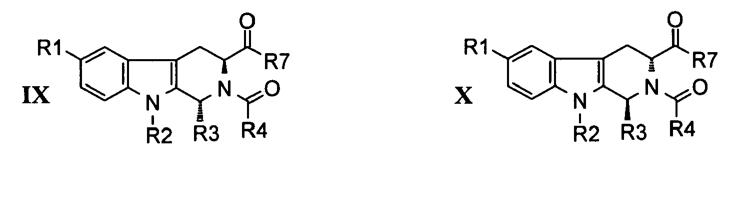 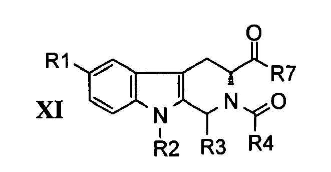 wobei R7 -OH oder C1-C4-Alkoxy ist.

35. Zusammensetzung nach Anspruch 34, wobei R1 -H, -OH, -F, -CH₃, -CF₃, -OCH₃ oder -OCF₃ ist.

36. Zusammensetzung nach Anspruch 35, wobei:
R3 dargestellt wird durch Strukturformel **R3^{d}**, **R3^{e}** oder **R3^{f}**, oder
R4 dargestellt wird durch Strukturformel **R4^{a}**, **R4^{c}** oder **R4^{e}**.

37. Zusammensetzung nach Anspruch 35, wobei:
R3 dargestellt wird durch Strukturformel **R3^{d}**, **R3^{e}** oder **R3^{f}**, und
R4 dargestellt wird durch Strukturformel **R4^{a}**, **R4^{c}** oder **R4^{e}**.

38. Zusammensetzung nach Anspruch 37, wobei R7 und R8 -OH sind.

39. Zusammensetzung nach Anspruch 37, wobei eines von R7 oder R8 -OH ist und das andere -OCH₃ oder -OCH₂CH₃ ist.

40. Zusammensetzung nach Anspruch 37, wobei R7 und R8 unabhängig -OCH₃ oder -OCH₂CH₃ sind.

## Revendications

1. Utilisation d'une quantité efficace d'un composé représenté par la formule développée I : 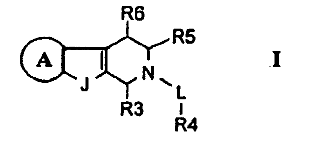 ou d'un sel pharmaceutiquement acceptable, d'un solvate ou d'un hydrate de celui-ci, dans laquelle le cycle A est un groupe phényle, éventuellement substitué par R1 sur n'importe quelle atome du cycle pouvant être substitué, où chaque R1 est indépendamment un atome d'halogène, -CN, -NO₂, -OR^{d}, -(CO)R^{d}, -(CO)OR^{d}, -O(CO)R^{d}, -(CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, ou un groupe aryle, aralkyle ou alkyle en C₁-C₅ éventuellement substitué ;
où
chaque R^{d} et R^{f} est indépendamment -H, un groupe aryle, aralkyle, alkyle en C₁-C₅ ou halogénoalkyle en C₁-C₅ ;
chaque R^{e} est indépendamment -H, un groupe aryle, aralkyle ou alkyle en C₁-C₅, ou NR^{e}₂ est un groupe hétérocyclique non aromatique, et s vaut 0 à 5 ;
J est -NR2-, R2 est -H ou un groupe alkyle en C₁-C₅ éventuellement substitué, et R3 est un groupe aryle, aralkyle, hétéroaryle, hétéroaralkyle, cycloaliphatique en C₃-C₇ ou cycloalkyle en C₃-C₇ éventuellement substitué ;
L est -(CH₂)-, -(CO)-, -(CS)-, -(SO)- ou -(SO₂)- ;
R4 est un groupe aryle, biaryle, hétéroaryle, bihétéroaryle, hétéroaryl-aryle, aryl-hétéroaryle, aralkyle, hétéroalkyle, aliphatique en C₁-C₈, cycloalkyle en C₃-C₇, cycloaliphatique en C₅-C₇, ou hétérocyclique non aromatique de 3 à 7 chaînons, où le groupe représenté par R4 est substitué par -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})₂, -O(PO) (OR^{a})₂, -B(OR^{a})₂, -(CO)NR^{b}₂, -NR^{c}(CO)R^{a}, -SO₂NR^{b}₂, ou -NR^{c}SO₂R^{a} ;
R5 est -(CO)OR^{a}, - (CO)O(CO)R^{a}, -(CS)OR^{a}, -(SO)-OR^{a}, -SO₃R^{a}, -OSO₃R^{a}, -P(OR^{a})₂, -(PO)(OR^{a})₂, -O-(PO)(OR^{a})₂, -B(OR^{a})₂, -(CO)NR^{b}_{2,} -NR^{c}(CO)R^{a}, -SO₂N-R^{b}₂, ou -NR^{c}SO₂R^{a} ;
R6 est -H, -OH, un atome d'halogène ou un groupe alkyle ou alcoxy en C₁-C₃ éventuellement substitué ;
chaque R^{a} et R^{c} est indépendamment -H, un groupe aryle, aralkyle ou alkyle en C₁-C₅ ; et chaque R^{b} est indépendamment -H, un groupe aryle, aralkyle ou alkyle en C₁-C₅, ou NR^{b}₂ est un groupe hétérocyclique non aromatique ;
où les substituants éventuels pour les groupes alkyles, aliphatiques, cycloalkyles, cycloaliphatiques, hétéro-cycliques, aryles, hétéroaryles, hétéroaralkyles et aralkyles sont -OH, les atomes d'halogène (-Br, -Cl, -I et -F), -R, -OR, -CH₂R, -CH₂CH₂R, -OCH₂R, -CH₂OR, -CH₂CH₂OR, -CH₂OC(O)R, -O-COR, -COR, -SR, -SCH₂R, -CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CON-H₂, -CONHR, -CON(R)₂, -NHCOR, -NRCOR, -NHCONH₂, -NHCONRH, -NHCON(R)₂, -NRCONH₂, -NRCONRH, -NR-CON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C(=NR)-NH₂, -NH-C(=NR)-NHR, -NH-C(=NR)-N(R)₂, -NRH-C(=NH)-NH₂, -NR-C (=NH) -NHR, -NR-C(=NH)-N(R)₂, -NR-C(=NR)-NH₂, -NR-C(=NR)-NHR, -NR-C(=NR)-N(R)₂, -SO₂NH₂, -SO₂NHR, -SO₂NR₂, -SH, -SOₖR, où k vaut 0, 1 ou 2 ou -NH-C(=NH)-NH₂ ; où chaque R est indépendamment un groupe alkyle, cycloalkyle, benzyle, aromatique, hétéroaromatique ou N-anilinyle ou -N(R)₂, pris ensemble, peut également former un groupe hétérocyclique ; et les substituants sur l'atome d'azote d'un groupe hétérocyclique ou d'un groupe hétéroaromatique comprennent -R', -N(R')₂, -C(O)R', -CO₂R', -C(O)C(O)R', -C(O)CH₂C(O)R', -SO₂R', -SO₂N(R')₂, -C(=S)N(R')₂, -C(=NH)-N(R')₂ et -NR'SO₂R', où R' est un atome d'hydrogène, un groupe alkyle, alcoxy, cycloalkyle, cycloalcoxy, phényle, phénoxy, benzyle, benzyloxy, hétéroaromatique ou hétérocyclique,
pour la fabrication d'un médicament destiné au traitement d'un sujet pour une infection bactérienne.

2. Utilisation selon la revendication 1, dans laquelle le sujet est un être humain.

3. Utilisation selon la revendication 2, dans laquelle l'infection est provoquée par une bactérie qui exprime la phosphopantéthéine adénylytransférase.

4. Utilisation selon la revendication 2, dans laquelle l'infection est provoquée par une bactérie d'un genre choisi parmi *Acinetobacter, Bacillus, Campylobacter, Chlamydia, Chlamydophila, Clostridium, Citrobacter, Escherichia, Enterobacter, Enterococcus,Francisella,Haemophilus, Helicobacter, Klebsiella, Listeria, Moraxella, Mycobacterium, Neisseria, Proteus, Pseudomonas, Salmonella, Serratia, Shigella, Stenotrophomonas,Staphyloccocus, Streptococcus,* et *Yersina.*

5. Utilisation selon la revendication 4, dans laquelle l'infection bactérienne est choisie parmi *Acinetobacter baumanii, Bacillus anthracis, Campylobacter jejuni, Chlamydia trachomatis, Chlamydia pneumoniae, Clostridium* spp. , *Citrobacter* spp., *Escherichia coli, Enterobacter* spp., *Enterococcus faecalis., Enterococcus faecium, Francisella tularensis, Haemophilus influenzae, Helicobacter pylori, Klebsiella* spp., *Listeria monocytogenes, Moraxella catarrhalis, Mycobacterium tuberculosis, Neisseria meningitidis, Neisseria gonorrhoeae, Proteus mirabilis, Proteus vulgaris, Pseudomonas aeruginosa, Salmonella* spp., *Serratia* spp., *Shigella* spp., *Stenotrophomonas maltophilia, Staphyloccocus aureus, Staphyloccocus epidermidis, Streptococcus pneumoniae, Streptococcus pyogenes, Streptococcus agalactiae, Yersina pestis,* et *Yersina enterocolitica.*

6. Utilisation selon l'une quelconque des revendications précédentes, dans laquelle R3 est un groupe phényle, pyridyle, benzo[1,3]dioxolyle, 2,3-dihydrobenzo[1,4]dioxine, pyrimidyle, pyrazyle, furanyle, pyrrolyle, thiényle, oxazolyle, isooxazolyle, thiazolyle, isothiazolyle, imidazolyle, naphtyle, quinoléinyle, biphényle, benzopyrimidyle, benzopyrazyle, benzofuranyle, indolyle, benzothiényle, benzoxazolyle, benzoisooxazolyle, benzothiazolyle, benzoisothiazolyle, ou benzimidazolyle éventuellement substitué.

7. Utilisation selon l'une quelconque des revendications précédentes, dans laquelle R4 est un groupe phényle, pyridyle, pyrimidyle, pyrazyle, naphtyle, biphényle, phénylpyridyle, bipyridyle, quinoléinyle, benzopyrimidyle, benzopyrazyle, cyclopropyle, cyclobutyle, cyclopentyle, cyclopentényle, cyclohexyle, cyclohexényle, ou alcényle en C₂-C₈ substitué.

8. Utilisation selon la revendication 7, dans laquelle le composé est représenté par la formule structurelle IV : 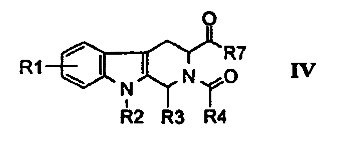 dans laquelle
R7 est -OR^{o} ou -NR^{p}₂ ;
R^{o} est -H ou un groupe aryle, aroyle, aralkyle, aralcanoyle, alkyle en C₁-C₅ ou alcanoyle en C₁-C₅ éventuellement substitué ; et chaque R^{p} est indépendamment -H, un groupe aryle, aralkyle ou alkyle en C₁-C₅, ou NR^{p}₂ est un groupe hétérocyclique non aromatique.

9. Utilisation selon la revendication 8, dans laquelle R4 est représenté par l'une des formules développées R4-i à R4-vii : 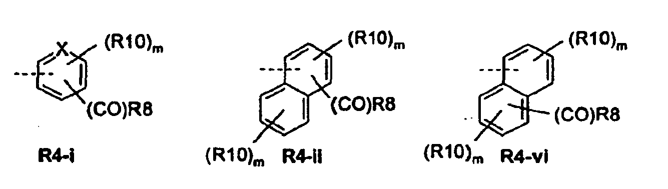 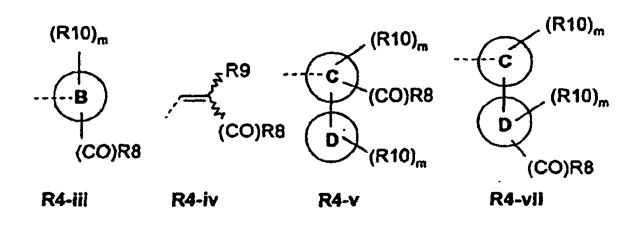 dans laquelle
chaque m vaut indépendamment 0, 1, 2 ou 3 ;
X est -N-, -CH-, ou -CR10- ;
le cycle B est un groupe cycloalkyle en C₃-C₆ ou cycloalcényle en C₃-C₆ ;
les cycles C et D sont chacun indépendamment un groupe aryle ou hétéroaryle ;
R8 est -OR^{q} ou -NR^{r}₂ ;
R9 est -H, un groupe aryle, aralkyle ou aliphatique en C₁-C₆ ;
chaque R10 est indépendamment un atome d'halogène, -CN, -NO₂, -CF₃, -OCF₃, -ORⁱ, -(CO)Rⁱ, -(CO)ORⁱ, -O(CO)Rⁱ, -(CO)O(CO)Rⁱ, -(CS)ORⁱ, -(SO)ORⁱ, -SO₃Rⁱ, -CONR^{j}₂, -O(CO)NR^{j}₂, -NR^{k}(CO)-NR^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂, ou un groupe aryle, aralkyle ou alkyle en C₁-C₅ éventuellement substitué ;
chaque Rⁱ et R^{k} est indépendamment -H, un groupe aryle, aralkyle, alkyle en C₁-C₅ ou halogénoalkyle en C₁-C₅ ;
chaque R^{j} et R^{r} est indépendamment -H, un groupe aryle, aralkyle ou alkyle en C₁-C₅, ou chaque NR^{j}₂ et NR^{r}₂ est indépendamment un groupe hétérocyclique non aromatique ;
R^{q} est -H ou un groupe aryle, aroyle, aralkyle, aralcanoyle, alkyle en C₁-C₅ ou alcanoyle en C₁-C₅ éventuellement substitué ; et
t vaut 0 à 5.

10. Utilisation selon la revendication 9, dans laquelle R3 est représenté par l'une des formules développées R3-i à R3-v : 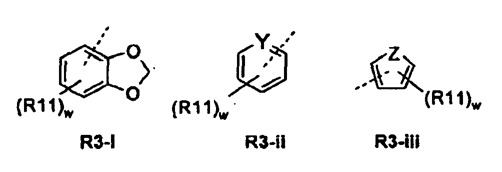 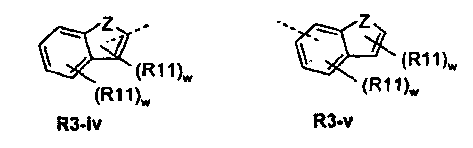 dans laquelle
Y est -N-, -CH-, ou -CR11- ;
chaque Z est indépendamment -NR^{z}-, -S-, ou -O-, où R^{z} est -H ou un groupe alkyle en C₁-C₃ ;
w vaut 0, 1, 2 ou 3 ;
chaque R11 est indépendamment un atome d'halogène, -CN, -NO₂, -CF₃, -OCF₃, -OR¹, -(CO)R¹, -(CO)OR¹, -O(CO)R¹, -(CO)O(CO)R¹, -(CS)OR¹, -(SO)OR¹, -SO₃R¹, -CONR^{m}₂, -O(CO)NR^{m}₂, -NRⁿ-(CO)NR^{m}₂, -NRⁿ(CO)OR¹, -NRⁿCOR¹, -(SO₂)NR^{m}₂, -NRⁿ-SO₂R¹, -(CH₂)ᵤNR¹₂, ou un groupe aryle, aralkyle ou alkyle en C₁-C₅ éventuellement substitué ;
chaque R¹ et Rⁿ est indépendamment -H, un groupe aryle, aralkyle, alkyle en C₁-C₅ ou halogénoalkyle en C₁-C₅ ;
chaque R^{m} est indépendamment -H, un groupe aryle, aralkyle ou alkyle en C₁-C₅, ou NR^{m}₂ est un groupe hétérocyclique non aromatique ; et u vaut 0 à 5.

11. Utilisation selon la revendication 10, dans laquelle R4 est représenté par l'une des formules développées R4-i' à R4-vii' : 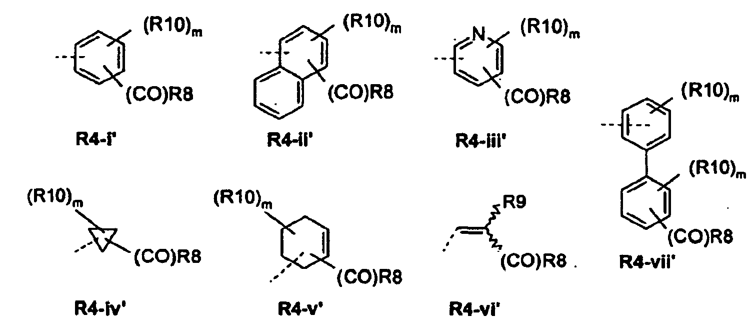 dans laquelle
chaque m vaut indépendamment 0, 1, 2 ou 3 ;
R8 est -NR^{y}₂, -OH, un groupe alcoxy en C₁-C₅ ou un groupe alcanoyloxy en C₁-C₅, où chaque R^{y} est indépendamment -H ou un groupe alkyle en C₁-C₃ ;
R9 est -H ou un groupe aliphatique en C₁-C₆ ; et chaque R10 est indépendamment -OH, -NO₂, -F, -Cl, -Br, un groupe alkyle en C₁-C₄, un groupe alcoxy en C₁-C₄, -CF₃, ou -OCF₃.

12. Utilisation selon la revendication 11, dans laquelle R3 est représenté par l'une des formules développées R3-i' à R3-v' : 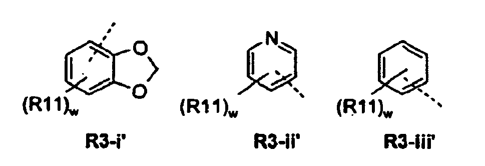 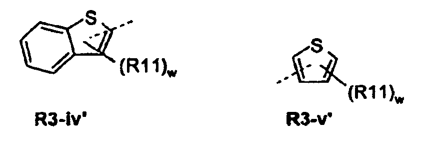 dans laquelle
w vaut indépendamment 0, 1, 2 ou 3 ; et chaque R11 est indépendamment -OH, -NO₂, -F, -Cl, -Br, un groupe alkyle en C₁-C₄, un groupe alcoxy en C₁-C₄, -CF₃, ou -OCF₃.

13. Utilisation selon la revendication 12, dans laquelle R7 est -NR^{x}₂, -OH, un groupe alcoxy en C₁-C₅ ou un groupe alcanoyloxy en C₁-C₅, où chaque R^{x} est indépendamment -H ou un groupe alkyle en C₁-C₃.

14. Utilisation selon la revendication 13, dans laquelle
R3 est représenté par l'une des structures développées R3^{a} à R3^{r} : 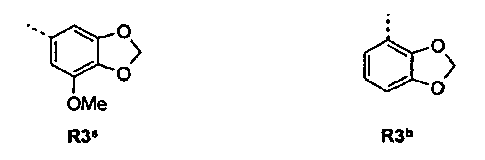 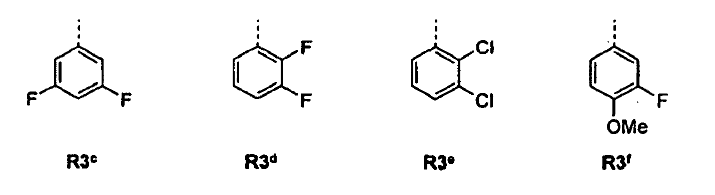 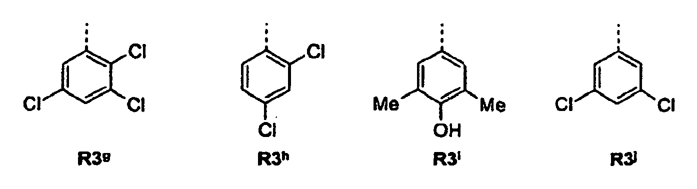 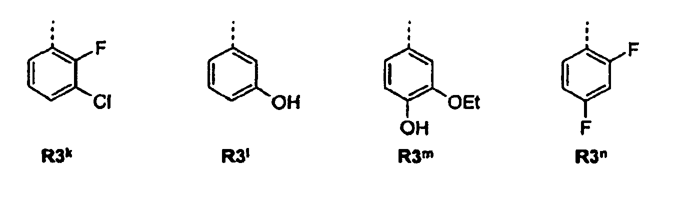 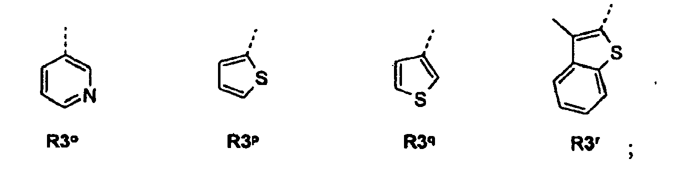 ou R4 est représenté par l'une des structures développées R4^{a} à R4^{q} :

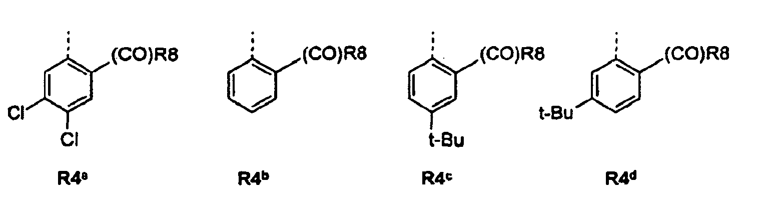

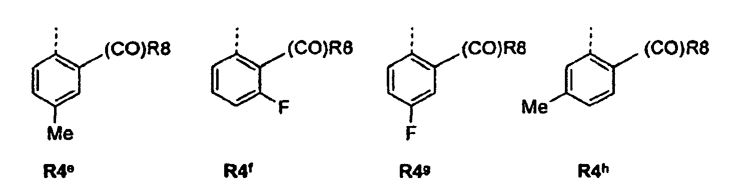

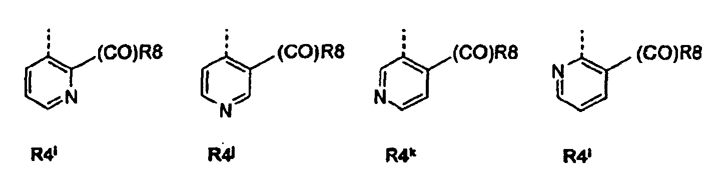

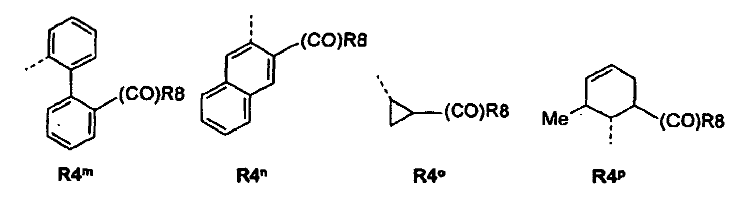

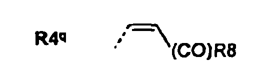

15. Utilisation selon la revendication 14, dans laquelle
R3 est représenté par l'une des structures développées R3^{a} à R3^{r} ; et
R4 est représenté par l'une des structures développées R4^{a} à R4^{q}.

16. Utilisation selon la revendication 15, dans laquelle le composé est représenté par l'une des formules développées V à XI : 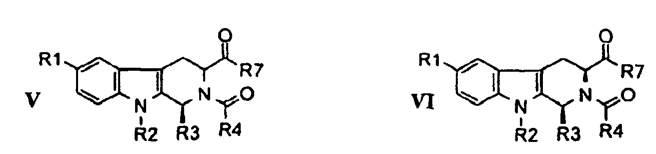 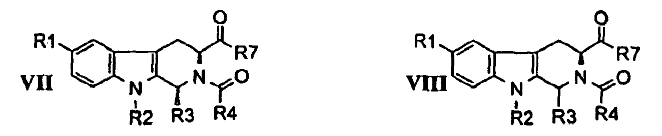 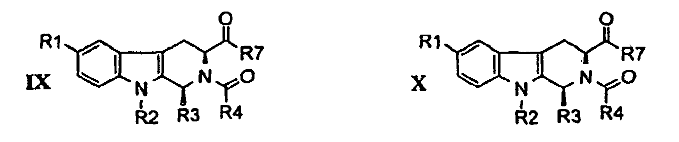 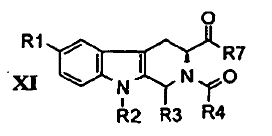 où R7 est -OH ou un groupe alcoxy en C₁-C₄.

17. Utilisation selon la revendication 16, dans laquelle R1 est -H, -OH, -F, -CH₃, -CF₃, -OCH₃ ou -OCF₃.

18. Utilisation selon la revendication 17, dans laquelle
R3 est représenté par la structure développée R3^{d}, R3^{e} ou R3^{f} ; ou
R4 est représenté par la structure développée R4^{a,} R4^{c} ou R4^{e}.

19. Utilisation selon la revendication 17, dans laquelle
R3 est représenté par la structure développée R3^{d}, R3^{e} ou R3^{f} ; et
R4 est représenté par la structure développée R4^{a}, R4^{c} ou R4^{e}.

20. Utilisation selon la revendication 19, dans laquelle R7 et R8 sont -OH.

21. Utilisation selon la revendication 19, dans laquelle l'un de R7 et R8 est -OH, et l'autre est -OCH₃ ou -OCH₂CH₃.

22. Utilisation selon la revendication 19, dans laquelle R7 et R8 sont indépendamment -OCH₃ ou - OCH₂CH₃.

23. Composition pharmaceutique comprenant un composé représenté par la formule développée I' : 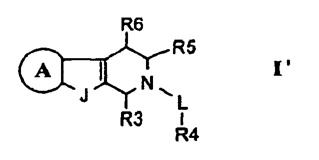 ou un sel pharmaceutiquement acceptable, un solvate ou un hydrate de celui-ci, dans laquelle le cycle A est un groupe phényle, éventuellement substitué par R1 sur n'importe quelle atome du cycle pouvant être substitué, où chaque R1 est indépendamment un atome d'halogène, -CN, -NO₂, -OR^{d}, -(CO)R^{d}, -(CO)OR^{d}, -O(CO)R^{d}, - (CO)O(CO)R^{d}, -(CS)OR^{d}, -(SO)OR^{d}, -SO₃R^{d}, -CONR^{e}₂, -O(CO)NR^{e}₂, -NR^{f}(CO)NR^{e}₂, -NR^{f}(CO)OR^{d}, -NR^{f}COR^{d}, -(SO₂)NR^{e}₂, -NR^{f}SO₂R^{d}, -(CH₂)ₛNR^{d}₂, ou un groupe aryle, aralkyle ou alkyle en C₁-C₅ éventuellement substitué ;
où
chaque R^{d} et R^{f} est indépendamment -H, un groupe aryle, aralkyle, alkyle en C₁-C₅ ou halogénoalkyle en C₁-C₅ ;
chaque R^{e} est indépendamment -H, un groupe aryle, aralkyle ou alkyle en C₁-C₅, ou NR^{e}₂ est un groupe hétérocyclique non aromatique, et s vaut 0 à 5 ;
J est -NR2-, R2 est -H ou un groupe alkyle en C₁-C₅ éventuellement substitué, et R3 est un groupe aryle, aralkyle, hétéroaryle, hétéro-aralkyle, cycloaliphatique en C₃-C₇ ou cycloalkyle en C₃-C₇ éventuellement substitué ;
L est -(CH₂)-, -(CO)-, -(CS)-, -(SO)- ou -(SO₂)- ;
R4 est un groupe aryle, biaryle, hétéroaryle, bihétéroaryle, hétéroaryl-aryle, aryl-hétéroaryle, aralkyle, hétéroalkyle, aliphatique en C₁-C₈, cycloalkyle en C₃-C₇, cycloaliphatique en C₅-C₇, ou hétérocyclique non aromatique de 3 à 7 chaînons, où le groupe représenté par R4 est substitué par -(CO)OR^{a}, -(CO)O(CO)R^{a}, -(CS)OR^{a}, -(SO)OR^{a}, -SO₃R^{a}, -OSO₃R^{a}, -P(OR^{a})₂, -(PO)(OR^{a})_{2,} -O(PO)(OR^{a})₂, -B(OR^{a})₂, - (CO)NR^{b}₂, -NR^{c}=(CO)R^{a}, -SO₂NR^{b}₂, ou -NR^{c}SO₂R^{a} ;
R5 est -(CO)OR^{a}, -(CO)O(CO)R^{a}, -(CS)OR^{a}, -(SO)-OR^{a}, -SO₃R^{a}, -OSO₃R^{a}, -P(OR^{a})₂, -(PO)(OR^{a})₂, -O-(PO)(OR^{a})₂, -B(OR^{a})₂, -(CO)NR^{b}₂, -NR^{c}(CO)R^{a}, -SO₂-NR^{b}₂, ou -NR^{c}SO₂R^{a} ;
R6 est -H, -OH, un atome d'halogène ou un groupe alkyle ou alcoxy en C₁-C₃ éventuellement substitué ;
chaque R^{a} et R^{c} est indépendamment -H, un groupe aryle, aralkyle ou alkyle en C₁-C₅ ; et
chaque R^{b} est indépendamment -H, un groupe aryle, aralkyle ou alkyle en C₁-C₅, ou NR^{b}₂ est un groupe hétérocyclique non aromatique ;
où les substituants éventuels pour les groupes alkyles, aliphatiques, cycloalkyles, cycloaliphatiques, hétérocycliques, aryles, hétéroaryles, hétéroaralkyles et aralkyles sont -OH, les atomes d' halogène (-Br, -Cl, -I et -F), -R, -OR, -CH₂R, -CH₂CH₂R, -OCH₂R, -CH₂OR, -CH₂CH₂OR, -CH₂OC (O) R, -O-COR, -COR, -SR, -SCH₂R, -CH₂SR, -SOR, -SO₂R, -CN, -NO₂, -COOH, -SO₃H, -NH₂, -NHR, -N(R)₂, -COOR, -CH₂COOR, -CH₂CH₂COOR, -CHO, -CON-H₂, -CONHR, -CON(R)₂, -NHCOR, -NRCOR, -NHCONH₂, -NHCONRH, -NHCON(R)₂, -NRCONH₂, -NRCONRH, -NR-CON(R)₂, -C(=NH)-NH₂, -C(=NH)-NHR, -C(=NH)-N(R)₂, -C(=NR)-NH₂, -C(=NR)-NHR, -C(=NR)-N(R)₂, -NH-C(=NH)-NH₂, -NH-C(=NH)-NHR, -NH-C(=NH)-N(R)₂, -NH-C (=NR) -NH₂, -NH-C (=NR) -NHR, -NH-C (=NR) - N(R)₂, -NRH-C(=NH)-NH₂, -NR-C(=NH)-NHR, -NR-C-(=NH)-N(R)₂, -NR-C(=NR)-NH₂, -NR-C (=NR) -NHR, -NR-C(=NR)-N(R)₂, -SO₂NH₂, -SO₂NHR, -SO₂NR₂, -SH, -SOₖR, où k vaut 0, 1 ou 2 ou -NH-C(=NH)-NH₂; où chaque R est indépendamment un groupe alkyle, cycloalkyle, benzyle, aromatique, hétéroaromatique ou N-anilinyle ou -N(R)₂, pris ensemble, peut également former un groupe hétérocyclique ; et les substituants sur l'atome d'azote d'un groupe hétérocyclique ou d'un groupe hétéroaromatique comprennent -R', -N-(R')₂, -C(O)R', -CO₂R' , -C(O)C(O)R', -C(O)CH₂C-(O)R', -SO₂R', -SO₂N(R')₂, -C(=S)N(R')₂, -C(=NH)-N(R')₂ et -NR'SO₂R', où R' est un atome d' hydrogène, un groupe alkyle, alcoxy, cycloalkyle, cycloalcoxy, phényle, phénoxy, benzyle, benzyloxy, hétéroaromatique ou hétérocyclique.

24. Composition selon la revendication 23, dans laquelle R3 est un groupe phényle, pyridyle, benzo[1,3]dioxolyle, 2,3-dihydro-benzo[1,4]dioxine, pyrimidyle, pyrazyle, furanyle, pyrrolyle, thiényle, oxazolyle, isooxazolyle, thiazolyle, isothiazolyle, imidazolyle, naphtyle, quinoléinyle, biphényle, benzo-pyrimidyle, benzopyrazyle, benzofuranyle, indolyle, benzothiényle, benzoxazolyle, benzoisooxazolyle, benzothiazolyle, benzoisothiazolyle, ou benzimidazolyle éventuellement substitué.

25. Composition selon la revendication 24, dans laquelle R4 est un groupe phényle, pyridyle, pyrimidyle, pyrazyle, naphtyle, biphényle, phénylpyridyle, quinoléinyle, benzopyrimidyle, benzopyrazyle, cyclopropyle, cyclobutyle, cyclopentyle, cyclopentényle, cyclohexyle, cyclohexényle, ou alcényle en C₂-C₈ substitué.

26. Composition selon la revendication 25, dans laquelle le composé est représenté par la formule structurelle IV : 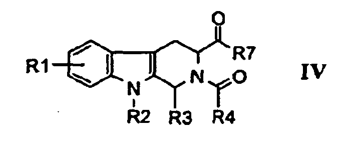 dans laquelle
R7 est -OR^{o} ou -NR^{p}₂ ;
R^{o} est -H ou un groupe aryle, aroyle, aralkyle, aralcanoyle, alkyle en C₁-C₅ ou alcanoyle en C₁-C₅ éventuellement substitué ; et
chaque R^{p} est indépendamment -H, un groupe aryle, aralkyle ou alkyle en C₁-C₅, ou NR^{p}₂ est un groupe hétérocyclique non aromatique.

27. Composition selon la revendication 26, dans laquelle R4 est représenté par l'une des formules développées R4-i à R4-vii : 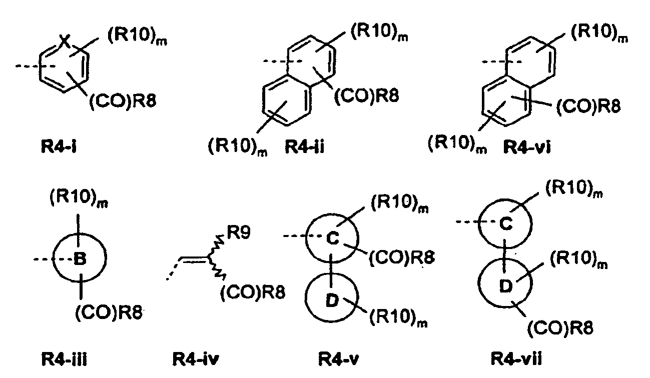 dans laquelle
chaque m vaut indépendamment 0, 1, 2 ou 3 ;
X est -N-, -CH-, ou -CR10- ;
le cycle B est un groupe cycloalkyle en C₃-C₆ ou cycloalcényle en C₃-C₆ ;
le cycle C est un groupe phényle ;
le cycle D est un groupe aryle ou hétéroaryle ;
R8 est -OR^{q} ou -NR^{r}₂ ;
R9 est -H, un groupe aryle, aralkyle ou aliphatique en C₁-C₆ ;
chaque R10 est indépendamment un atome d'halogène, -CN, -NO₂, -CF₃, -OCF₃, -ORⁱ, - (CO) Rⁱ, -(CO)ORⁱ, -O(CO)Rⁱ, -(CO)O(CO)Rⁱ, - (CS)ORⁱ, -(SO)ORⁱ, -SO₃Rⁱ, -CONR^{j}₂, -O(CO)NR^{j}₂, -NR^{k}(CO)N-R^{j}₂, -NR^{k}(CO)ORⁱ, -NR^{k}CORⁱ, -(SO₂)NR^{j}₂, -NR^{k}SO₂Rⁱ, -(CH₂)ₜNR^{j}₂, ou un groupe aryle, aralkyle ou alkyle en C₁-C₅ éventuellement substitué ;
chaque Rⁱ et R^{k} est indépendamment -H, un groupe aryle, aralkyle, alkyle en C₁-C₅ ou halogénoalkyle en C₁-C₅ ;
chaque R^{j} et R^{r} est indépendamment -H, un groupe aryle, aralkyle ou alkyle en C₁-C₅, ou chaque NR^{j}₂ et NR^{r}₂ est indépendamment un groupe hétérocyclique non aromatique ;
R^{q} est -H ou un groupe aryle, aroyle, aralkyle, aralcanoyle, alkyle en C₁-C₅ ou alcanoyle en C₁-C₅ éventuellement substitué ; et
t vaut 0 à 5.

28. Composition selon la revendication 27, dans laquelle R3 est représenté par l'une des formules développées R3-i à R3-v : 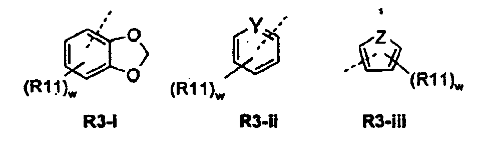 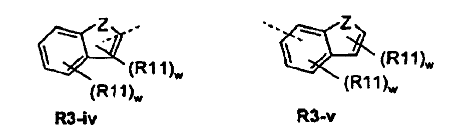 dans laquelle
Y est -N-, -CH-, ou -CR11- ;
chaque Z est indépendamment -NR^{Z}-, -S-, ou -O-, où R^{z} est -H ou un groupe alkyle en C₁-C₃ ;
w vaut 0, 1, 2 ou 3 ;
chaque R11 est indépendamment un atome d'halogène, -CN, -NO₂, -CF₃, -OCF₃, -OR¹, - (CO)R¹, -(CO)OR¹, -O(CO)R¹, -(CO)O(CO)R¹-(CS)OR¹, - (SO)OR¹, -SO₃R¹, -CONR^{m}₂, -O(CO)NR^{m}₂, -NRⁿ(CO)N-R^{m}₂, -NRⁿ(CO)OR¹, -NRⁿCOR¹, -(SO₂)NR^{m}₂, -NRⁿSO₂R¹, -(CH₂)ᵤNR¹₂, ou un groupe aryle, aralkyle ou alkyle en C₁-C₅ éventuellement substitué ;
chaque R¹ et Rⁿ est indépendamment -H, un groupe aryle, aralkyle, alkyle en C₁-C₅ ou halogénoalkyle en C₁-C₅ ;
chaque R^{m} est indépendamment -H, un groupe aryle, aralkyle ou alkyle en C₁-C₅, ou NR^{m}₂ est un groupe hétérocyclique non aromatique ; et u vaut 0 à 5.

29. Composition selon la revendication 28, dans laquelle R4 est représenté par l'une des formules développées R4-i' à R4-vii' : 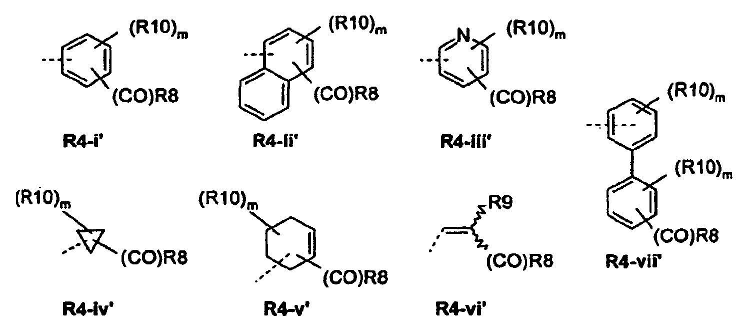 dans laquelle
chaque m vaut indépendamment 0, 1, 2 ou 3 ;
R8 est -NR^{y}₂, -OH, un groupe alcoxy en C₁-C₅ ou un groupe alcanoyloxy en C₁-C₅, où chaque R^{y} est indépendamment -H ou un groupe alkyle en C₁-C₃ ;
R9 est -H ou un groupe aliphatique en C₁-C₆ ; et chaque R10 est indépendamment -OH, -NO₂, -F, -Cl, -Br, un groupe alkyle en C₁-C₄, un groupe alcoxy en C₁-C₄, -CF₃, ou -OCF₃.

30. Composition selon la revendication 29, dans laquelle R3 est représenté par l'une des formules développées R3-i' à R3-v' : 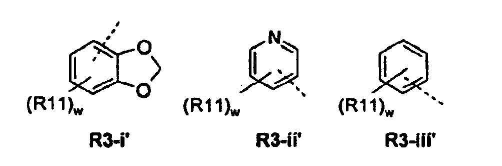 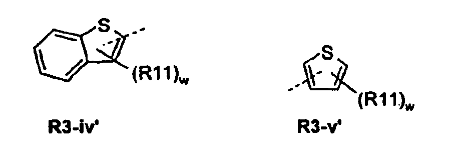 dans laquelle
w vaut 0, 1, 2 ou 3 ; et
chaque R11 est indépendamment -OH, -NO₂, -F, -Cl, -Br, un groupe alkyle en C₁-C₄, un groupe alcoxy en C₁-C₄, -CF₃, ou -OCF₃.

31. Composition selon la revendication 30, dans laquelle R7 est -NR^{x}₂, -OH, un groupe alcoxy en C₁-C₅ ou un groupe alcanoyloxy en C₁-C₅, où chaque R^{x} est indépendamment -H ou un groupe alkyle en C₁-C₃.

32. Composition selon la revendication 31, dans laquelle
R3 est représenté par l'une des structures développées R3^{a} à R3^{r} : 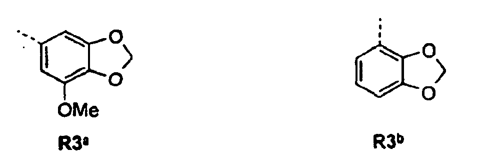 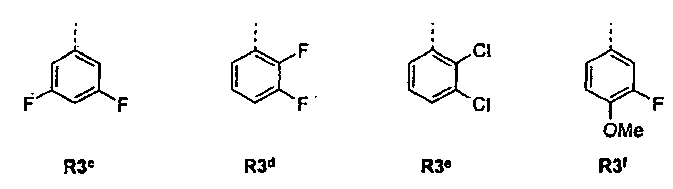 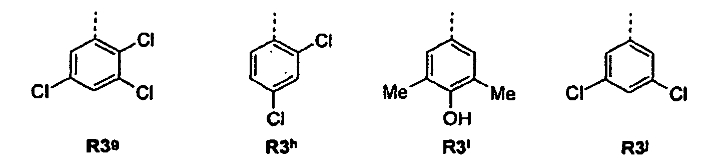 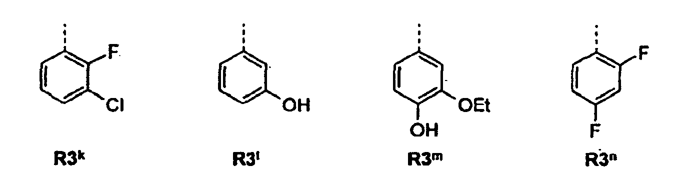 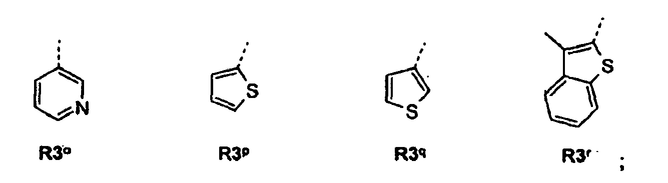 ou R4 est représenté par l'une des structures développées R4^{a} à R4^{q} :

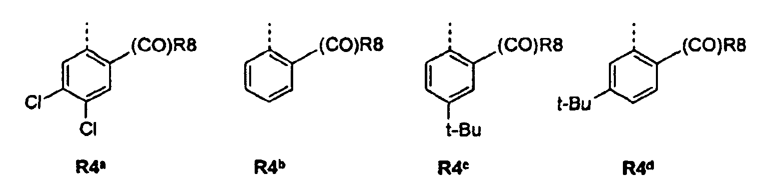

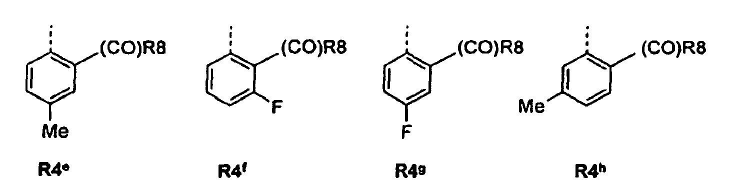

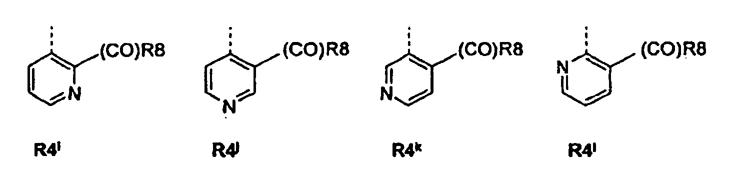

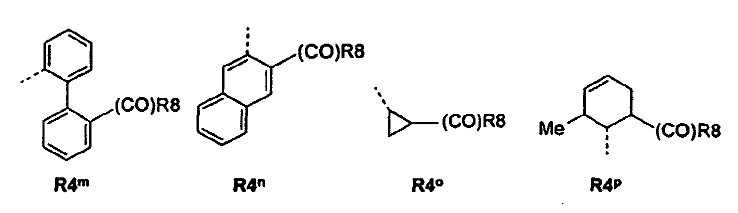

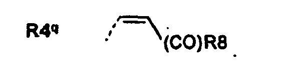

33. Composition selon la revendication 32, dans laquelle
R3 est représenté par l'une des structures développées R3^{a} à R3^{r} ; et
R4 est représenté par l'une des structures développées R4^{a} à R4^{q} .

34. Composition selon la revendication 33, dans laquelle le composé est représenté par l'une des formules développées V à XI : 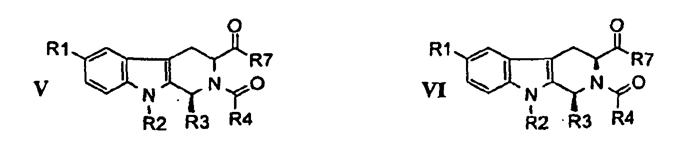 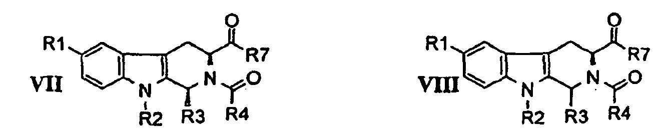 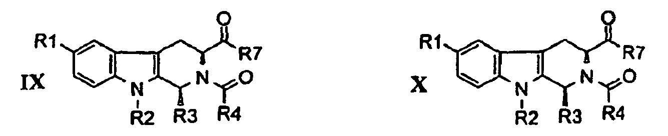 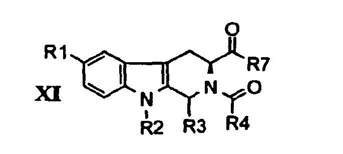 où R7 est -OH ou un groupe alcoxy en C₁-C₄.

35. Composition selon la revendication 34, dans laquelle R1 est -H, -OH, -F, -CH₃, -CF₃, -OCH₃ ou -OCF₃.

36. Composition selon la revendication 35, dans laquelle
R3 est représenté par la structure développée R3^{d}, R3^{e} ou R3^{f} ; ou
R4 est représenté par la structure développée R4^{a}, R4^{c} ou R4^{e}.

37. Composition selon la revendication 35, dans laquelle
R3 est représenté par la structure développée R3^{d}, R3^{e} ou R3^{f} ; et
R4 est représenté par la structure développée R4^{a}, R4^{c} ou R4^{e}.

38. Composition selon la revendication 37, dans laquelle R7 et R8 sont -OH.

39. Composition selon la revendication 37, dans laquelle l'un de R7 ou R8 est -OH, et l'autre est -OCH₃ ou -OCH₂CH₃.

40. Composition selon la revendication 37, dans laquelle R7 et R8 sont indépendamment -OCH₃ ou - OCH₂CH₃.
